# EUROPÄISCHE PATENTSCHRIFT

(11) **EP 1 347 978 B1**
(45) Veröffentlichungstag und Bekanntmachung des Hinweises auf die Patenterteilung: **31.08.2005**
(21) Anmeldenummer: 01271375.6
(22) Anmeldetag: 10.12.2001
(51) Int. Cl.: C07D 487/04, C07D 253/06, A61K 31/53, A61P 25/28

(54) **NEUE SUBSTITUIERTE IMIDAZOTRIAZINONE ALS PDE II-INHIBITOREN**
NOVEL SUBSTITUTED IMIDAZOTRIAZINONES AS PDE II-INHIBITORS
NOUVELLES IMIDAZOTRIAZINONES SUBSTITUEES EN TANT QU'INHIBITEURS DE PDE II

(30) Priorität: 21.12.2000 DE 10064105
(43) Veröffentlichungstag der Anmeldung: 01.10.2003
(73) Patentinhaber: Bayer HealthCare AG, 51368 Leverkusen (DE)
(72) Erfinder: NIEWÖHNER, Ulrich, 42929 Wermelskirchen (DE); SCHAUSS, Dagmar, 42697 Solingen (DE); HENDRIX, Martin, 51519 Odenthal (DE); KÖNIG, Gerhard, Arlington, MA 02476 (US); BÖSS, Frank-Gerhard, Sunninghill, Berkshire SL5 7DF (GB); VAN DER STAAY, Franz-Josef, 53797 Lohmar (DE); SCHREIBER, Rudy, Menlo Park, CA 94025 (US); SCHLEMMER, Karl-Heinz, 42113 Wuppertal (DE); GROSSER, Rolf, 51373 Leverkusen (DE)
(86) Internationale Anmeldenummer: PCT/EP2001/014450
(87) Internationale Veröffentlichungsnummer: WO 2002/050078

(56) Entgegenhaltungen:
- WO-A-99/24433
- DE-A- 19 827 640

## Beschreibung

Die vorliegende Erfindung betrifft neue substituierte Imidazotriazinone, Verfahren zu ihrer Herstellung, und ihre Verwendung zur Herstellung von Arzneimitteln insbesondere zur Verbesserung der Wahrnehmung, Konzentrationsleistung, Lernleistung und/oder Gedächtnisleistung.

Phosphodiesterasen (PDE's) spielen eine wesentliche Rolle in der Regulation der intrazellulären cGMP- und cAMP-Spiegel. Von den bisher beschriebenen Phosphodiesterase-Isoenzymgruppen PDE 1 bis PDE 10 (Beavo und Reifsnyder *Trends in Pharmacol. Sci.* **1990**, *11*, 150 - 155; Sonderling und Beavo *Curr. Opin. Cell Biol.* **2000**, *12*, 174-179) sind die PDE 1, 2, 5, 9 und 10 im Wesentlichen für den Metabolismus von cGMP verantwortlich. Aufgrund der unterschiedlichen Verteilung dieser cGMP-metabolisierenden PDE's im Gewebe sollten selektive Inhibitoren je nach Gewebsverteilung des entsprechenden Isoenzyms die c-GMP-Spiegel im entsprechenden Gewebe anheben.

Die Besonderheit der PDE 2 liegt in ihrer positiven kooperativen Kinetik bzgl. des Substrates cGMP. Es wurde postuliert, dass geringe Mengen von cGMP an die sogenannte cGMP-bindende Domäne binden und dadurch eine Aktivierung des Enzyms bewirken. Hierdurch erhöht sich auch die Affinität der katalytischen Domäne gebenüber cGMP und cAMP (Martins et al. *J. Biol. Chem*. **1982**, 257, 1973-1979). Deswegen kann PDE 2 durch geringe Mengen von cGMP beide second messenger-Systeme hydrolisieren und dadurch auch kontrollieren.

PDE 2 ist aus verschiedenen Geweben, beispielsweise aus Herz, Nebenniere, Leber, Thrombocyten und insbesondere Gehirn isoliert worden. Im Gehirn wird PDE 2-mRNA stark in der Gehirnrinde (cortex), den Basalganglien sowie im Hippocampus exprimiert (Sonnenburg et al. *Biol. Chem*. **1991**, *266,* 17655-17661). Die Sequenz der menschlichen Isoform PDE 2A3 Sequenz wurde von Rosman et al. *Gene* **1997**, *191*, 89-95. berichtet. Von den untersuchten Geweben wurde hierin die Expression von PDE 2A stark in Gehirn und Herz und schwächer in Leber, Skelettmuskel, Niere und Pankreas nachgewiesen.

Das US-A-4,278,673 offenbart Imidazopyrimidinone mit cAMP-PDE-inhibitorischer Wirkung zur Behandlung von Asthma und Bronchitis.

Aus der WO-A-99/67244 und der WO-A-99/24433 sind 7-Alkyl-2-phenyl-imidazotriazinone mit PDE 5-inhibierender Wirkung zur Behandlung von Gefäßerkrankungen bekannt.

Die EP-A-0 771 799, die WO-A-98/40384 und die WO-A-00/12504 beschreiben Purinon-, Allopurinol- bzw. Triazolopyrimidinon-Derivate, deren inhibitorische Wirkung auf cGMP-metabolisierende PDEs und ihre Eignung zur Behandlung von Gefäßerkrankungen.

Gegenstand der vorliegenden Erfindung sind Verbindungen der allgemeinen Formel (I), 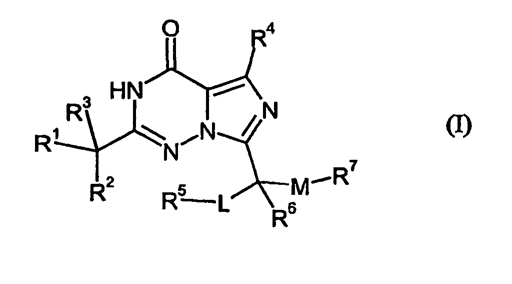 worin
- R¹: Phenyl, Naphthyl, Chinolinyl oder Isochinolinyl, die bis zu dreifach gleich oder verschieden mit Resten ausgewählt aus der Gruppe bestehend aus (C₁-C₄)-Alkyl, (C₁-C₄)-Alkoxy, Halogen, Cyano, -NHCOR⁸, -NHSO₂R⁹, -SO₂NR¹⁰R¹¹, -SO₂R¹², und -NR¹³R¹⁴ substituiert sein können, bedeutet,
worin
R⁸, R¹⁰, R¹¹, R¹³ und R¹⁴ unabhängig voneinander Wasserstoff oder (C₁-C₄)-Alkyl sind, und
R⁹ und R¹² unabhängig voneinander (C₁-C₄)-Alkyl sind,
oder
R¹⁰ und R¹¹ zusammen mit dem benachbarten Stickstoffatom einen Azetidin-1-yl-, Pyrrol-1-yl-, Piperid-1-yl-, Azepin-1-yl, 4-Methyl-piperazin-1-yl- oder Morpholin-1-yl-Rest bilden,
oder
R¹³ und R¹⁴ zusammen mit dem benachbarten Stickstoffatom einen Azetidin-1-yl-, Pyrrol-1-yl-, Piperid-1-yl-, Azepin-1-yl, 4-Methyl-piperazin-1-yl- oder Morpholin-1-yl-Rest bilden,
- R² und R³: unabhängig voneinander Wasserstoff oder Fluor bedeuten,
- R⁴: (C₁-C₄)-Alkyl bedeutet,
- R⁵: (C₁-C₃)-Alkyl bedeutet,
- R⁶: Wasserstoff oder Methyl bedeutet,
- R⁷: Phenyl, Thiophenyl, Furanyl, die bis zu dreifach gleich oder verschieden mit Resten ausgewählt aus der Gruppe bestehend aus (C₁-C₄)-Alkyl, (C₁-C₄)-Alkoxy, Halogen und Cyano substituiert sein können, oder (C₅-C₈)-Cycloalkyl bedeutet,
- L: Carbonyl oder Hydroxymethandiyl bedeutet, und
- M: (C₂-C₅)-Alkandiyl, (C₂-C₅)-Alkendiyl oder (C₂-C₅)-Alkindiyl bedeutet,
und deren physiologisch verträgliche Salze.

(C₁-C₄)-Alkyl und (C₁-C₃)-Alkyl stehen im Rahmen der Erfindung für einen geradkettigen oder verzweigten Alkylrest mit 1 bis 4 bzw. 1 bis 3 Kohlenstoffatomen. Beispielsweise seien genannt: Methyl, Ethyl, n-Propyl, Isopropyl, i-, s-, t-Butyl. Bevorzugt sind Methyl und Ethyl.

(C₂-C.₅)-Alkandiyl steht im Rahmen der Erfindung für einen geradkettigen oder verzweigten Alkandiylrest mit 2 bis 5 Kohlenstoffatomen. Beispielsweise seien genannt Ethylen, Propan-1,3-diyl, Propan-1,2-diyl, Propan-2,2-diyl, Butan-1,3-diyl, Butan-2,4-diyl, Pentan-2,4-diyl. Bevorzugt ist ein geradkettiger (C₂-C₅)-Alkan-1,ω-diyl-Rest. Beispielsweise seien genannt Ethylen, Propan-1,3-diyl, Butan-1,4-diyl, Pentan-1,5-diyl. Besonders bevorzugt sind Propan-1,3-diyl und Butan-1,4-diyl.

(C₂-C₅)-Alkendiyl steht im Rahmen der Erfindung für einen geradkettigen oder verzweigten Alkendiylrest mit 2 bis 5 Kohlenstoffatomen. Beispielsweise seien genannt Ethen-1,2-diyl, Ethen-1,1-diyl, Propen-1,1-diyl, Propen-1,2-diyl, Prop-2-en-1,3-diyl, Propen-3,3-diyl, Propen-2,3-diyl, But-2-en-1,4-diyl, Pent 2-en-1,4-diyl. Bevorzugt ist ein geradkettiger (C₂-C₅)-Alken-1,ω-diyl-Rest. Beispielsweise seien genannt Ethen-1,2-diyl, Prop-2-en-1,3-diyl, But-2-en-1,4-diyl, But-3-en-1,4-diyl, Pent-2-en-1,5-diyl, Pent-4-en-1,5-diyl. Besonders bevorzugt sind Prop-2-en-1,3-diyl, But-2-en-1,4-diyl und But-3-en-1,4-diyl.

(C₂-C₅)-Alkindiyl steht im Rahmen der Erfindung für einen geradkettigen oder verzweigten Alkindiylrest mit 2 bis 5 Kohlenstoffatomen. Beispielsweise seien genannt Ethin-1,2-diyl, Ethin-1,1-diyl, Prop-2-in-1,3-diyl, Prop-2-in-1,1-diyl, But-2-in-1,4-diyl, Pent-2-in-1,4-diyl. Bevorzugt ist ein geradkettiger (C₂-C₅)-Alken-1,ω-diyl-Rest. Beispielsweise seien genannt Ethin-1,2-diyl, Prop-2-in-1,3-diyl, But-2-in-1,4-diyl, But-3-in-1,4-diyl, Pent-2-in-1,5-diyl, Pent-4-in-1,5-diyl. Besonders bevorzugt sind Prop-2-in-1,3-diyl, But-2-in-1,4-diyl und But-3-in-1,4-diyl.

(C₁-C₄)-Alkoxy steht im Rahmen der Erfindung für einen geradkettigen oder verzweigten Alkoxyrest mit 1 bis 4 Kohlenstoffatomen. Beispielsweise seien genannt: Methoxy, Ethoxy, n-Propoxy, Isopropoxy, t-Butoxy, n-Pentoxy und n-Hexoxy. Bevorzugt sind Methoxy und Ethoxy.

(C₅-C₈)-Cycloalkyl steht im Rahmen der Erfindung für Cyclopentyl, Cyclohexyl, Cycloheptyl oder Cyclooctyl. Bevorzugt seien genannt: Cyclopentyl, Cyclohexyl oder Cycloheptyl.

Halogen steht im Rahmen der Erfindung im allgemeinen für Fluor, Chlor, Brom und Jod. Bevorzugt sind Fluor, Chlor und Brom. Besonders bevorzugt sind Fluor und Chlor.

Als Salze sind im Rahmen der Erfindung physiologisch unbedenkliche Salze der erfindungsgemäßen Verbindungen bevorzugt.

Physiologisch unbedenkliche Salze der erfindungsgemäßen Verbindungen können Säureadditionssalze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können aber auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methyhnorpholin, Dihydroabietylamin, 1-Ephenamin oder Methyl-piperidin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweilige Mischungen. Die Racemformen lassen sich ebenso wie die Diastereomeren in bekannter Weise in die stereoisomer einheitlichen Bestandteile trennen.

Bevorzugt sind Verbindungen der allgemeinen Formel (I), wobei R¹ Phenyl, dessen meta- und/oder para-Positionen bis zu dreifach gleich oder verschieden mit Resten ausgewählt aus der Gruppe bestehend aus (C₁-C₄)-Alkyl, (C₁-C₄)-Alkoxy und -SO₂NR¹⁰R¹¹ substituiert sind, bedeutet, und R², R³ R⁴, R⁵, R⁶, R⁷, R¹⁰, R¹¹, L und M die oben angegebene Bedeutung haben.

Unter den meta- und para-Positionen des Phenylringes sind diejenigen Positionen zu verstehen, die meta bzw. para zur CR²R³-Gruppe stehen. Diese Positionen können durch die folgende Strukturformel (Ic) veranschaulicht werden:

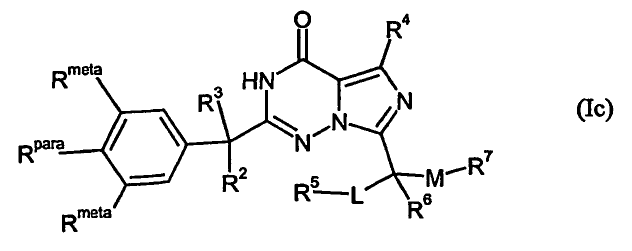

Besonders bevorzugt sind Verbindungen der allgemeinen Formel (Ic), in welchen die para- und eine meta-Position des Phenylrestes substituiert sind, und die zweite meta-Position unsubstituiert ist.

Ebenso bevorzugt sind Verbindungen der allgemeinen Formel (I), wobei R⁷ Phenyl bedeutet und R¹, R², R³, R⁴, R⁵, R⁶, L und M die oben angegebene Bedeutung haben.

Ganz besonders bevorzugt sind Verbindungen der allgemeinen Formel (I),
wobei
- R¹: Phenyl, dessen meta- und/oder para-Positionen bis zu dreifach gleich oder verschieden mit Resten ausgewählt aus der Gruppe bestehend aus (C₁-C₄)-Alkyl, (C₁-C₄)-Alkoxy und -SO₂NR¹⁰R¹¹ substituiert sind, Naphthyl oder Chinolinyl bedeutet,
worin R¹⁰ und R¹¹ unabhängig voneinander Wasserstoff oder (C₁-C₄)-Alkyl sind,
- R¹ und R²: Wasserstoff bedeuten,
- R⁴: Methyl oder Ethyl bedeutet,
- R⁵: Methyl bedeutet,
- R⁶: Wasserstoff oder Methyl bedeutet,
- L: Carbonyl oder Hydroxymethandiyl bedeutet, und
- M: geradkettiges (C₂-C₅)-Alkan-1,ω-diyl, geradkettiges (C₂-C₅)-Alken-1,ω-diyl oder geradkettiges (C₂-C₅)-Alkin-1,ω-diyl bedeutet.

Ein weiterer Aspekt der Erfindung betrifft ein neues Herstellungsverfahren für die Verbindungen der allgemeinen Formel (I), worin R¹, R², R³, R⁴, R⁵, R⁶, R⁷, L und M die oben angegebene Bedeutung haben,
wobei
[A] eine Verbindung der allgemeinen Formel (IIa), 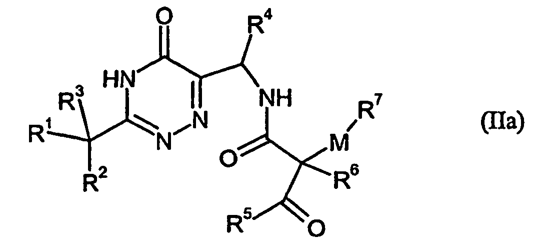 worin R¹, R², R³, R⁴, R⁵, R⁶, R⁷ und M die in Anspruch 1 angegebene Bedeutung haben,
   unter geeigneten Kondensationsbedingungen zu einer Verbindung der allgemeinen Formel (Ia), 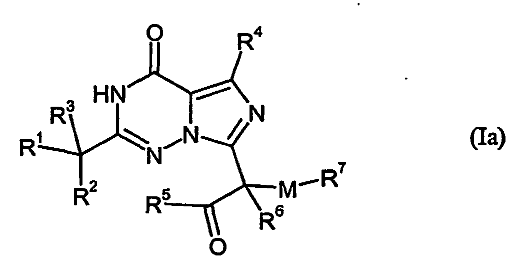 worin R¹, R², R³, R⁴, R⁵, R⁶, R⁷ und M die in Anspruch 1 angegebene Bedeutung haben,
   umgesetzt wird,
   und dann gegebenenfalls
[B] zu einer Verbindung der allgemeinen Formel (Ib) 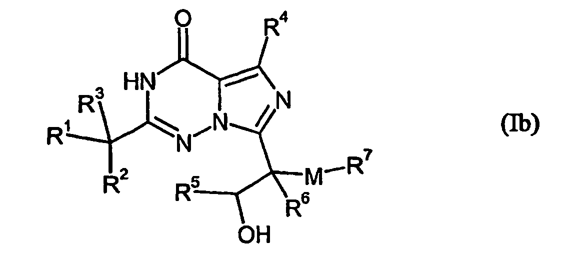
worin R¹, R², R³, R⁴, R⁵, R⁶, R⁷ und M die in Anspruch 1 angegebene Bedeutung haben,
unter geeigneten Bedingungen reduziert wird.

Die Kondensation gemäß Reaktionsschritt [A] kann durch Erhitzen der Verbindungen der allgemeinen Formel (IIa) in Abwesenheit eines Lösungsmittels oder in Anwesenheit eines inerten Lösungsmittels, insbesondere eines solchen Lösungsmittels, welches mit Wasser ein azeotropes Gemisch bildet wie beispielsweise Toluol oder Xylol, gegebenenfalls in Anwesenheit eines Säurekatalysators und/oder eines wasserbindenden Mittels erfolgen. Als Säurekatalysator eignet sich beispielsweise Chlorwasserstoff, als wasserbindende Mittel können beispielsweise Acetylchlorid, Phosphorpentoxid oder Phosphorylchlorid verwendet werden. Bevorzugt wird die Kondensation in einem inerten Lösungsmittel in Gegenwart von 1 - 10, vorzugsweise 3 - 7 Äquivalenten Phosphorylchlorid durchgefiihrt (vgl. *Chem. Ind*. **1983**, 331-335).

Als inerte Lösungsmittel für die Kondensation eignen sich die üblichen organischen Lösungsmittel, die sich unter den Reaktionsbedingungen nicht verändern. Hierzu gehören beispielsweise Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethan, Trichlorethylen oder Chlorbenzol, oder Essigester, Dimethylformamid, Hexamethylphosphorsäuretriamid, Acetonitril, Aceton, Dimethoxyethan oder Pyridin. Ebenso ist es möglich Gemische der genannten Lösungsmittel zu verwenden. Bevorzugt ist 1,2-Dichlorethan.

Die Reaktionstemperatur kann in einem größeren Bereich variiert werden. Im Allgemeinen arbeitet man in einem Bereich von -20°C bis 200°C, bevorzugt von -20°C bis 90°C.

Die erfindungsgemäßen Verfahrensschritte werden im allgemeinen bei Normaldruck durchgeführt. Es ist aber auch möglich, bei Überdruck oder bei Unterdruck zu arbeiten (z.B. in einem Bereich von 0,5 bis 5 bar).

Die Reduktion gemäß Reaktionsschritt [B] kann nach üblichen Methoden erfolgen.

Die Reduktionen erfolgen im Allgemeinen mit Hydriden oder mit Boranen, Diboranen oder ihren Komplexverbindungen in inerten Lösungsmitteln.

Die Reduktionen können auch durch Wasserstoff in Wasser oder in inerten Lösungsmitteln wie Alkoholen, Ethern oder Halogenkohlenwasserstoffen, oder deren Gemischen, mit Katalysatoren wie Raney-Nickel, Palladium, Palladium auf Aktivkohle oder Platin durchgeführt werden.

Bevorzugt werden die Reduktionen mit Hydriden, wie komplexen Borhydriden oder Aluminiumhydriden sowie Boranen durchgeführt. Besonders bevorzugt werden hierbei Natriumborhydrid, Lithiumborhydrid, Natriumcyanoborhydrid, Lithiumaluminiumhydrid, Natrium-bis-(2-methoxyethoxy)aluminiumhydrid oder Boran-Tetrahydrofuran eingesetzt.

Als Lösungsmittel für die Reduktion eignen sich hierbei alle Lösungsmittel, die sich unter den Reduktionsbedingungen nicht verändern. Hierzu gehören bevorzugt Alkohole wie Methanol, Ethanol, n-Propanol oder Isopropanol, oder Ether wie Diethylether, Dioxan, Tetrahydrofuran, Glycoldimethylether, Diethylenglycoldimethylether oder Amide wie Hexamethylphosphorsäuretriamid oder Dimethylformamid, oder Essigsäure. Ebenso ist es möglich, Gemische der genannten Lösungsmittel zu verwenden.

Die Reduktion erfolgt im allgemeinen in einem Temperaturbereich von -50°C bis zum jeweiligen Siedepunkt des Lösungsmittels, bevorzugt von -20°C bis +90°C, besonders bevorzugt von -5°C bis 30°C.

Falls erforderlich, können die Verbindungen der allgemeinen Formel (I) in die reinen Diasteromeren und/oder reinen Enantiomeren getrennt werden. Dazu eignet sich beispielsweise die chromatographische Trennung unter Normal-, Mittel- oder Hochdruckbedingungen auf stationären Phasen wie beispielsweise Kieselgel oder Reversed Phase-modifizierten Kieselgelen oder chiral modifizierten Kieselgelen. Bevorzugt wird nach der Methode der High Performance Liquid Chromatogaphy (=HPLC) mit chiralen stationären Kieselgelphasen gearbeitet. Besonders geeignet für die Trennung der Racemate sind chirale Polyamid-Kieselgelphasen auf Basis der Monomeren N-Methacryloyl-L-leucin-d-menthylamid oder N-Methacryloyl-L-leucin-1-menthylamid (vgl. EP-A-0 379 917).

Es kann sich auch als günstig erweisen, diasteromeren- und/oder enantiomerenreine Verbindungen der allgemeinen Formel (IIa) im Reaktionsschritt [A] einzusetzen und/oder gegebenenfalls vor der Durchführung des Reaktionsschrittes [B] die Verbindungen der allgemeinen Formel (Ia) in die reinen Diastereomeren und/oder Enantiomeren zu trennen.

Ebenfalls ist es möglich, die Reduktion [B] diastereoselektiv durchzuführen. Dazu wird die Reduktion zweckmäßig mit Hydriden, wie komplexen Borhydriden oder Aluminiumhydriden sowie Boranen in Gegenwart von Metallsalzen durchgeführt. Besonders bevorzugt werden solche Metallsalze, deren Kationen zu einer bidentaten Koordinierung befähigt sind wie z.B. Metalle der Hauptgruppen IIa und IIIa oder Metalle der Nebengruppen inklusive der Lanthanoide. Besonders bevorzugt sind Salze von Zn, Mn, Mg oder Ca. Als Anionen können z.B. Halogenide oder Acetate verwendet werden. Die Reaktion wird zweckmäßig in einem Alkohol oder einem Gemisch aus einem Alkohol und einem weiteren inerten Lösungsmittel durchgeführt. Bevorzugt werden Gemische aus Methanol oder Ethanol und Dichlormethan. Die Reduktion erfolgt im allgemeinen in einem Temperaturbereich von -50°C bis zum jeweiligen Siedepunkt des Lösungsmittels, bevorzugt von -20°C bis +90°C, besonders bevorzugt von -5°C bis 30°C.

Die Reduktion erfolgt i.A. unter Verwendung von 1 bis 20 Äquivalenten des Reduktionsmittels in Gegenwart von 0,1 bis 10 Äquivalenten Metallsalz. In einer bevorzugten Ausfiihrungsform verwendet man 0,2 bis 3 Äquivalente Metallsalz. Bevorzugt als Reduktionsmittel sind z.B. Natriumborhydrid, Lithiumborhydrid, Natriumcyanoborhydrid oder Zinkborhydrid.

Die Zwischenprodukte der allgemeinen Formel (II) sind neu.

Daher betrifft ein weiterer Aspekt der Erfindung die neuen Verbindungen der allgemeinen Formel (II), 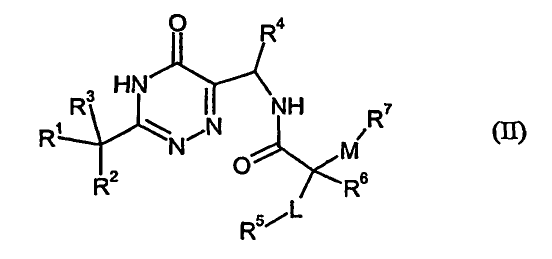 worin R¹, R², R³, R⁴, R⁵, R⁶, R⁷, L und M die oben angegebene Bedeutung haben,
und deren Salze.

Die Verbindungen der allgemeinen Formel (IIa) können beispielsweise nach bekannten Methoden durch die Oxidation entsprechender Verbindungen der allgemeinen Formel (IIb), 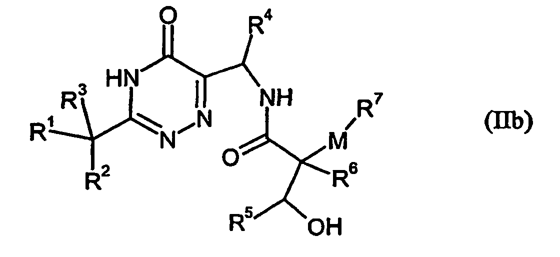 worin R¹, R², R³, R⁴, R⁵, R⁶, R⁷ und M die in Anspruch 1 angegebene Bedeutung haben, hergestellt werden, beispielsweise durch Swern-Oxidation oder Collins-Oxidation (für weitere Oxidationsmethoden siehe auch March, J.M. ,Advanced Organic Chemistry', 3. Auflage, John Wiley, New York, 1985, S. 1057-1060 und dort zitierte Literatur).

Die Herstellung der Verbindungen der allgemeinen Formel (II) kann durch das folgende Syntheseschema beispielhaft erläutert werden:

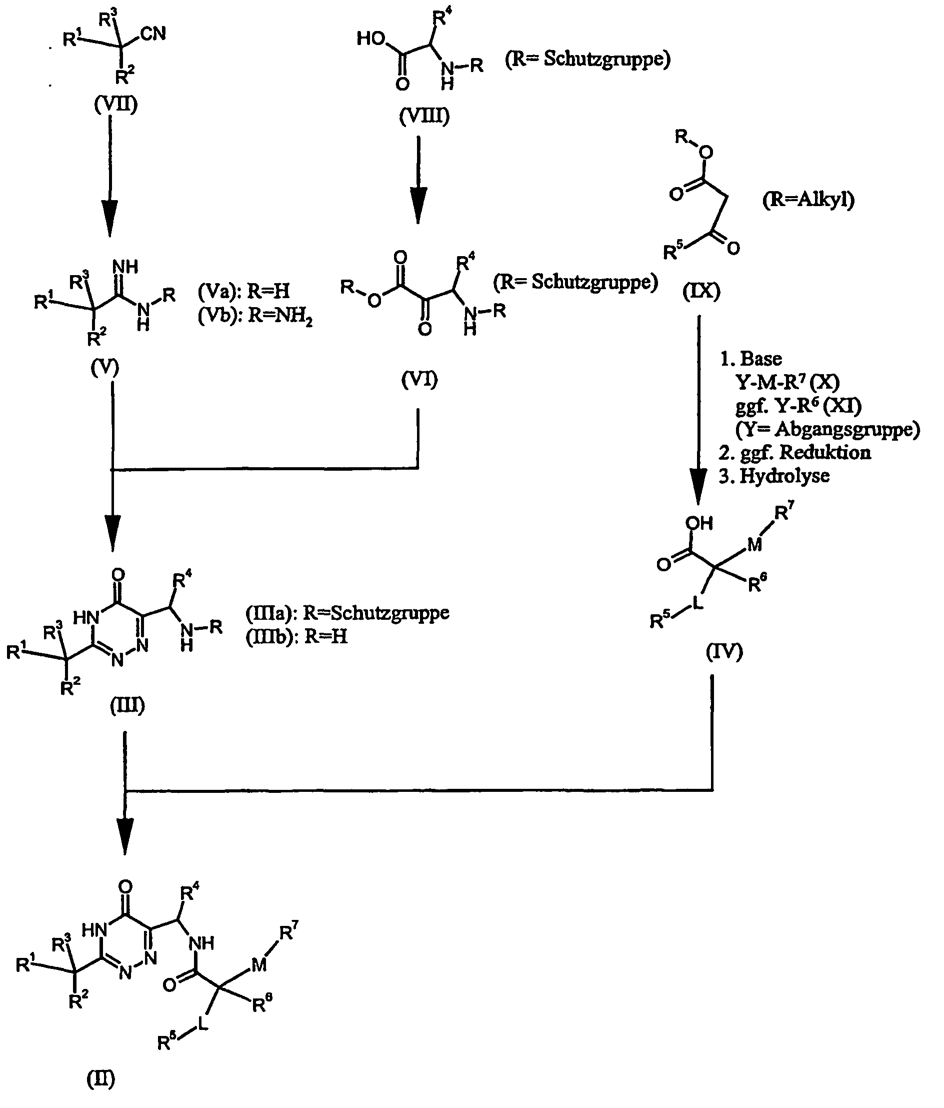

Die Verbindungen der allgemeinen Formeln (III), (IV), (V), (VI), (VII), (VIII), (IX), (X) und (XI) sind bekannt oder nach bekannten Verfahren herstellbar.

Nach diesem Reaktionsschema werden die Aminomethyltriazinone (IIIb) mit den Carbonsäuren (IV) unter für die Bildung von Amidbindungen üblichen Bedingungen mit einem Dehydratisierungsreagenz in einem inerten Lösungsmittel gegebenenfalls in Gegenwart einer Base kondensiert.

Als Dehydratisierungsreagenzien eignen sich Carbodiimide wie beispielsweise Diisopropylcarbodiimid, Dicyclohexylcarbodiimid (DCC) oder N-(3-Dimethylaminopropyl)-N'-ethylcarbodiimid-Hydrochlorid (EDC) oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Isobutylchloroformat oder Benzotriazolyloxy-tris-(dimethylamino)phosphonium-hexafluorophosphat oder Phosphorsäurediphenylesteramid oder Methansulfonsäurechlorid, gegebenenfalls in Anwesenheit von Basen wie Triethylamin, N-Ethylmorpholin, N-Methylmorpholin oder N-Methylpiperidin, und gegebenenfalls in Anwesenheit eines Katalysators wie N-Hydroxysuccinimid oder N-Hydroxybenzotriazols (HOBT). Vorzugsweise wird die Kondensation mit EDC in Gegenwart von NMM und HOBT durchgeführt.

Als Lösungsmittel eignen sich die üblichen, oben beschriebenen inerten Lösungsmittel. Bevorzugt ist Dichloromethan.

Die Aminomethyltriazinone (IIIb) sind durch Entschützung der entsprechenden N-geschützten Aminomethyltriazinone (IIIa) erhältlich, welche wiederum über Cyclokondensation der entsprechenden Amidrazone (Vb) und α-Ketoester (VI) zugänglich sind.

Als Aminoschutzgruppen für die Zwischenstufen (IIIa), (VI) und (VIII) sind beispielsweise Acyl-Reste, insbesondere die Acetyl-Gruppe geeignet. Diese Gruppen lassen sich in den N-geschützten Aminomethyltriazinone (IIIa) unter sauren Bedingungen, beispielsweise durch Erhitzen in Salzsäure, abspalten.

Die Cyclokondensation zu den N-geschützten Aminomethyltriazinonen (IIIa) läßt sich durch Erhitzen der Einzelkomponenten, der Amidrazone (Vb) und α-Ketoester (VI), in einem alkoholischen Lösungsmittel, vorzugsweise durch Erhitzen in Ethanol zum Rückfluß, bewirken.

Die Amidrazone (Vb) können durch Umsetzung der entsprechenden Amidine (Va) mit beispielsweise Hydrazin-Hydrat hergestellt werden und entweder isoliert oder in situ in der folgenden Reaktion eingesetzt werden. Die Amidine (Va) sind nach üblichen Methoden aus den entsprechenden Nitrilen (VII) zugänglich, beispielsweise durch Umsetzung der Nitrile (VII) mit Ammoniumchlorid und einer Lösung von Trimethylaluminium in Hexan zunächst in einem Temperaturbereich von -20°C bis Raumtemperatur, vorzugsweise bei 0°C und dann bei 60 bis 100°C, bevorzugt 70 bis 90°C, und vorzugsweise bei Normaldruck.

Die Nitrile (VII) sind bekannt oder nach üblichen Methoden herstellbar. Beispielsweise lassen sich Aryl-difluoro-acetonitrile aus Arylacetonitrilen oder Aryloxoacetonitrilen herstellen (vgl. *J. Org. Chem.* **1998**, *63*, 8052-8057 bzw. *J. Fluorine Chem.* **1996**, *76,* 15-20).

Die α-Ketoester (VI) können aus den entsprechenden N-geschützten a-Aminosäuren (VIII) hergestellt werden, beispielsweise durch Umsetzung mit Oxalsäurechloridmonoethylester.

Die Carbonsäuren (TV) sind durch Alkylierung der entsprechenden β-Ketoester (IX) mit den Elektrophilen (X) und gegebenenfalls (XI), gefolgt von Esterhydrolyse und gegebenenfalls Reduktion der β-Carbonylfunktion, zugänglich.

Zur Alkylierung wird der β-Ketoester (IX) beispielsweise mit einer Base, vorzugsweise einem Hydrid wie Natriumhydrid, in einem inerten Lösungsmittel wie z.B. Tetrahydrofuran in einem Temperaturbereich von vorzugsweise 0°C bis Raumtemperatur deprotoniert und nach Isolierung oder in situ mit einer Lösung des Elektrophils (X) oder (XI) in vorzugsweise 1,3-Dimethyltetrahydro-2-(1H)-pyrimidon unter Zugabe einer katalytischen Menge von Kaliumiodid versetzt. Wenn R⁶ nicht Wasserstoff ist, kann die Alkylierung mit einem zweiten Elektrophil wiederholt werden, nachdem das Monoalkylierungsprodukt gegebenenfalls isoliert worden ist. Die Abgangsgruppe Y im Elektrophil (X) oder (XI) ist vorzugsweise ein Halogen, besonders bevorzugt Brom.

Die β-Carbonylfunktion kann nach den oben beschriebenen Bedingungen für den Reaktionsschritt [B] reduziert werden.

Die Hydrolyse des Esters zur Carbonsäure (IV) erfolgt nach üblichen Bedingungen, im Falle des Methyl- oder Ethylesters bevorzugt mit Natron- oder Kalilauge.

Substituenten, beispielsweise in R¹, lassen sich über die Startmaterialien einfuhren, wie beispielsweise über das Nitril (VII), können aber auch auf einer späteren Verfahrensstufe eingeführt oder modifiziert werden.

So läßt sich beispielsweise der Substituent -SO₂NR¹⁰R¹¹ in R¹ einführen, indem ein entsprechendes N-geschütztes Aminomethyltriazinon (IIIa) mit Chlorsulfonsäure chlorsulfoniert wird und dann mit einem entsprechenden Amin HNR¹⁰R¹¹ weiter umgesetzt wird zum entsprechenden Sulfonsäureamid.

Dies lässt sich durch folgendes Reaktionsschema verdeutlichen:

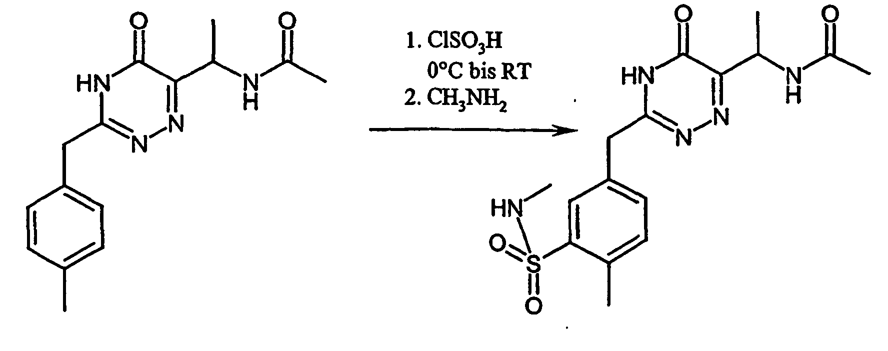

Die erfindungsgemäßen Verbindungen zeigen ein nicht vorhersehbares, wertvolles pharmakologisches Wirkspektruin: sie inhibieren bevorzugt PDE 2, und/oder zeigen ein günstiges pharmakokinetisches Profil.

Die Inhibition der PDE 2 führt zu einem differenzierten Anstieg von cGMP. Die differenzierende Wirkung wird von der Verteilung der Isoenzyme im Gewebe mitbestimmt.

Außerdem verstärken die erfindungsgemäßen Verbindungen die Wirkung von Substanzen, wie beispielsweise EDRF (Endothelium derived relaxing factor) und ANP (atrial natriuretic peptide), die den cGMP-Spiegel steigern.

Wegen ihrer selektiven PDE 2-Inhibition eignen sich die erfindungsgemäßen Verbindungen besonders zur Verbesserung der Wahrnehmung, Konzentrationsleistung, Lernleistung, oder Gedächtnisleistung, insbesondere nach kognitiven Störungen, wie sie beispielsweise bei Situationen/Krankheiten/Syndromen auftreten wie "Mild cognitive impairment", Altersassozüerte Lern- und Gedächtnisstörungen, Altersassozüerte Gedächtnisverluste, Vaskuläre Demenz, Schädel-Hirn-Trauma, Schlaganfall, Demenz, die nach Schlaganfällen auftritt ("post stroke dementia"), post-traumatisches Schädel Hirn Trauma, allgemeine Konzentrationsstörungen, Konzentrationsstörungen in Kindern mit Lern- und Gedächtnisproblemen, Alzheimersche Krankheit, Vaskuläre Demenz, Demenz mit Lewy-Körperchen, Demenz mit Degeneration der Frontallappen einschließlich des Pick's Syndroms, Parkinsonsche Krankheit, Progressive nuclear palsy, Demenz mit corticobasaler Degeneration, Amyolateralsklerose (ALS), Huntingtonsche Krankheit, Multiple Sklerose, Thalatnische Degeneration, Creutzfeld-Jacob-Demenz, HIV-Demenz, Schizophrenie mit Demenz oder Korsakoff-Psychose.

Die erfindungsgemäßen Verbindungen eignen sich allgemein zur Behandlung und/oder Prophylaxe von Demenz.

Der Wirkstoff kann systemisch und/oder lokal wirken. Zu diesem Zweck kann er auf geeignete Weise appliziert werden, wie z.B. oral, parenteral, pulmonal, nasal, sublingual, lingual, buccal, rectal, transdermal, conjunctival, otisch oder als Implantat.

Für diese Applikationswege kann der Wirkstoff in geeigneten Applikationsformen verabreicht werden.

Für die orale Applikation eignen sich bekannte, den Wirkstoff schnell und/oder modifiziert abgebende Applikationsformen, wie z.B. Tabletten (nichtüberzogene sowie überzogene Tabletten, z.B. magensaftresistente Überzüge), Kapseln, Dragees, Granulate, Pellets, Pulver, Emulsionen, Suspensionen und Lösungen.

Die parenterale Applikation kann unter Umgehung eines Resorptionsschrittes geschehen (intravenös, intraarteriell, intrakardial, intraspinal oder intralumbal) oder unter Einschaltung einer Resorption (intramuskulär, subcutan, intracutan, percutan, oder intraperitoneal). Für die parenterale Applikation eignen sich als Applikationsformen u.a. Injektions- und Infusionszubereitungen in Form von Lösungen, Suspensionen, Emulsionen, Lyophilisaten und sterilen Pulvern.

Für die sonstigen Applikationswege eignen sich z.B. Inhalationsarzneiformen (u.a. Pulverinhalatoren, Nebulizer), Nasentropfen/-lösungen, Sprays; lingual, sublingual oder buccal zu applizierende Tabletten oder Kapseln, Suppositorien, Ohren- und Augen-präparationen, Vaginalkapseln, wäßrige Suspensionen (Lotionen, Schüttelmixturen), lipophile Suspensionen, Salben, Cremes, Milch, Pasten, Streupuder oder Implantate.

Die Wirkstoffe können in an sich bekannter Weise in die angeführten Applikationsformen überführt werden. Dies geschieht unter Verwendung inerter nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) oder Geschmacks- und/oder Geruchskorrigentien.

Im Allgemeinen hat es sich als vorteilhaft erwiesen, bei parenteraler Applikation Mengen von etwa 0,001 bis 30 mg/kg, vorzugsweise etwa 0,01 bis 10 mg/kg Körpergewicht zur Erzielung wirksamer Ergebnisse zu verabreichen. Bei oraler Applikation beträgt die Menge etwa 0,01 bis 100 mg/kg, vorzugsweise etwa 0,1 bis 30 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt.

### Messung der PDE-Inhibition

Die cGMP-stimulierbare PDE (PDE 2), die cGMP-hemmbare PDE (PDE 3) und die cAMP-spezifische PDE (PDE 4) wurden entweder aus Schweine- oder Rinderherzmyokard isoliert. Die Ca²⁺-Calmodulin-stimulierbare PDE 1 wurde aus Schweineaorta, Schweinehirn oder bevorzugt aus Rinderaorta isoliert. Die cGMP-spezifische PDE (PDE 5) wurde aus Schweinedünndarm, Schweineaorta, humanen Blutplättchen und bevorzugt aus Rinderaorta gewonnen. Die Reinigung erfolgte durch Anionenaustauschchromatographie an MonoQ^{R} Pharmacia im Wesentlichen nach der Methode von Hoey, M; Houslay, M.D., *Biochem: Pharmacol.* **1990,** *40*, 193-202 und Lugman et al. *Biochem. Pharmacol*. **1986,** *35*, 1743-1751.

Die Bestimmung der Enzymaktivität erfolgt in einem Testansatz von 100 µl in 20 mM Tris/HCl-Puffer pH 7,5 der 5 mM MgCl₂, 0,1 mg/ml Rinderserumalbumin und entweder 800 Bq [³H]-cAMP oder [³H]-cGMP enthält. Die Endkonzentration der entsprechenden Nucleotide ist 10⁻⁶ mol/l. Die Reaktion wird durch Zugabe des Enzyms gestartet, die Enzymmenge ist so bemessen, daß während der Inkubationszeit von 30 min ca. 50 % des Substrates umgesetzt werden. Um die cGMP stimulierbare PDE 2 zu testen, wird als Substrat [³H]-cAMP verwendet und dem Ansatz 10⁻⁶ mol/l nicht markiertes cGMP zugesetzt. Um die Ca-Calmodulinabhängige PDE 1 zu testen, werden dem Reaktionsansatz noch CaCl₂ 1 µM und Calmodulin 0,1 µM zugesetzt. Die Reaktion wird durch Zugabe von 100 µl Acetonitril, das 1 mM cAMP und 1 mM AMP enthält, gestoppt 100 µl des Reaktionsansatzes werden auf der HPLC getrennt und die Spaltprodukte "Online" mit einem Durchflußscintillationszähler quantitativ bestimmt. Es wird die Substanzkonzentration gemessen, bei der die Reaktionsgeschwindigkeit um 50 % vermindert ist. Zusätzlich wurde zur Testung der "Phosphodiesterase [³H] cAMP-SPA enzyme assay" und der "Phosphodiesterase [³H] cGMP-SPA enzyme assay" der Firma Amersham Life Sciences verwendet. Der Test wurde nach dem vom Hersteller angegebenen Versuchsprotokoll durchgefiihrt.

Die Bestimmung der Aktivität der Testsubstanzen an PDE 2 erfolgt mit dem [³H]cAMP Scintillation Proximity Assay (SPA) Kit (TRKQ7090) von Amersham International (Little Chalfont, England) bzw. an PDE1 und PDE5 mit dem [³H]cGMP Scintillation Proximity Assay (SPA) Kit (TRKQ7100) von Amersham International (Little Chalfont, England).

Testsubstanzen wurden in 100 % DMSO gelöst (10 mM), und diese Lösung wurde weiter mit H₂O verdünnt (höchste Endkonzentration im Test: 10µM). Zur Vorstimulation der PDE2 wird cGMP beigefiigt (Endkonzentration im Test: 10⁻⁶ M). Das Enzym wird in PDE Puffer (20mM TRIS/HCI, 5mM MgCl₂, 0,1mg/ml Albumin, pH 7,5) verdünnt. In einer 96-Loch Platte (Wallac, 1450-401) werden pro Loch folgende Volumina pipettiert: 10 µl Substanzlösung (beim 100 % Wert 10µl H₂O), 10 µl cGMP (10⁻⁵ M), 70 µl [³H]-cAMP Testgemisch (siehe Kit), 10 µl Enzym (beim 0-Wert kein Enzym, stattdessen + 10µl H₂O) zum Start der Reaktion. Nach 15 min Inkubation bei 30°C wurde die Reaktion mit 50 µl SPA-Beadlösung (siehe Kit) gestoppt, die Platte mit einer Folie verschlossen und 30 Sekunden geschüttelt. Nach dem Absetzen der Beads (ca. 15 min) wurde die Platte im beta-counter gemessen.

Für die Messung der PDE 1 wurden Calmodulin 10⁻⁷ M und CaCl₂ 1µM zum Reaktionsansatz zugegeben. Die PDE 5 wurde mit dem [³H] cGMP SPA Assay gemessen.

Beispielsweise inhibiert unter den oben angegebenen Bedingungen Beispiel 2 die PDE 2 mit einem IC₅₀-Wert von 10 nM.

### Messung der Erhöhung der intrazellulären neuronalen cGMP-Konzentration in Zellkulturen

PDE 2-Inhibitoren erhöhen die intrazelluläre neuronale cGMP Konzentration nach Vorstimulierung der Guanylatzyklase mit 10⁻⁴ M Natriumnitroprussid (SNP) in primären Rattenhirnzellkulturen.

Rattenembryonen wurden dekapitiert, die Köpfe in Präparationsschalen überführt. Die Kopfhaut und Schädeldecke wurden entfernt, und die freipräparierten Gehirne wurden in eine weitere Petrischale überfiihrt. Mithilfe eines Binokulars und zweier Pinzetten wurden Hippocampi aus dem Großhirn (Cortex) isoliert und mit Eis auf 4°C gekühlt. Diese Präparation und die Vereinzelung der hippocampalen Neuronen wurden dann nach einem Standardprotokoll mit dem Papain Dissociationssystem (Worthington Biochemical Corporation, Lakewood, New Jersey 08701, USA) durchgeführt (Huettner et al. *J. Neurosci.* **1986**, 6, 3044-3060). Die mechanisch vereinzelten Neuronen wurden zu 150.000 Zellen/Loch in 200 µl Neurobasalmedium/Loch (Neurobasal; Gibco/BRL; 2mM L-Glutamin; in Anwesenheit von Penicillin/Streptomycin) 7 Tage in 96 Lochplatten (mit Poly-D Lysin 100 µg/ml für 20 min vorbehandelt) unter Standard Bedingungen kultiviert (37°C, 5 % CO₂). Nach 7 Tagen wurde das Medium abgenommen und die Zellen mit HBS Puffer (GibcoBRL) gewaschen. Anschliessend wurden je 100µl SNP-Lösung und 100µL des Racemats von Beispiel 1 (zuvor in 100 % DMSO gelöst: 10 mM) in HBS auf die Zellen gegeben, so dass die Endkonzentration von SNP 100 mM und die des Racemats von Beispiel 1 so lag wie in Figur 1 angegeben und bei 37°C für 20 min inkubiert. Danach wurden die Zellen in 200µl Lysispuffer (cGMP Kit code RPN 226; von Amersham Pharmacia Biotech.) lysiert und die cGMP Konzentration nach den Angaben des Herstellers gemessen. Alle Messungen wurden in Triplicaten durchgeführt. Die Statistische Auswertung erfolgte mit Prism Software Version 2.0 (GraphPad Software Inc., San Diego, CA USA; *** p<0,001).

Bei paralleler Inkubation von Neuronen mit SNP (einem Stimulator der Guanylatzyklase) und Beispiel 15 zeigte sich bereits ab einer Konzentration von 100 nM eine deutliche Erhöhung des intrazellulären cGMP Spiegels.

### Obiekt-Wiedererkennunestest

Der Objekt-Wiedererkennungstest ist ein Gedächtnistest. Er misst die Fähigkeit von Ratten (und Mäusen), zwischen bekannten und unbekannten Objekten zu unterscheiden und eignet sich daher zur Bestimmung der gedächtnisverbessernden Wirkung der erfindungsgemäßen Verbindungen.

Der Test wird wie beschrieben durchgeführt (Blokland et al. *NeuroReport* **1998**, *9*, 4205-4208; Ennaceur, A., Delacour, J.,. *Behav. Brain Res.* **1988,** *31*, 47-59; Ennaceur, A., Meliani, K.,. *Psychopharmacology* **1992,** *109,* 321-330; Prickaerts, et al. *Eur. J. Pharmacol.* **1997,** *337*, 125-136).

In einem ersten Durchgang wird eine Ratte in einer ansonsten leeren größeren Beobachtungsarena mit zwei identischen Objekten konfrontiert. Die Ratte wird beide Objekte ausgiebig untersuchen, d.h. beschnüffeln und berühren. In einem zweiten Durchgang, nach einem Intervall von 24 Stunden, wird die Ratte erneut in die Beobachtungsarena gesetzt. Nun ist eines der bekannten Objekte durch ein neues, unbekanntes Objekt ersetzt. Wenn eine Ratte das bekannte Objekt wiedererkennt, wird sie vor allem das unbekannte Objekt untersuchen. Nach 24 Stunden hat eine Ratte jedoch normalerweise vergessen, welches Objekt sie bereits im ersten Durchgang untersucht hat, und wird daher beide Objekte gleichstark inspektieren. Die Gabe einer Substanz mit lem- und gedächtnisverbessernder Wirkung wird dazu führen, dass eine Ratte das bereits 24 Stunden vorher, im ersten Durchgang, gesehene Objekt als bekannt wiedererkennt. Sie wird das neue, unbekannte Objekt ausfiihrlicher untersuchen als das bereits bekannte. Diese Gedächtnisleistung wird in einem Diskriminationsindex ausgedrückt. Ein Diskriminationsindex von Null bedeutet, dass die Ratte beide Objekte, das alte und das neue, gleichlang untersucht; d.h. sie hat das alte Objekt nicht wiedererkannt und reagiert auf beide Objekte als wären sie unbekannt und neu. Ein Diskriminationsindex größer Null bedeutet, dass die Ratte das neue Objekt länger inspektiert als das alte; d.h. die Ratte hat das alte Objekt wiedererkannt.

Beispiel 16 zeigt unter diesen Bedingungen einen Effekt bei einer Dosierung von 1,0 mg/kg Körpergewicht p.o.

### Begriffsdefinitionen

Chromatographie wurde, wenn nicht anders ausgeführt, auf Kieselgel Si 60 durchgeführt. Bei der Flash-Chromatographie wurden normalerweise den beschriebenen Bedingungen gefolgt (vgl. Still J. Org. Chem.).

Wenn nicht anders bezeichnet, wurden die Reaktionen unter Argon und, wo notwendig, unter wasserfreien Bedingungen durchgeführt.
- HPLC =: Hochdruck- Flüssigchromatographie
- MS =: Massenspekrometrie
- NMR =: Kernresonanzspektroskopie
- LC-MS =: Flüssigchromatographie kombiniert mit Massenspekrometrie
- MeOH=: Methanol
- DMSO=: Dimethylsulfoxid
- d. Th. =: der Theorie

### Ausgangsverbindungen

### Beispiel 1A

*N*-Acetylalanin

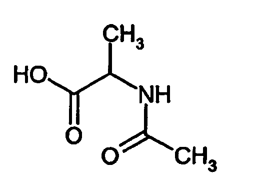

134 g (1,50 Mol) DL-Alanin werden in Essigsäure vorgelegt und tropfenweise mit 230 g (2,25 Mol) Acetanhydrid versetzt. Es wird zur Vollendung der Reaktion noch 2 h bei 100°C gerührt und dann im Vakuum das Lösemittel abgezogen. Der erhaltene Feststoff wird in Essigester suspendiert und abgesaugt. Zur Reinigung wird der Feststoff mehrmals mit Diethylether gewaschen.
Ausbeute: 162 g (82, 6 % d. Th.)
¹H-NMR (Methanol-d₄): δ/ppm 1,38 (d, 3H), 1,97 (s, 3H), 4,37 (q, 1H).

### Beispiel 2A

2-(Acetylamino)butansäure

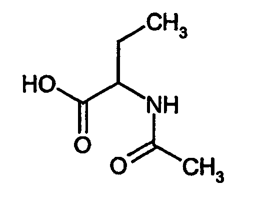

163 g (1,58 Mol) 2-Aminobuttersäure werden analog **Beispiel 1A** mit 242 g (2,37 Mol) Acetanhydrid zu 2-(Acetylamino)butansäure umgesetzt.
Ausbeute: 220 g (95,9 % d. Th.)
¹H-NMR (Methanol-d₄): δ/ppm 0,97 (t, 3H), 1,65-1,93 (m, 2H), 1,99 (s, 3H), 4,29 (q, 1H).

### Beispiel 3A

2-(4-Methylphenyl)ethanamidin Hydrochlorid

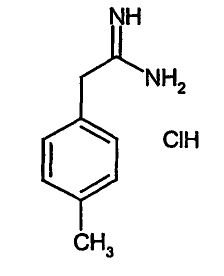

10,8 g (201 mmol) Ammoniumchlorid werden unter Argon in 200 ml trockenem Toluol suspendiert und die Suspension wird auf 0°C abgekühlt. 100 ml einer 2M Lösung von Trimethylaluminium in Hexan werden zugetropft und die Mischung bis zur beendeten Gasentwicklung bei Raumtemperatur gerührt. Nach Zugabe von 13,2 g (100 mmol) 4-Methylbenzylcyanid wird die Reaktionsmischung über Nacht bei 80°C (Bad) gerührt. Die abgekühlte Reaktionsmischung wird unter Eiskühlung mit 35 ml Methanol versetzt und im Anschluß noch 1 h bei Raumtemperatur gerührt. Dann wird das Filtrat vom Feststoff abgesaugt, und der Filterkuchen noch mehrmals mit Methanol gewaschen. Das Filtrat wird eingeengt, in Dichlormethan/Methanol 10/1 resuspendiert, und vom unlöslichen Feststoff abgetrennt. Dann wird das Lösungsmittel abermals im Vakuum vom Filtrat evakuiert.
Ausbeute: 16,4 g (88,1 % d. Th.)
¹H-NMR (Methanol-d₄): δ/ppm 2,35 (s, 3H), 3,77 (s, 2H), 7,21-7,29 (m, 4H).

### Beispiel 4A

2-(4-Methoxyphenyl)ethanamidin Hydrochlorid

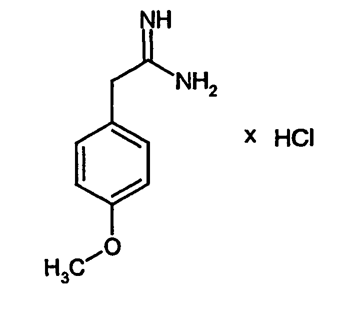

Analog zum **Beispiel 3A** erhält man aus 21,4 g (400 mmol) Ammoniumchlorid, 200 ml einer 2M Lösung von Trimethylaluminium in Hexan und 29,4 g (200 mmol) 4-Methoxybenzylcyanid 28,5 g (71,3 % d. Th.) 2-(4-Methoxyphenyl)ethanamidin Hydrochlorid.
Schmelzpunkt: 126°C

### Beispiel 5A

2-(3,4-Dimethoxyphenyl)ethanamidin Hydrochlorid

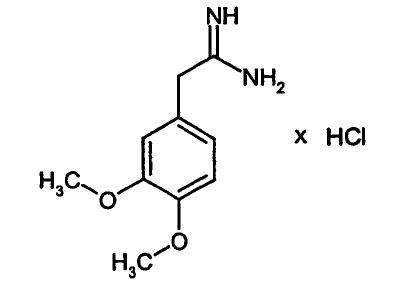

Analog zum **Beispiel 3A** erhält man aus 72,5 g (1,35 Mol) Ammoniumchlorid, 672 ml einer 2M Lösung von Trimethylaluminium in Hexan und 120 g (677 mmol) 3,4-Dimethoxybenzylcyanid 112 g (71,7 % d. Th.) 2-(3,4-Dimethoxyphenyl)ethanamidin Hydrochlorid.
¹H-NMR (DMSO-d₆): δ/ppm 3,62 (s, 2H), 3,74 (s, 3H), 3,76 (s, 3H), 6,92-7,14 (m, 3H).

### Beispiel 6A

2-(3-Ethoxy-4-methoxyphenyl)ethanamidin Hydrochlorid

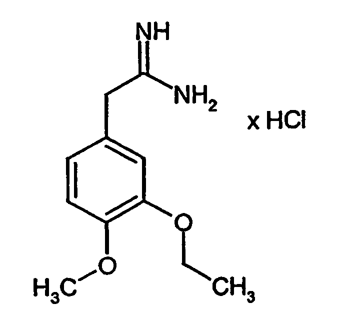

Analog zum **Beispiel 3A** erhält man aus 5,59 g (105 mmol) Ammoniumchlorid, 52,1 ml einer 2M Lösung von Trimethylaluminium in Toluol und 10 g (52,3 mmol) 3-Ethoxy-4-methoxybenzylcyanid 3,8 g (29,7 % d. Th.) 2-(3-Ethoxy-4-methoxyphenyl)ethanamidin Hydrochlorid.
¹H-NMR (DMSO-d₆): δ/ppm 1,34 (t, 3H), 3,59 (s, 2H), 3,73 (s, 3H), 4,02 (q, 2H), 6,92-7,12 (m, 3H).

### Beispiel 7A

2-(1-Naphthyl)ethanamidin Hydrochlorid

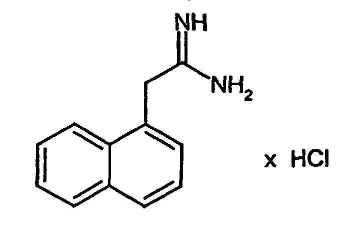

Analog zum **Beispiel 3A** erhält man aus 32,9 g (614 mmol) Ammoniumchlorid, 307 ml einer 2M Lösung von Trimethylaluminium in Toluol und 51,4 g (307 mmol) 1-Naphthylacetonitril 56,3 g (83,1 % d. Th.) 2-(1-Naphthyl)ethanamidin Hydrochlorid.
¹H-NMR (DMSO-d₆): δ/ppm 4,28 (s, 2H), 7,48-8,06 (m, 7H).

### Beispiel 8A

2-(6-Chinolinyl)ethanamidin Hydrochlorid

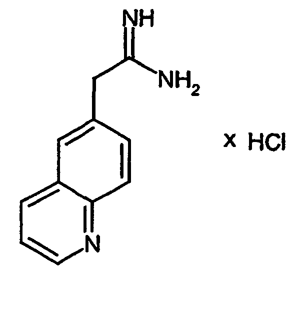

21,4 g (400 mmol) Ammoniumchlorid werden in 400 ml trockenem Toluol suspendiert und die Suspension auf 0°C abgekühlt. 200 ml einer 2M Lösung von Trimethylaluminium in Toluol werden zugetropft und die Mischung bis zur beendeten Gasentwicklung bei Raumtemperatur gerührt. Nach Zugabe von 17,9 g (88,9 mmol) Methyl 6-chinolinylacetat wird die Reaktionsmischung über Nacht bei 100 °C (Bad) gerührt. Die abgekühlte Reaktionsmischung wird mit 90 g Kieselgel versetzt und im Anschluß noch 15 min bei Raumtemperatur gerührt. Dann wird das Filtrat vom Feststoff abgesaugt, und der Filterkuchen noch mehrmals mit Dichlormethan/Methanol 10/1 gewaschen. Das Filtrat wird eingeengt.
¹H-NMR (DMSO-d₆): δ/ppm 3,97 (s, 2 H), 7,54-9,31 (m, 6 H).

### Beispiel 9A

Ethyl 3-(acetylamino)-2-oxobutanoat

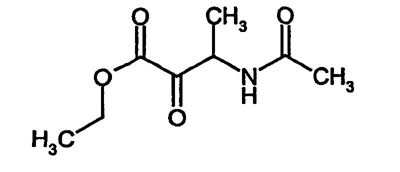

10,65 g (81,2 mmol) Acetylalanin werden in 150 ml Tetrahydrofuran aufgenommen und mit 19,3 g (244 mmol) Pyridin und einer Spatelspitze *N,N*-Dimethylaminopyridin am Rückfluß erhitzt. In der Siedehitze werden 22,2 g (162 mmol) Oxalsäuresterchlorid zugetropft. Das Gemisch wird im Anschluß noch so lange am Rückfluß erhitzt, bis keine Gasentwicklung mehr beobachtet werden kann. Nach dem Erkalten wird der Ansatz auf Eiswasser gegeben und die organische Phase in Essigsäureethylester extrahiert. Die getrocknete organische Phase wird eingeengt und in Ethanol gelöst direkt weiter umgesetzt.

### Beispiel 10A

*N*-{1-[3-(4-Methylbenzyl)-5-oxo-4,5-dihydro-1,2,4-triazin-6-yl]ethyl}acetamid

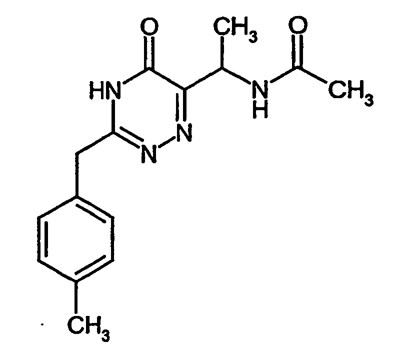

10 g (54,2 mmol) 2-(4-Methylphenyl)ethanamidin Hydrochlorid werden im 100 ml Ethanol aufgenommen und mit 3,25 g (65,0 mmol) Hydrazinhydrat versetzt. Es wird 45 min gerührt, dann erfolgt die Zugabe von **Beispiel 9A**. Im Anschluß wird 4 h bei 80°C (Bad) und über Nacht bei Raumtemperatur gerührt. Gereinigt wird die Substanz per *flash*-Chromatographie, wobei Vorfraktionen zuerst mit Essigsäureethylester abgetrennt werden. Das Produkt wird mit Dichlormethan/Methanol 30/1 eluiert.
Ausbeute: 5,63 g (36,3% d. Th.)
¹H-NMR (Methanol-d₄): δ/ppm 1,40 (d, 3H), 1,93 (s, 3H), 2,29 (s, 3H), 3,85 (s, 2 H), 5,12 (q, 1H), 7,12-7,23 (m, 4H).

### Beispiel 11A

6-(1-Aminoethyl)-3-(4-methylbenzyl)-1,2,4-triazin-5(4H)-on

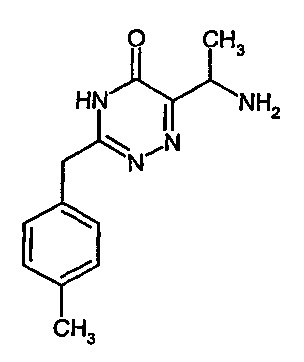

20 g (69,9 mmol) *N*-{1-[3-(4-Methylbenzyl)-5-oxo-4,5-dihydro-1,2,4-triazin-6-yl]-ethyl}acetamid werden in 200 ml 2 N Salzsäure 18 h am Rückfluß gerührt. Dann wird das abgekühlte Gemisch mit 6 N NaOH neutralisiert und bis zur Trockene am Vakuum evakuiert. Es wird in Methanol suspendiert und das Filtrat vom Salz abgetrennt. Das eingeengte Filtrat wird *flash-* chromatographiert mit Dichlormethan/Methanol 20/1 und 5/1.
Ausbeute: 8 g (46,9 % d. Th.)
¹H-NMR (Methanol-d₄): δ/ppm 1,50 (d, 3H), 2,20 (s, 3H), 3,84 (s, 2H), 4,52 (q, 1 H), 7,03 (d, 2H), 7,13 (d, 2H).

### Beispiel 12A

*N*-{1-[3-(4-Methoxybenzyl)-5-oxo-4,5-dihydro-1,2,4-triazin-6-yl]ethyl}acetamid 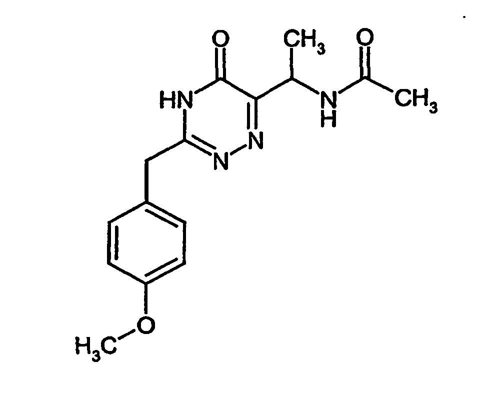 Analog zum **Beispiel 10A** werden 5,1 g (25,4 mmol) 2-(4-Methoxyphenyl)-ethanamidin Hydrochlorid mit 1,53 g (30,5 mmol) Hydrazinhydrat und 7,14 g (38,1 mmol) Ethyl 3-(acetylamino)-2-oxobutanoat zu *N*-{1-[3-(4-Methoxybenzyl)-5-oxo-4,5-dihydro-1,2,4-triazin-6-yl]ethyl}acetamid umgesetzt.
Ausbeute: 2,97 g (38,7 % d. Th.)
¹H-NMR (Methanol-d₄): δ/ppm 1,44 (d, 3H), 1,99 (s, 3H), 3,78 (s, 3H), 3,91 (s, 2 H), 5,23 (q, 1H), 6,90 (d, 2H), 7,28 (d, 2H).

### Beispiel 13A

6-(1-Aminoethyl)-3-(4-methoxybenzyl)-1,2,4-triazin-5(4H)-on

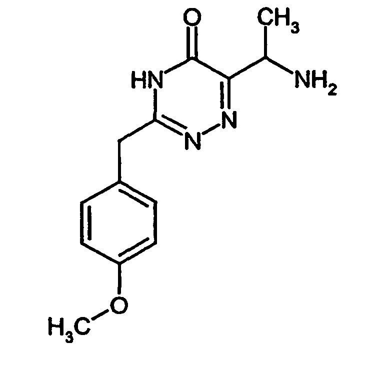

Analog zum **Beispiel 11A** werden 17 g (56,2 mmol) *N*-{1-[3-(4-Methoxybenzyl)-5-oxo-4,5-dihydro-1,2,4-tnazin-6-yl]ethyl}acetamid umgesetzt zu 6-(1-Aminoethyl)-3-(4-methoxybenzyl)-1,2,4-triazin-5(4H)-on.
Ausbeute: 5 g (34,2 % d. Th.)
¹H-NMR (Methanol-d₄): δ/ppm 1,55 (d, 3H), 3,74 (s, 3H), 3,84 (s, 2H), 4,51 (q, 1 H), 6,83 (d, 2H), 7,24 (d, 2H).

### Beispiel 14A

*N*-{1-[3-(3,4-Dimethoxybenzyl)-5-oxo-4,5-dihydro-1,2,4-triazin-6-yl]ethyl}acetamid

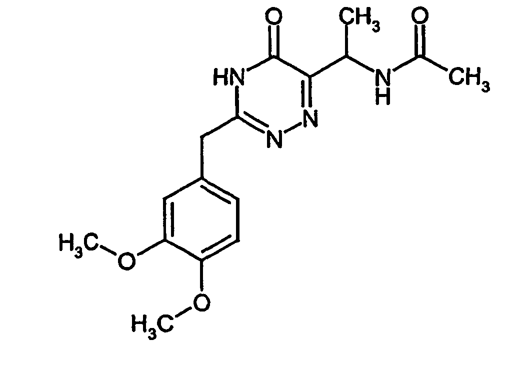

Analog zum **Beispiel 10A** werden 20,0 g (86,7 mmol) 2-(3,4-Dimethoxyphenyl)-ethanamidin Hydrochlorid mit 5,21 g (104 mmol) Hydrazinhydrat und 24,3 g (130 mmol) Ethyl 3-(acetylamino)-2-oxobutanoat zu *N*-{1-[3-(3,4-Dimethoxybenzyl)-5-oxo-4,5-dihydro-1,2,4-triazin-6-yl]ethyl}acetamid umgesetzt.
Ausbeute: 15,5 g (77,5% d. Th.)
¹H-NMR (Methanol-d₄): δ/ppm 1,40 (d, 3H), 1,95 (s, 3H), 3,78 (s, 3H), 3,81 (s, 3 H), 3,82 (s, 2H), 5,16 (q, 1H), 6,86-6,97 (m, 3H).

### Beispiel 15A

6-(1-Aminoethyl)-3-(3,4-dimethoxybenzyl)-1,2,4-triazin-5(4H)-on

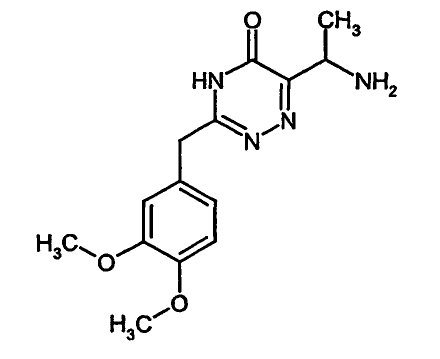

Analog zum **Beispiel 11A** werden 23 g *N*-{1-[3-(3,4-Dimethoxybenzyl)-5-oxo-4,5-dihydro-1,2,4-triazin-6-yl]ethyl}acetamid umgesetzt zu 6-(1-Aminoethyl)-3-(3,4-dimethoxybenzyl)-1,2,4-triazin-5(4H)-on.
Ausbeute: 10,1 g (50,4% d. Th.)
¹H-NMR (Methanol-d₄): δ/ppm 1,55 (d, 3H), 3,78 (s, 3H), 3,80 (s, 3H), 3,83 (s, 2H), 4,52 (q, 1H), 6,83-6,98 (m, 3H).

### Beispiel 16A

*N*-{1-[3-(3-Ethoxy-4-methoxybenzyl)-5-oxo-4,5-dihydro-1,2,4-triazin-6-yl]ethyl}acetamid 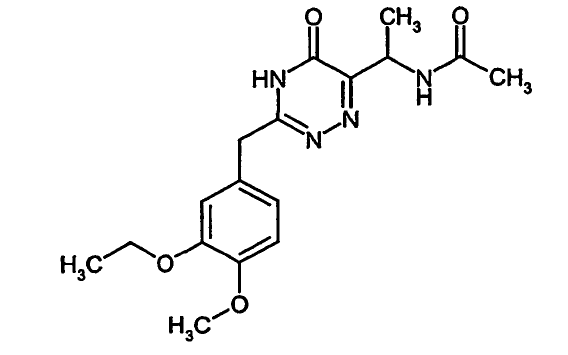 Analog zum **Beispiel 10A** werden 979 mg (4,00 mmol) 2-(3-Ethoxy-4-methoxyphenyl)ethanamidin Hydrochlorid mit 200 mg (4,00 mmol) Hydrazinhydrat und 1,12 g (6,00 mmol) Ethyl 3-(acetylamino)-2-oxobutanoat zu *N*-{1-[3-(3,4-Dimethoxybenzyl)-5-oxo-4,5-dihydro-1,2,4-triazin-6-yl]ethyl}acetamid umgesetzt.
Ausbeute: 540 mg (38,2 % d. Th.)
¹H-NMR (Methanol-d₄): δ/ppm 1,37-1,41 (m, 6H), 1,94 (s, 3H), 3,80 (s, 3H), 3,82 (s, 2H), 4,05 (q, 2H), 5,11 (q, 1H), 6,85-6,96 (m, 3H).

### Beispiel 17A

6-(1-Ammoethyl)-3-(3-ethoxy-4-methoxybenzyl)-1,2,4-triazin-5(4H)-on

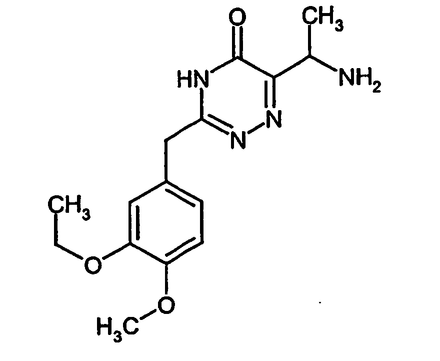

Analog zum **Beispiel 11A** werden 540 mg (1,56 mmol) *N*-{1-[3-(3-Ethoxy-4-methoxybenzyl)-5-oxo-4,5-dihydro-1,2,4-triazin-6-yl]ethyl}acetamid umgesetzt zu 6-(1-Aminoethyl)-3-(3-ethoxy-4-methoxybenzyl)-1,2,4-triazin-5(4H)-on.
Ausbeute: 491 mg (88,0 % d. Th.)
¹H-NMR (Methanol-d₄: δ/ppm 1,39 (t, 3H), 1,59 (d, 3H), 3,81 (s, 3H), 3,92 (s, 2H), 4,06 (q, 2H), 4,58 (q, 1H), 6,91-7,00 (m, 3H).

### Beispiel 18A

*N*-{1-[3-(1-Naphthylinethyl)-S-oxo-4,5-dihydro-1,2,4-triazin-6-yl]ethyl}acetamid 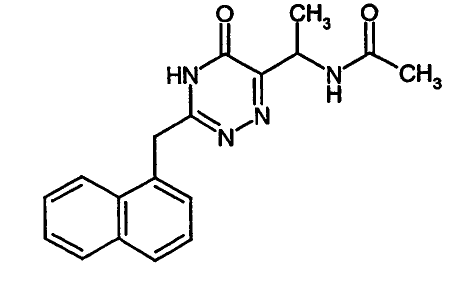 Analog zum **Beispiel 10A** werden 1,67 g (8,00 mmol) 2-(1-Naphthyl)ethanamidin Hydrochlorid mit 401 mg (8,00 mmol) Hydrazinhydrat und 2,70 g (14,4 mmol) Ethyl 3-(acetylamino)-2-oxobutanoat zu *N*-{1-[3-(1-Naphthylmethyl)-5-oxo-4,5-dihydro-1,2,4-triazin-6-yl]ethyl}acetamid umgesetzt.
Ausbeute: 1,9 g Rohausbeute

### Beispiel 19A

6-(1-Aminoethyl)-3-(1-naphthylmethyl)-1,2,4-triazin-5(4H)-on

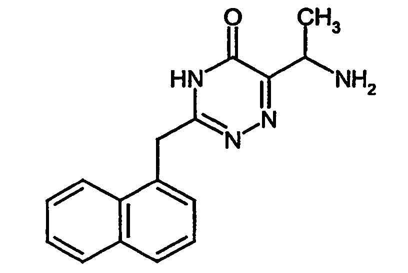

Analog zum **Beispiel 11A** werden 730 mg (2,26 mmol) *N*-{1-[3-(1-Naphthylmethyl)-5-oxo-4,5-dihydro-1,2,4-triazin-6-yl]ethyl}acetamid umgesetzt zu 6-(1-Aminoethyl)-3-(1-naphthyhnethyl)-1,2,4-triazin-5(4H)-on.
Ausbeute: 655 mg (85,0 % d. Th.)
Schmelzpunkt: 180°C
¹H-NMR (DMSO-d₆): δ/ppm 1,43 (d, 3H), 4,28-4,48 (q, 1H), 4,50 (s, 2H), 7,38-8,30 (m, 7H).

### Beispiel 20A

*N*-{1-[5-Oxo-3-(6-Chinolinylmethyl)-4,5-dihydro-1,2,4-triazin-6-yl]ethyl}acetamid 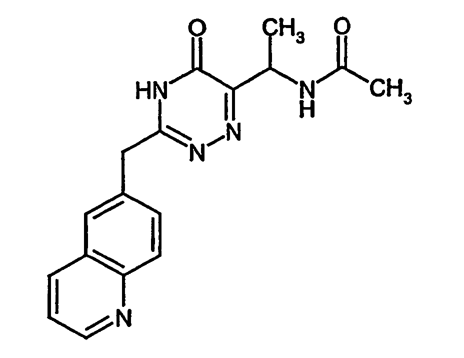 Analog zum **Beispiel 10A** werden 3,5 g (15,8 mmol) 2-(6-Chinolinyl)ethanamidin Hydrochlorid mit 950 mg (19,0 mmol) Hydrazinhydrat und 4,43 g (23,7 mmol) Ethyl 3-(acetylamino)-2-oxobutanoat zu *N*-{1-[5-oxo-3-(6-Chinolinylmethyl)-4,5-dihydro-1,2,4-triazin-6-yl]ethyl}acetamid umgesetzt.
Ausbeute: 3,4 g (55,2% d. Th.)
¹H-NMR (DMSO-d₆): δ/ppm 1,26 (d, 3H), 1,81 (s, 3H), 4,11 (s, 2H), 4,95 (q, 1H), 7,48-8,49 (m, 6H).

### Beispiel 21A

6-(1-Aminoethyl)-3-(6-chinolinylmethyl)-1,2,4-triazin-5(4H)-on

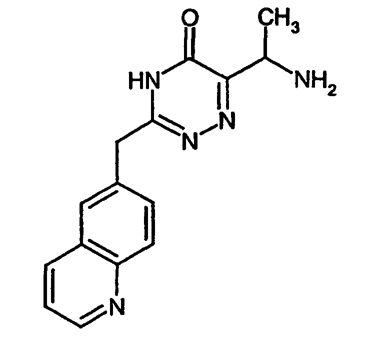

Analog zum **Beispiel 11A** werden 500 mg (1,55 mmol) *N*-{1-[5-oxo-3-(6-Chinolinylmethyl)-4,5-dihydro-1,2,4-triazin-6-yl]ethyl}acetamid umgesetzt zu 6-(1-Aminoethyl)-3-(6-quinolinylmethyl)-1,2,4-triazin-5(4H)-on.
Ausbeute: 360 mg (82,8 % d. Th.)
¹H-NMR (DMSO-d₆): δ/ppm 1,38 (d, 3H), 3,95 (s, 2H), 4,24 (q, 1H), 7,46-8,84 (m, 6H).

### Beispiel 22A

Ethyl 3-(acetylamino)-2-oxopentanoat

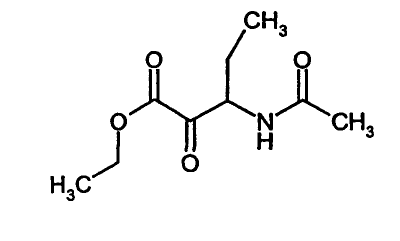

9,2 g (63,4 mmol) Acetyl-2-aminobuttersäure werden in 120 ml Tetrahydrofuran aufgenommen und mit 15,0 g (190 mmol) Pyridin und einer Spatelspitze *N,N-*Dimethylaminopyridin am Rückfluß erhitzt. In der Siedehitze werden 17,3 g (127 mmol) Oxalsäureesterchlorid zugetropft. Das Gemisch wird im Anschluß noch so lange am Rückfluß erhitzt, bis keine Gasentwicklung mehr beobachtet werden kann. Nach dem Erkalten wird der Ansatz auf Eiswasser gegeben und die organische Phase in Essigsäureethylester extrahiert. Die getrocknete organische Phase wird eingeengt und in Ethanol gelöst direkt weiter umgesetzt.

### Beispiel 23A

*N*-{1-[3-(3,4-Dimethoxybenzyl)-5-oxo-4,5-dihydro-1,2,4-triazin-6-yl]propyl} acetamid

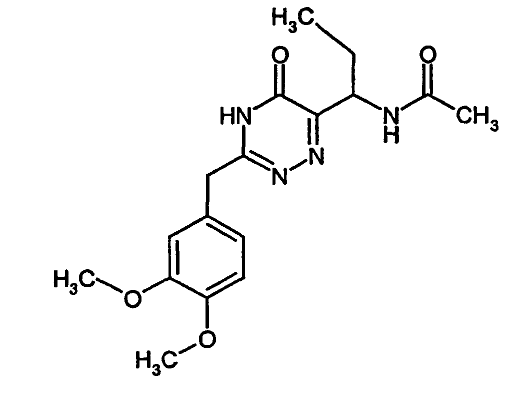

Analog zum **Beispiel 10A** werden 9,75 g (42,3 mmol) 2-(3,4-Dimethoxyphenyl)-ethanamidin Hydrochlorid mit 2,54 g (50,7 mmol) Hydrazinhydrat und 12,8 g (63,4 mmol) Ethyl 3-(acetylamino)-2-oxopentanoat zu *N*-{1-[3-(3,4-Dimethoxybenzyl)-5-oxo-4,5-dihydro-1,2,4-triazin-6-yl]propyl}acetamid umgesetzt.
Ausbeute: 2,05 g (14,0 % d. Th.)
¹H-NMR (Methanol-d₄): δ/ppm 0,96 (t, 3H), 1,63-1,99 (m, 2H), 1,96 (s, 3H), 3,80 (s, 3H), 3,82 (s, 3H), 3,84 (s, 2H), 4,98 (q, 1H), 6,86-6,97 (m, 3H).

### Beispiel 24A

6-(1-Aminopropyl)-3-(3,4-dimethoxybenzyl)-1,2,4-triazin-5(4H)-on

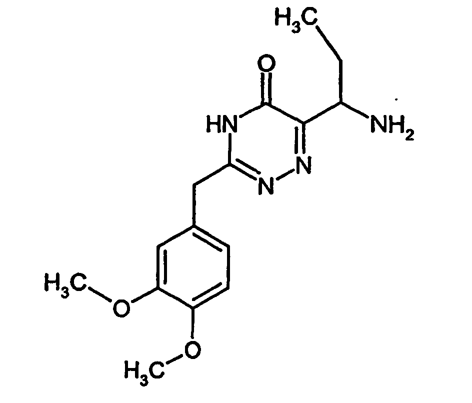

Analog zum **Beispiel 11A** werden 2,05 g (5,91 mmol) *N*-{1-[3-(3,4-Dimethoxybenzyl)-5-oxo-4,5-dihydro-1,2,4-triazin-6-yl]propyl}acetamid umgesetzt zu 6-(1-Aminopropyl)-3-(3,4-dimethoxybenzyl)-1,2,4-triazin-5(4H)-on.
Ausbeute: 2,15 g (67% d. Th.)
¹H-NMR (Methanol-d₄): δ/ppm 1,02 (t, 3H), 1,90-2,13 (m, 2H), 3,80 (s, 3H), 3,83 (s, 3H), 3,86 (s, 2H), 4,44 (m, 1H), 6,91-6,70 (m, 3H).

### Beispiel 25A

N-[1-(3-{4-Methyl-3-[(methylamino)sulfonyl]benzyl}-5-oxo-4,5-dihydro-1,2,4-triazin-6-yl)ethyl]acetamid

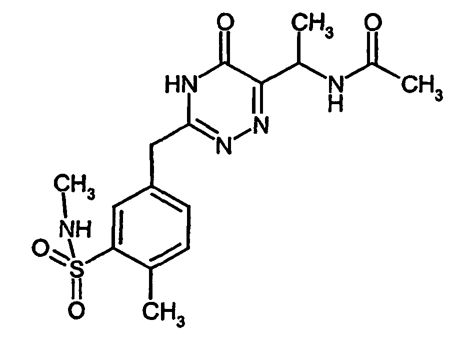

Zu 56,6 g (485 mmol) eisgekühlter Chlorsulfonsäure werden 13,9 g (48,5 mmol) *N*-{1-[3-(4-Methylbenzyl)-5-oxo-4,5-dihydro-1,2,4-triazin-6-yl]ethyl}acetamid in 50 ml Dichlormethan zugetropft und im Anschluß 2 h, unter Erwärmen auf Raumtemperatur, gerührt. Dann wird das Gemisch auf 500 ml Dichlormethan/Eiswasser 1:5 gegeben und das Produkt in Dichlormethan extrahiert. Die getrocknete organische Phase wird mit 55 ml einer 2 M Lösung von Methylamin in Tetrahydrofuran versetzt und 10 min. gerührt. Dann wird mit 1 N Salzsäure neutralisiert, vom Lösemittel eingeengt und chromatographiert mit Laufmittel Dichlormethan/Methanol 20/1.
Ausbeute: 8,25 g (44,8% d. Th.)
¹H-NMR (200 MHz, Methanol-d₄): δ/ppm 1,40 (d, 3H), 1,94 (s, 3H), 2,49 (s, 3H), 2,58 (s, 3H), 3,97 (s, 2H), 5,04-5,17 (m, 1H), 7,33-7,92 (m, 3H).

### Beispiel 26A

5-{[6-(1-Aminoethyl)-5-oxo-4,5-dihydro-1,2,4-triazin-3-yl]methyl}-N,2-dimethylbenzenesulfonamid

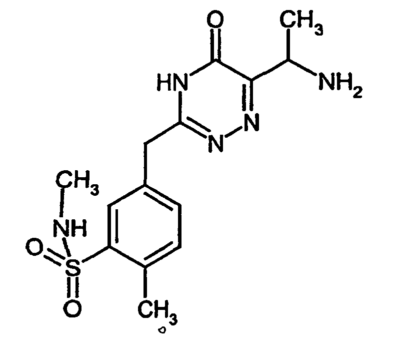

Analog zum **Beispiel 11A** werden 10,2 g (26,9 mmol) N-[1-(3-{4-Methyl-3-[(methylamino)sulfonyl]benzyl}-5-oxo-4,5-dihydro-1,2,4-triazin-6-yl)ethyl]acetamid umgesetzt zu 5-{[6-(1-Aminoethyl)-5-oxo-4,5-dihydro-1,2,4-triazin-3-yl]methyl}-N,2-dimethylbenzenesulfonamid.
Ausbeute: 4,4 g (48,5 % d. Th.)
¹H-NMR (Methanol-d₄): δ/ppm 1,55 (d, 3H), 2,51 (s, 3H), 2,57 (s, 3H), 3,97 (s, 2 H), 4,54 (q, 1H), 7,27-7,89 (m, 3H).

### Beispiel 27A

Natrium (2E)-4-methoxy-4-oxo-2-buten-2-olat

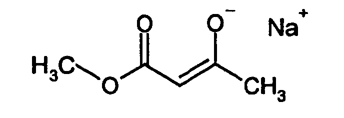

60 g einer 30 %igen Natriumhydrid-Suspension in Mineralöl (744 mmol NaH) werden in Inertgasatmospäre in 250 ml trockenem THF suspendiert. 86,4 g (744 mmol) Acetessigsäuremethylester in 200 ml THF werden langsam zugetropft, wobei der entstehende Wasserstoff direkt in die Abluft geleitet wird. Nach erfolgtem Zutropfen wird eine halbe Stunde am Rückfluß erhitzt und dann abgekühlt. Das Lösemittel wird im Vakuum abgezogen und der zurückbleibende Feststoff mit Diethylether gewaschen.
Ausbeute: 81,9 g (79,7% d. Th.)
Festpunkt: Substanz zersetzt sich bei 200°C.

### Beispiel 28A

Methyl-2-acetyl-5-phenylpentanoat 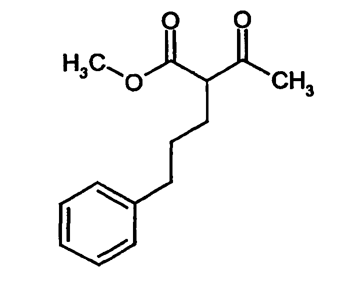 85 g (616 mmol) in 1,3-Dimethyltetrahydro-2(1H)-pyrimidon suspendiertes Natrium-(2E)-4-methoxy-4-oxo-2-buten-2-olat und 3,50 g (21,1 mmol) Kaliumjodid werden tropfenweise mit 129 g (646 mmol) 1-Brom-3-phenylpropan versetzt und 1 h am Rückfluß bei 80°C gerührt. Im Anschluß wird das abgekühlte Gemisch auf Eiswasser gegeben und mit Diethylether extrahiert. Die Etherphase wird mit Natriumthiosulfatiösung gewaschen, getrocknet, eingeengt und chromatographiert. Als Laufmittel dient Cyclohexan mit steigendem Essigsäureethylesteranteil.
Ausbeute: 57,0 g (39,5 % d. Th.)
¹H-NMR (CDCl₃): δ/ppm 1,55-1,70 (m, 2H), 1,85-1,98 (m, 2H), 2,20 (s, 3H), 2,65 (t, 3H), 3,43 (t, 1H), 3,73 (s, 3H), 7,11-7,33 (m, 5H).

### Beispiel 29A

2-Acetyl-5-phenylpentansäure

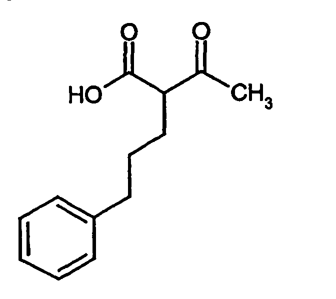

1,00 g (4,45 mmol) Methyl-2-acetyl-5-phenylpentanoat wird in 10 ml Dioxan gelöst und auf 0°C gekühlt. Unter Kühlung erfolgt die Zugabe von 8,5 ml einer 1 M Natriumhydroxidlösung. Nach 5 h Reaktionszeit wird der Ansatz eingeengt, mit 10 ml Essigsäureethylester und 10 ml Wasser versetzt und ausgeschüttelt. Die Wasserphase wird gewonnen, auf 0°C abgekühlt und unter Kühlung langsam mit 1 N Salzsäure versetzt bis pH 1 erreicht ist. Dann wird mit Dichlormethan extrahiert. Die Dichlormethanphase wird getrocknet und ohne einzuengen direkt weiter umgesetzt.
Ausbeute: 560 mg (59,6 % d. Th.)
¹H-NMR (CDCl₃): δ/ppm 1,58-1,73 (m, 2H), 1,87-1,98 (m, 2H), 2,25 (s, 3H), 2,63 (t, 3H), 3,50 (t, 1H), 7,14-7,33 (m, 5H), 12,39 (s, COOH).

### Beispiel 30A

Methyl 2-(1-hydroxyethyl)-5-phenylpentanoat

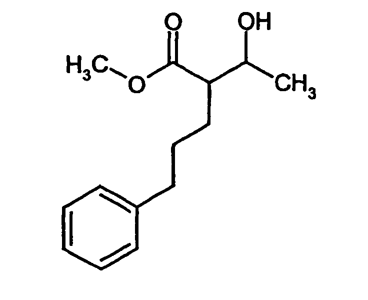

5,50 g (23,5 mmol) Methyl 2-acetyl-5-phenylpentanoat werden in 100 ml Methanol vorgelegt und eisgekühlt. 0,44 g (11,7 mmol) Natriumborhydrid werden portionsweise zugegeben und noch 1 h nachgerührt. Dann wird der Ansatz vom Lösemittel evakuiert, in Diethylether aufgenommen und mit 1 N Salzsäure gewaschen. Die organische Phase wird dann wiederum eingeengt und *flash*-chromatographiert mit Laufmittel Petrolether/Essigsäureethylester 10/1.
Ausbeute: 4,5 g (81,1 % d. Th.)
¹H-NMR (CDCl₃, Diastereomerengemisch): δ/ppm 1,21 (d, 3H), 1,57-1,72 (m, 4H), 2,43 (m,1H), 2,61 (t, 2H), 3,71 (s, 3H), 3,82-4,00 (m, 1H), 7,12-7,34 (m, 5H).

### Beispiel 31A

2-(1-Hydroxyethyl)-5-phenylpentansäure

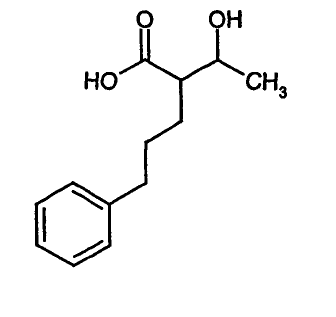

Analog **Beispiel 29A** werden 4,00 g (16,9 mmol) Methyl 2-(1-hydroxyethyl)-5-phenylpentanoat mit 34,0 ml einer 1 M Natriumhydroxidlösung zu 2-(1-Hydroxyethyl)-5-phenylpentansäure umgesetzt.
Ausbeute: 3,56 g (94,6 % d. Th.)
¹H-NMR (CDCl₃, Diastereomerengemisch): δ/ppm 1,18-1,30 (d, 3H), 1,56-1,83 (m, 4H), 2,36-2,50 (m,1H), 2,57-2,71 (m, 2H), 3,89-4,02 (m, 1H), 7,11-7,33 (m, 5H).

### Beispiel 32A

Methyl-(4E)-2-acetyl-5-phenyl-4-pentenoat

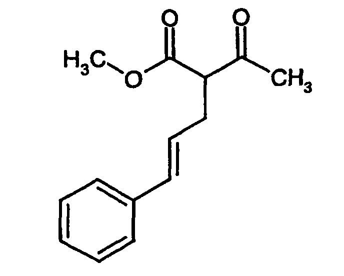

Analog **Beispiel 28A** werden 10 g (72,4 mmol) Natrium-(2E)-4-methoxy-4-oxo-2-buten-2-olat und 0,40 g (2,41 mmol) Kaliumjodid mit 14,3 g (72,4 mmol) [(1E)-3-Bromo-1-propenyl]benzol zu Methyl-(4E)-2-acetyl-5-phenyl-4-pentenoat umgesetzt.
Ausbeute: 8,30 g (49,4 % d. Th.)
LC-MS : Retentionzeit 4,60 min., m/z 233 [M+H]⁺
- HPLC-Parameter: Lsg. A Acetonitril
Lsg. B 0,6 g 30% ige HCl/l Wasser
Fluß 0,6 ml/min.; Säulenofen 50°C;
Säule Symmetry C18 2,1x150 mm

| Gradient | Zeit [min] | %A | %B | Fluß [ml/min] |
|---|---|---|---|---|
| | 0 | 10 | 90 | 0,6 |
| | 4 | 90 | 10 | 0,6 |
| | 9 | 90 | 10 | 0,8 |

### Beispiel 33A

Methyl-(4E)-2-(1-hydroxyethyl)-5-phenyl-4-pentenoat

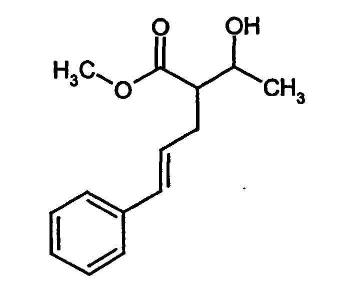

Analog **Beispiel 30A** werden 8,00 g (34,44 mmol) Methyl (4E)-2-acetyl-5-phenyl-4-pentenoat werden mit 0,72 g (18,9 mmol) Natriumborhydrid zu Methyl (4E)-2-(1-hydroxyethyl)-5-phenyl-4-pentenoat umgesetzt.
Ausbeute: 4,5 g (81,1 % d. Th.)
Masse (DCI, NH₃): m/z = 235 [M+H]⁺, 252,1 [M+NH₄]⁺.

### Beispiel 34A

(4E)-2-(1-Hydroxyethyl)-5-phenyl-4-pentenonsäure

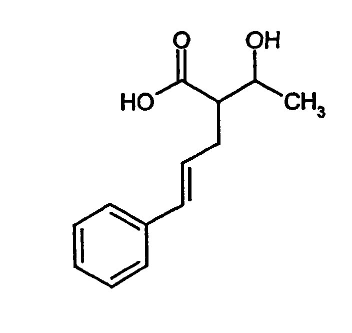

Analog **Beispiel 29A** werden 3,80 g (16,2 mmol) Methyl (4E)-2-(1-hydroxyethyl)-5-phenyl-4-pentenoat mit 24,0 ml einer 1 M Natriumhydroxidlösung zu (4E)-2-(1-Hydroxyethyl)-5-phenyl-4-pentenonsäure umgesetzt.
Ausbeute: 2,30 g (64,4% d. Th.)
¹H-NMR (CDCl₃): δ/ppm 1,26-1,35 (m, 3H), 2,49-2,71 (m, 3H), 3,98-4,17 (m,1H), 6,11-6,26 (m, 1H), 6,33-6,54 (m, 1H), 7,17-7,37 (m, 5H).

### Beispiel 35A

Methyl-2-acetyl-5-cyclohexylpentanoat

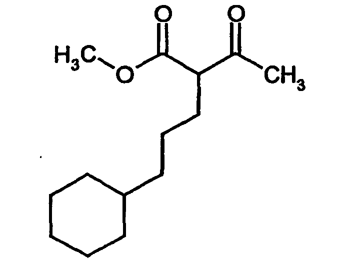

Analog **Beispiel 28A** werden 10 g (72,4 mmol) Natrium-(2E)-4-methoxy-4-oxo-2-buten-2-olat und 0,4 g (2,41 mmol) Kaliumjodid mit 14,9 g (72,4 mmol) (3-Bromopropyl)cyclohexan zu Methyl 2-acetyl-5-cyclohexylpentanoat umgesetzt.
Ausbeute: 7,3 g (42,0 % d. Th.)
¹H-NMR (CDCl₃): δ/ppm 0,73-0,98 (m, 2H), 1,04-1,41 (m, 8H), 1,53-1,92 (m, 7 H), 2,20 (s, 3H), 3,42 (t, 1H), 3,74 (s, 3H).

### Beispiel 36A

2-Acetyl-5-cyclohexylpentansäure

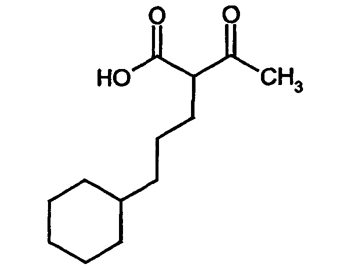

Analog **Beispiel 29A** werden 450 mg (2,08 mmol) Methyl 2-acetyl-5-cyclohexylpentanoat mit 1,5 ml einer 3,5 M Kaliumhydroxidlösung umgesetzt zu 2-Acetyl-5-cyclohexylpentansäure in Dichlormethan.

### Beispiel 37A

Methyl-2-acetyl-2-methyl-5-phenylpentanoat

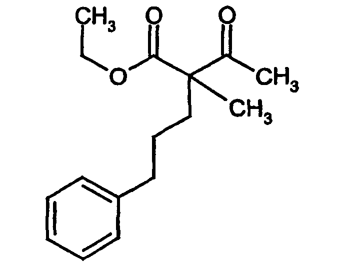

Analog **Beispiel 28A** werden 24 g (144 mmol) Natrium-(2E)-4-methoxy-4-oxo-2-buten-2-olat und 0,70 g (4,22 mmol) Kaliumjodid mit 29,9 g (150 mmol) 3-Phenylpropylbromid zu Methyl-2-acetyl-2-methyl-5-phenylpentanoat umgesetzt.
Ausbeute: 29,5 g (77,7 % d. Th.)
¹H-NMR (CDCl₃): δ/ppm 1,23 (t, 3H), 1,32 (s, 3H), 1,42-1,60 (m, 2H), 1,70-1,98 (m, 2H), 2,09 (s, 3H), 2,62 (t, 2H), 4,17 (q, 2H), 7,12-7,32 (m, 5H).

### Beispiel 38A

2-Acetyl-2-methyl-5-phenylpentansäure

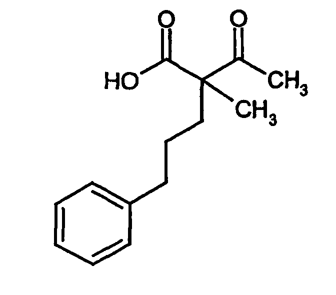

Analog **Beispiel 29A** werden 4,90 g (18,7 mmol) Methyl 2-acetyl-2-methyl-5-phenylpentanoat mit 10,0 ml einer 3,5 M Kaliumhydroxidlösung zu 2-Acetyl-2-methyl-5-phenylpentansäure umgesetzt.

### Beispiel 39A

2-(1-Hydroxyethyl)-N-{1-[3-(4-methylbenzyl)-5-oxo-4,5-dihydro-1,2,4-triazin-6-yl]ethyl}-5-phenylpentansäureamid

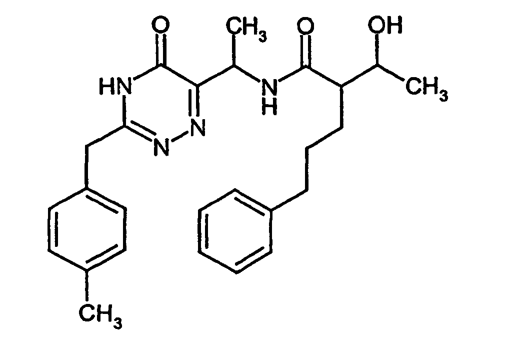

1,09 g (4,91 mmol) 2-(1-Hydroxyethyl)-5-phenylpentansäure werden mit 660 mg (4,61 mmol) 1-Hydroxy-1H-benzotriazol und 990 mg (9,82 mmol) 4-Methylmorpholin versetzt und auf -20°C gekühlt. Nach Zugabe von 940 mg (4,61 mmol) N'-(3-Dimethylaminopropyl)-N-ethylcarbodiimid Hydrochlorid wird 30 min gerührt. Dabei wird das Kühlbad entfernt. Dann werden nach erneutem Kühlen auf -20°C 1,00 g (4,09 mmol) 6-(1-Aminoethyl)-3-(4-methoxybenzyl)-1,2,4-triazin-5(4H)-on hinzugefügt und das Gemisch über Nacht unter Erwärmen auf Raumtemperatur gerülut. Zur Aufarbeitung wird die Dichlormethanphase mit IN Kaliumhydrogensulfatlösung und anschließend mit gesättigter Natriumhydrogencarbonatlösung gewaschen. Die getrocknete organische Phase wird eingeengt und chromatographiert mit Laufmittel Dichlormethan/Methanol 100/1 bis 30/1.
Ausbeute: 800 mg (44,8% d. Th.)

### Beispiel 40A

2-Acetyl-N-{1-[3-(4-methylbenzyl)-5-oxo-4,5-dihydro-1,2,4-triazin-6-yl]ethyl}-5-phenylpentansäureamid

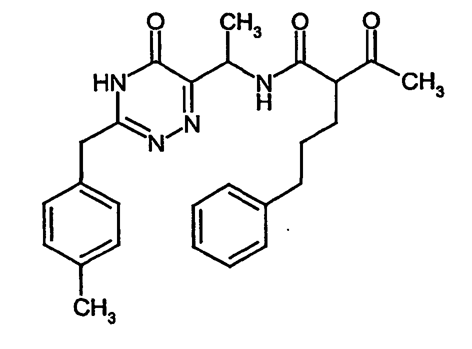

270 mg (2,14 mmol) Oxalsäuredichlorid in 10 ml Dichlormethan werden bei -70°C tropfenweise mit 360 mg (4,64 mmol) Dimethylsulfoxid versetzt. Es wird 30 min bei -70°C gerührt, dann erfolgt die Zugabe von 800 mg (1,78 mmol) 2-(1-Hydroxyethyl)-N-{1-[3-(4-methylbenzyl)-5-oxo-4,5-dihydro-1,2,4-triazin-6-yl]ethyl}-5-phenylpentansäureamid. Nach weiteren 30 min, während derer die Temperatur im Ansatz auf ca. -60°C steigt, werden 900 mg (8,92 mmol) Triethylamin zugegeben und im Anschluß das Kühlbad entfemt. Ist die Ansatztemperatur fast auf Raumtemperatur erwärmt werden 10 ml Wasser zugefügt und, nach kurzem Rühren, die Phasen getrennt. Die getrocknete organische Phase wird chromatographiert in Dichlormethan/Methanol 50/1.
Ausbeute: 550 mg (69,1% d. Th.)
¹H-NMR (Methanol-d₄, Diastereomerengemisch): δ/ppm 1,39-1,46 (m, 3H), 1,48-1,61 (m, 2H), 1,72-1,81 (m, 2H), 2,15 und 2,16 (je s, 3H), 2,30 (s, 3H), 2,55-2,64 (m, 2H), 3,47 (m,1H), 3,84 und 3,85 (je s, 2H), 5,12 (m, 1H), 7,07-7,26 (m, 9H).

### Beispiel 41A

2-(1-Hydroxyethyl)-N- {1-[3-(4-methoxybenzyl)-5-oxo-4,5-dihydro-1,2,4-triazin-6-yl]ethyl}-5-phenylpentansäureamid

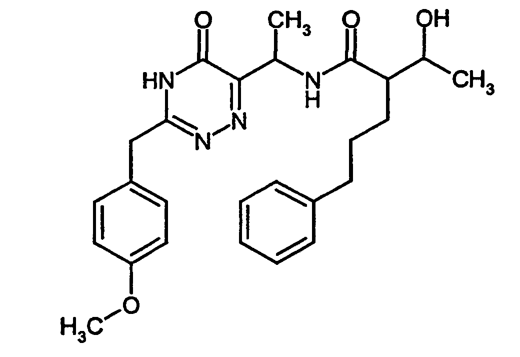

1,02 g (4,61 mmol) 2-(1-Hydroxyethyl)-5-phenylpentansäure werden analog **Beispiel** 39A mit 620 mg (4,61 mmol) 1-Hydroxy-1H-benzotriazol, 930 mg (9,22 mmol) 4-Methylmorpholin, 880 mg (4,61 mmol) N'-(3-Dimethylaminopropyl)-N-ethylcarbodiimid Hydrochlorid und 1,00 g (3,84 mmol) 6-(1-Aminoethyl)-3-(4-methoxybenzyl)-1,2,4-triazin-5(4H)-on zu 2-(1-Hydroxyethyl)-N-{1-[3-(4-methoxybenzyl)-5-oxo-4,5-dihydro-1,2,4-triazin-6-yl]ethyl}-5-phenylpentansäureamid umgesetzt.
Ausbeute: 800 mg (44,8% d. Th.)
LC-MS : Retensionszeit 3,36, 3,46 und 3,56 min., m/z 465,4 [M+H]⁺
- LC-Parameter: Lsg. A Acetonitril + 0,1% Ameisensäure
Lsg. B Wasser + 0,1 %Ameisensäure
Säulenofen 40°C;
Säule Symmetry C18 50 mm x 2,1 mm

| Gradient | Zeit [min] | %A | %B | Fluß [ml/min] |
|---|---|---|---|---|
| | 0 | 10 | 90 | 0,5 |
| | 4 | 90 | 10 | 0,5 |
| | 6 | 90 | 10 | 0,5 |
| | 6,1 | 10 | 90 | 1,0 |
| | 7,5 | 10 | 90 | 0,5 |
| | 9 | 90 | 10 | 0,8 |

### Beispiel 42A

2-Acetyl-N-{1-[3-(4-methoxybenzyl)-5-oxo-4,5-dihydro-1,2,4-triazin-6-yl]ethyl}-5-phenylpentansäureamid

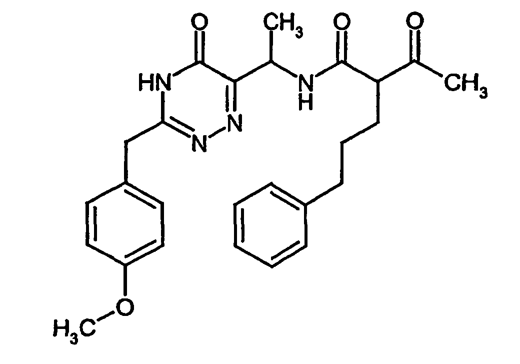

Analog **Beispiel 40A** werden 250 mg (1,94 mmol) Oxalsäuredichlorid, 330 mg (4,2 mmol) Dimethylsulfoxid, 750 mg (1,61 mmol) 2-(1-Hydroxyethyl)-N-{1-[3-(4-methoxybenzyl)-5-oxo-4,5-dihydro-1,2,4-triazin-6-yl]ethyl}-5-phenylpentansäureamid und 820 mg (8,07 mmol) Triethylamin zu 2-Acetyl-N-{1-[3-(4-methoxybenzyl)-5-oxo-4,5-dihydro-1,2,4-triazin-6-yl]ethyl}-5-phenylpentansäureamid umgesetzt.
Ausbeute: 320 mg (42,9 % d. Th.)
¹H-NMR (Methanol-d₄, Diastereomerengemisch): δ/ppm 1,44 (m, 3H), 1,48-1,61 (m, 2H), 1,71-1,82 (m, 2H), 2,15 (s, 3H), 2,54-2,64 (m, 2H), 3,46 (m,1H), 3,75 (s, 3H), 3,82 (s, 2H), 5,14 (m, 1H), 6,82-7,28 (m, 9H).

### Beispiel 43A

2-(1-Hydxoxyethyl)-N-{1-[3-(3, 4-dimethoxybenzyl)-5-oxo-4,5-dihydro-1,2,4-triazin-6-yl]ethyl}-5-phenylpentansäureamid

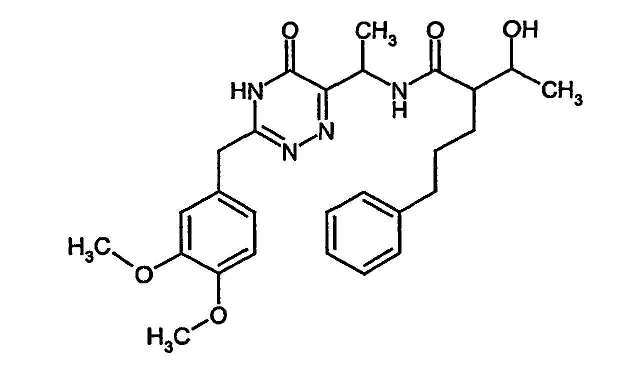

1,01 g (4,55 mmol) 2-(1-Hydroxyethyl)-5-phenylpentansäure wird analog **Beispiel** 39A mit 0,67 g (4,96 mmol) 1-Hydroxy-1H-benzotriazol, 1,05 g (10,3 mmol) 4-Methylmorpholin, 0,95 g (4,96 mmol) N'-(3-Dimethylaminopropyl)-N-ethylcarbodümid Hydrochlorid und 1,20 g (4,13 mmol) 6-(1-Aminoethyl)-3-(3,4-dimethoxybenzyl)-1,2,4-triazin-5(4H)-on zu 2-(1-Hydmxyethyl)-N-{1-[3-(3, 4-dimethoxybenzyl)-5-oxo-4,5-dihydro-1,2,4-triazin-6-yl]ethyl} 5-phenylpentansäureamid umgesetzt.
Ausbeute: 1,45 g (70,9 % d. Th.)
¹H-NMR (Methanol-d₄, Diastereomerengemisch): δ/ppm 1,10-1,18 (m, 3H), 1,40-1,46 (m, 3H), 1,51-1,65 (m, 4H), 2,19-2,29 (m, 1H), 2,50-2,66 (m, 2H), 3,77-3,84 (m, 9H), 5,12 (m, 1H), 6,86-7,25 (m, 8H).

### Beispiel 44A

2-Acetyl-N-{1-[3-(3,4-dimethoxybenzyl)-5-oxo-4,5-dihydro-1,2,4-triazin-6-yl]ethyl}-5-phenylpentansäureamid

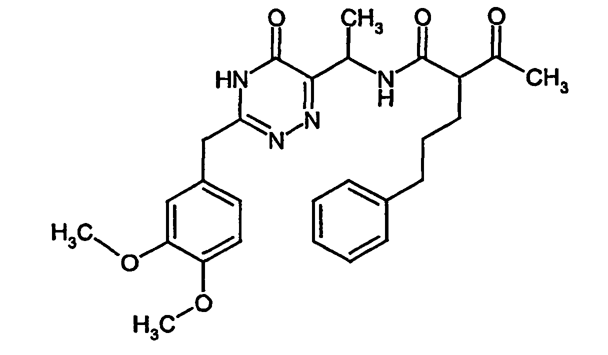

Analog **Beispiel 40A** werden 0,76 g (6,00 mmol) Oxalsäuredichlorid, 1,01 g (12,9 mmol) Dimethylsulfoxid, 1,95 g (3,93 mmol) 2-(1-Hydroxyethyl)-N-{1-[3-(3, 4-dimethoxybenzyl)-5-oxo-4,5-dihydro-1,2,4-triazin-6-yl]ethyl}-5-phenylpentansäureamid und 4,36 g (43,1 mmol) Triethylamin zu 2-Acetyl-N-{1-[3-(3,4-dimethoxybenzyl)-5-oxo-4,5-dihydro-1,2,4-triazin-6-yl]ethyl}-5-phenylpentansäureamid umgesetzt.
Ausbeute: 1,30 g (67,2 % d. Th.)
¹H-NMR (Methanol-d₄, Diastereomerengemisch): δ/ppm 1,45 (m, 3H), 1,51-1,61 (m, 2H), 1,73-1,81 (m, 2H), 2,15 (s, 3H), 2,55-2,63 (m, 2H), 3,47 (m,1H), 3,78-3,85 (m, 8H), 5,14 (m, 1H), 6,85-7,26 (m, 8H).

### Beispiel 45A

(4E)-N-{1-[3-(3,4-Dimethoxybenzyl)-5-oxo-4,5-dihydro-1,2,4-triazin-6-yl]ethyl}-2-(1-hydroxyethyl)-5-phenyl-4-pentenamid

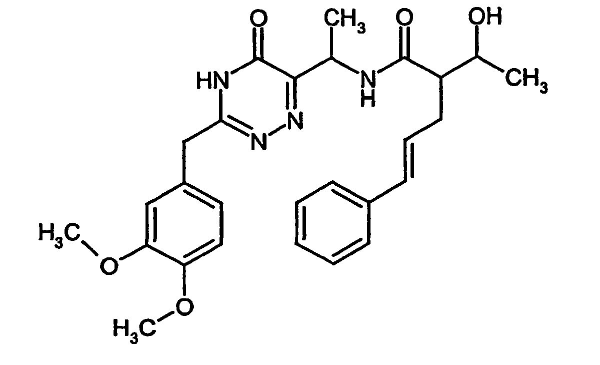

1,14 g (5,17 mmol) (4E)-2-(1-Hydroxyethyl)-5-phenyl-4-pentenonsäure werden analog **Beispiel 39A** mit 0,70 g (5,17 mmol) 1-Hydroxy-1H-benzotriazol, 1,05 g (10,3 mmol) 4-Methybnorpholin, 0,99 g (5,17 mmol) N'-(3-Dimethylaminopropyl)-N-ethylcarbodiimid Hydrochlorid und 0,60 g (2,07 mmol) 6-(1-Aminoethyl)-3-(3,4-dimethoxybenzyl)-1,2,4-triazin-5(4H)-on zu (4E)-N-{1-[3-(3,4-Dimethoxybenzyl)-5-oxo-4,5-dihydro-1,2,4-triazin-6-yl]ethyl}-2-(1-hydroxyethyl)-5-phenyl-4-pentenamid umgesetzt.
Ausbeute: 625 mg (61,4 % d. Th.)
¹H-NMR (Methanol-d₄, Diastereomerengemisch): δ/ppm 1,15-1,43 (m, 6H), 2,31-2,70 (m, 3H), 3,70-3,94 (m, 9H), 5,07-5,23 (m, 1H), 6,11-6,23 (m, 1H), 6,31-6,44 (m, 1H), 6,81-7,34 (m, 8H).

### Beispiel 46A

(4E)-2-Acetyl-N-{1-[3-(3,4-dimethoxybenzyl)-5-oxo-4,5-dihydro-1,2,4-triazin-6-yl]-ethyl}-5-phenyl-4-pentenamid

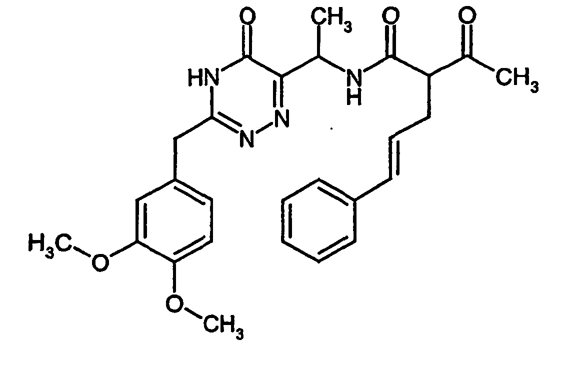

Analog **Beispiel 40A** werden 320 mg (2,50 mmol) Oxalsäuredichlorid, 390 mg (5,00 mmol) Dimethylsulfoxid, 800 mg (1,63 mmol) (4E)-N-{1-[3-(3,4-Dimethoxybenzyl)-5-oxo-4,5-dihydro-1,2,4-triazin-6-yl]ethyl}-2-(1-hydroxyethyl)-5-phenyl-4-pentenamid und 1,82 g (18,0 mmol) Triethylamin zu (4E)-2-Acetyl-N-{1-[3-(3,4-dimethoxybenzyl)-5-oxo-4,5-dihydro-1,2,4-triazin-6-yl]ethyl}-5-phenyl-4-pentenamid umgesetzt.
Ausbeute: 540 mg (67,5 % d. Th.)
¹H-NMR (Methanol-d₄, Diastereomerengemisch): δ/ppm 1,37 und 1,44 (je d, 3H), 2,20 und 2,21 (je s, 3H), 2,52-2,71 (m, 2H), 3,57-3,86 (m, 9H), 5,14 (m, 1H), 6,07-6,19 (m, 1H), 6,35-6,48 (m, 1H), 6,82-7,34 (m, 8H).

### Beispiel 47A

2-Acetyl-5-cyclohexyl-N-{ 1-[3-(3,4-dimethoxybenzyl)-5-oxo-4,5-dihydro-1,2,4-triazin-6-yl]ethyl}pentansäureamid

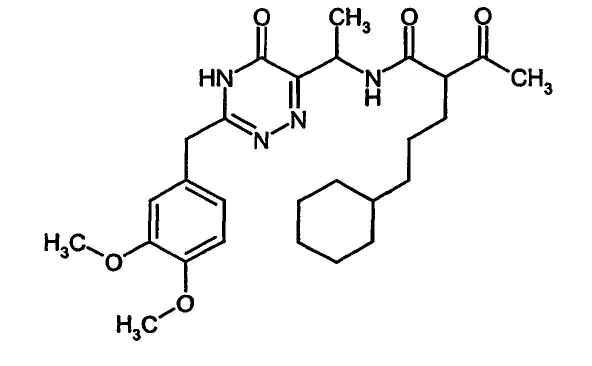

Die Menge 2-Acetyl-5-cyclohexylpentansäure in Dichlormethan aus **Beispiel 36A** wird analog **Beispiel 39A** mit 280 mg (2,08 mmol) 1-Hydroxy-1H-benzotriazol, 610 mg (6,00 mmol) 4-Methylmorpholin, 400 mg (2,08 mmol) N'-(3-Dimethylaminopropyl)-N-ethylcarbodiimid Hydrochlorid und 600 mg (2,08 mmol) 6-(1-Aminoethyl)-3-(3,4-dimethoxybenzyl)-1,2,4-triazin-5(4H)-on zu 2-Acetyl-5-cyclohexyl-N-{1-[3-(3,4-dimethoxybenzyl)-5-oxo-4,5-dihydro-1,2,4-triazin-6-yl]ethyl}-pentansäureamid umgesetzt.
Ausbeute: 248 mg (23,9 % d. Th.)
¹H-NMR (Methanol-d₄, Diastereomerengemisch): δ/ppm 0,65-0,81 (m, 2H), 0,99-1,19 (m, 8H), 1,34 (d, 3H), 1,48-1,64 (m, 7H), 2,06 (s, 3H), 3,33 (m, 1H), 3,66-3,74 (m, 8H), 4,97-5,07 (m, 1H), 6,74-6,88 (m, 3H).

### Beispiel 48A

2-Acetyl-N-{1-[3-(3,4-dimethoxybenzyl)-5-oxo-4,5-dihydro-1,2,4-triazin-6-yl]ethyl}-2-methyl-5-phenylpentansäureamid

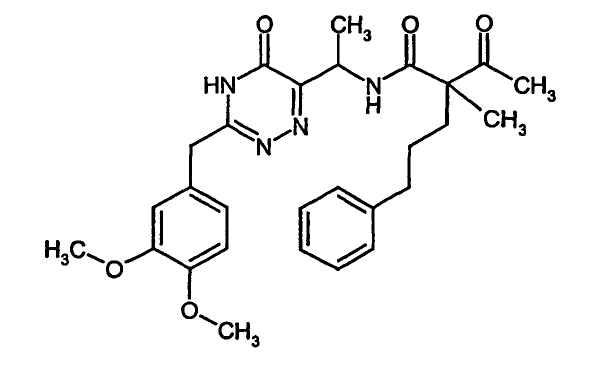

Die Menge 2-Acetyl-2-methyl-5-phenylpentansäure in 20 ml Dichlormethan aus **Beispiel 38A** wird analog **Beispiel 39A** mit 300 mg (2,20 mmol) 1-Hydroxy-1H-benzotriazol, 670 mg (6,60 mmol) 4-Methylmorpholin, 420 mg (2,20 mmol) N'-(3-Dimethylaminopropyl)-N-ethylcarbodümid Hydrochlorid und 620 mg (2,14 mmol) 6-(1-Aminoethyl)-3-(3,4-dimethoxybenzyl)-1,2,4-triazin-5(4H)-on zu 2-Acetyl-N-{1-[3-(3,4-dimethoxybenzyl)-5-oxo-4,5-dihydro-1,2,4-triazin-6-yl]ethyl}-2-methyl-5-phenylpentansäureamid umgesetzt.
Ausbeute: 544 mg (50,1 % d. Th.)
¹H-NMR (Methanol-d₄ Diastereomerengemisch): δ/ppm 1,19 (s, 3H), 1,28-1,41 (m, 5H), 1,60-1,81 (m, 2H), 1,96 und 1,97 (je s, 3H), 2,41-2,54 (m, 2H), 3,66-3,74 (m, 8H), 4,97-5,05 (m, 1H), 6,74-7,14 (m, 8H).

### Beispiel 49A

N-{1-[3-(3,4-Dimethoxybenzyl)-5-oxo-4,5-dihydro-1,2,4-triazin-6-yl]propyl}-2-(1-hydroxyethyl)-5-phenylpentansäureamid

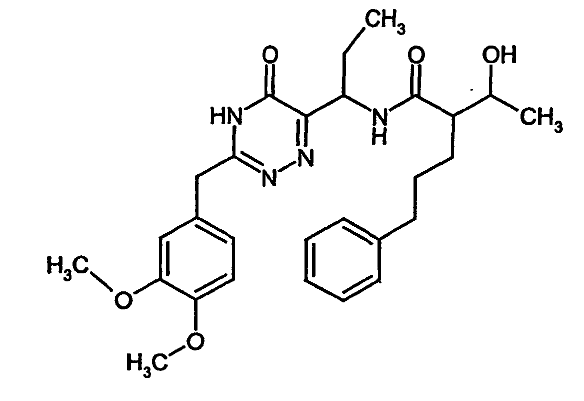

1,44 g (6,50 mmol) 2-(1-Hydroxyethyl)-5-phenylpentansäure werden analog **Beispiel 39A** mit 1,00 g (6,50 mmol) 1-Hydroxy-1H-benzotriazol, 1,97 g (19,5 mmol) 4-Methylmorpholin, 1,25 g (6,350 mmol) N'-(3-Dimethylaminopropyl)-N-ethylcarbodiimid Hydrochlorid und 2,15 g (6,32 mmol) 6-(1-Aminopropyl)-3-(3,4-dimethoxybenzyl)-1,2,4-triazin-5(4H)-on zu N-{1-[3-(3,4-Dimethoxybenzyl)-5-oxo-4,5-dihydro-1,2,4-triazin-6-yl]propyl}-2-(1-hydroxyethyl)-5-phenylpentansäureamid umgesetzt.
Ausbeute: 881 mg (27,4 % d. Th.)
¹H-NMR (Methanol-d₄, Diastereomerengemisch): δ/ppm 0,90-1,03 (m, 3H), 1,09-1,20 (d, 3H), 1,46-1,99 (m, 6H), 2,25 (m, 1H), 2,49-2,66 (m, 2H), 3,66-3,86 (m, 9 H), 5,01 (m, 1H), 6,83-7,26 (m, 8H).

### Beispiel 50A

2-Acetyl-N-{1-[3-(3,4-dimethoxybenzyl)-5-oxo-4,5-dihydro-1,2,4-triazin-6-yl]propyl}-5-phenylpentansäureamid

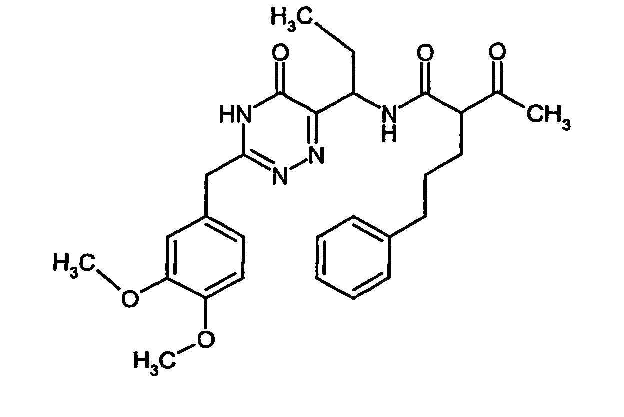

Analog **Beispiel 40A** werden 440 mg (305 mmol) Oxalsäuredichlorid, 550 mg (7,00 mmol) Dimethylsulfoxid, 920 mg (1,82 mmol) N-{1-[3-(3,4-Dimethoxybenzyl)-5-oxo-4,5-dihydro-1,2,4-triazin-6-yl]propyl}-2-(1-hydroxyethyl)-5-phenylpentansäureamid und 1,82 g (18 mmol) Triethylamin zu 2-Acetyl-N-{1-[3-(3,4-dimethoxybenzyl)-5-oxo-4,5-dihydro-1,2,4-triazin-6-yl]propyl}-5-phenylpentansäureamid umgesetzt.
Ausbeute: 537 mg (58,3 % d. Th.)
¹H-NMR (Methanol-d₄, Diastereomerengemisch): δ/ppm 0,93-1,05 (dt, 3H), 1,46-2,04 (m, 6H), 2,14 und 2,16 (je s, 3H), 2,51-2,67 (m, 2H,), 3,51 (m, 1H), 3,76-3,85 (m, 8H), 4,99 (m, 1H), 6,85-7,26 (m, 8H).

### Beispiel 51A

N-{1-[3-(3-Ethoxy-4-methoxybenzyl)-5-oxo-4,5-dihydro-1,2,4-triazin-6-yl]propyl}2-(1-hydroxyethyl)-5-phenylpentansäureamid

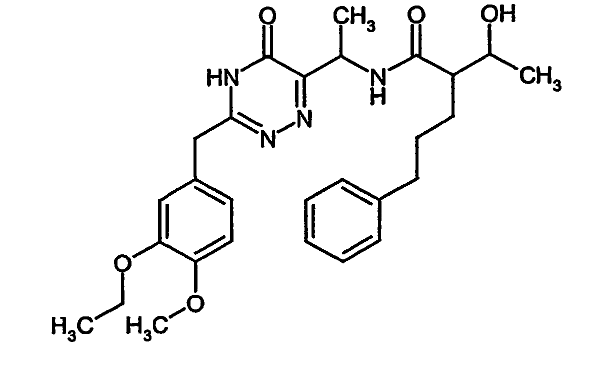

423 mg (1,90 mmol) 2-(1-Hydzoxyethyl)-5-phenylpentansäure werden analog **Beispiel 39A** mit 257 mg (1,90 mmol) 1-Hydroxy-1H-benzotriazol, 328 mg (2,54 mmol) N-Ethyldüsopropylamin, 365 mg (1,90 mmol) N'-(3-Dimethylaminopropyl)-N-ethylcarbodiimid Hydrochlorid und 432 mg (1,27 mmol) 6-(1-Aminopropyl)-3-(3-ethoxy-4-methoxybenzyl)-1,2,4-triazin-5(4H)-on zu N-{1-[3-(3-Ethoxy-4-methoxybenzyl)-5-oxo-4,5-dihydro-1,2,4-triazin-6-yl]propyl}2-(1-hydroxyethyl)-5-phenylpentansäureamid umgesetzt.
Ausbeute: 390 mg (56,9 % d. Th.)
¹H-NMR (Methanol-d₄, Diastereomerengemisch): δ/ppm 1,10-1,19 (m, 3H), 1,34-1,45 (m, 6H), 1,50-1,80 (m, 4H), 2,17-2,28 (m, 1H), 2,51-2,66 (m, 2H), 3,64-3,75 (m, 1H), 3,75-3,85 (m, 5H), 3,99-4,07 (m, 2H), 5,17 (m, 1H), 6,84-7,26 (m, 8H).

### Beispiel 52A

2-Acetyl-N-{1-[3-(3-ethoxy-4-methoxybenzyl)-5-oxo-4,5-dihydro-1,2,4-triazin-6-yl]propyl}-5-phenylpentansäureamid

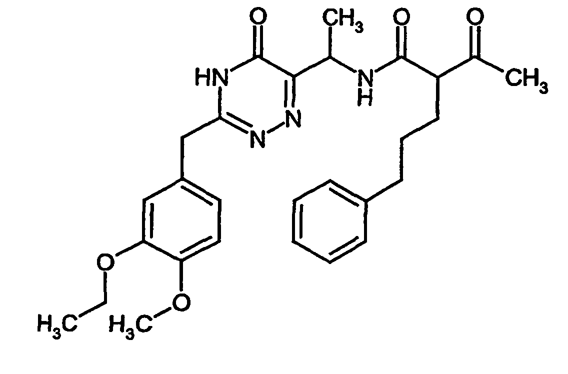

Analog **Beispiel 40A** werden 12,9 mg (0,10 mmol) Oxalsäuredichlorid, 9,91 mg (0,13 mmol) Dimethylsulfoxid, 43,0 mg (0,08 mmol) N-{1-[3-(3-Ethoxy-4-methoxybenzyl)-5-oxo-4,5-dihydro-1,2,4-triazin-6-yl]propyl}2-(1-hydroxyethyl)-5-phenylpentansäureamid und 32,8 mg (0,25 mmol) *N*-Ethyldiisopropylamin zu 2-Acetyl-N-{1-[3-(3-ethoxy-4-methoxybenzyl)-5-oxo-4,5-dihydro-1,2,4-triazin-6-yl]-propyl}-5-phenylpentansäureamid umgesetzt.
Ausbeute: 40 mg (93,4 % d. Th.)
¹H-NMR (Methanol-d₄, Diastereomerengemisch): δ/ppm 1,22-1,36 (m, 6H), 1,38-1,50 (m, 2H), 1,54-1,67 (m, 2H), 2,08 (m, 3H), 2,45-2,60 (m, 2H, unter DMSO-Signal), 3,47 (m, 1H), 3,72 (s, 3H), 3,75 (s, 2H), 3,92-4,02 (q, 2H), 4,90-5,06 (m, 1 H), 6,79-7,30 (m, 8H).

### Beispiel 53A

2-(1-Hydroxyethyl)-N-{1-[3-(1-naphthylmethyl)-5-oxo-4,5-dihydro-1,2,4-triazin-6-yl]ethyl}-5-phenylpentansäureamid

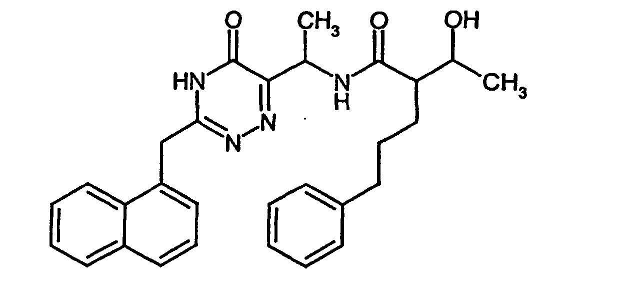

526 mg (2,37 mmol) 2-(1-Hydroxyethyl)-5-phenylpentansäure werden analog **Beispiel 39A** mit 320 mg (2,37 mmol) 1-Hydroxy-1H-benzotriazol, 408 mg (3,16 mmol) N-Ethyldiisopropylamin, 454 mg (6,350 mmol) N'-(3-Dimethylaminopropyl)-N-ethylcarbodiimid Hydrochlorid und 500 mg (1,58 mmol) 6-(1-Aminoethyl)-3-(1-naphthylmethyl)-1,2,4-triazin-5(4H)-on zu 2-(1-hydroxyethylrN-{1-[3-(1-naphthylmethyl)-5-oxo-4,5-dihydro-1,2,4-triazin-6-yl]ethyl}-5-phenylpentansäureamid umgesetzt.
Ausbeute: 557 mg (66,3 % d. Th.)
¹H-NMR (Methanol-d₄, Diastereomerengemisch): δ/ppm 1,06-1,18 (m, 3H), 1,38-1,44 (m, 3H), 1,44-1,82 (m, 4H), 2,15-2,27 (m, 1H), 2,49-2,68 (m, 2H), 3,65-3,83 (m, 1H), 4,39 und 4,41 (s, 2H), 5,09-5,21 (m, 1H), 7,06-7,92 (m, 12H).

### Beispiel 54A

2-Acetyl-N-{1-[3-(1-naphthylmethyl)-5-oxo-4,5-dihydro-1,2,4-triazin-6-yl]ethyl}-5-phenylpentansäureamid

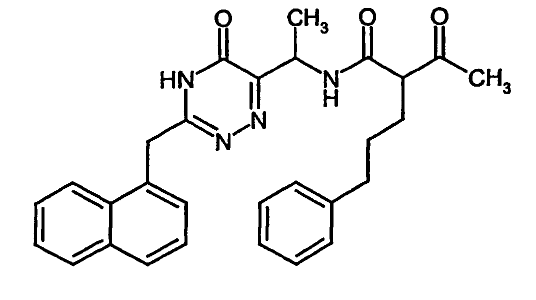

Analog **Beispiel 40** werden 310 mg (2,44 mmol) Oxalsäuredichlorid, 239 mg (3,05 mmol) Dimethylsulfoxid, 740 mg (1,53 mmol) 2-(1-hydroxyethyl)-N-{1-[3-(1-naphthylinethyl)-5-oxo-4,5-dihydro-1,2,4-triazin-6-yl]ethyl}-5-phenylpentansäureamid und 790 mg (6,11 mmol) *N*-Ethyldiisopropylamin zu 2-Acetyl-N-{1-[3-(1-naphthylmethyl)-5-oxo-4,5-dihydro-1,2,4-triazin-6-yl]ethyl}-5-phenylpentansäureamid umgesetzt.
Ausbeute: 90 mg (12 % d. Th.)
¹H-NMR (DMSO-d₆, Diastereomerengemisch): δ/ppm 1,19-1,71 (m, 7H), 2,07 (s, 3H), 2,45-2,61 (m, 2 H, unter DMSO-Signal), 3,46 (m, 1H), 4,37 (s, 2H), 4,91-5,01 (m, 1H), 7,10-8,16 (m, 12H).

### Beispiel 55A

2-Acetyl-N-[1-(3-{4-methyl-3-[(methylamino)sulfonyl]benzyl}-5-oxo-4,5-dihydro-12,4-triazin-6-yl)ethyl]-5-phenylpentansäureamid

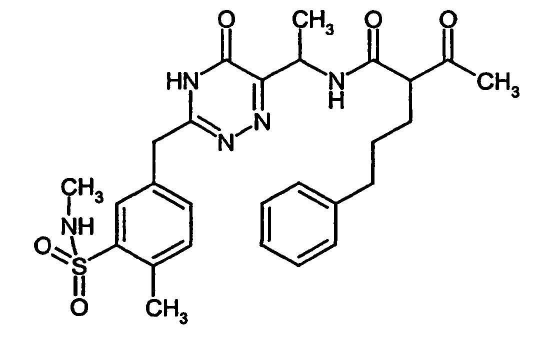

Die Menge an 2-Acetyl-5-phenylpentansäure aus **Beispiel 29A** wird analog **Beispiel 39A** mit 600 mg (4,45 mmol) 1-Hydroxy-1H-benzotriazol, 410 mg (4,08 mmol) 4-Methylmorpholin, 850 mg (4,45 mmol) N'-(3-Dimethylaminopropyl)-N-ethylcarbodiimid Hydrochlorid und 1,25 g (3,70 mmol) 5-{[6-(1-Aminoethyl)-5-oxo-4,5-dihydro-1,2,4-triazin-3-yl]methyl}-N,2-dimethylbenzolsulfonamid zu 2-Acetyl-N-[1-(3- {4-methyl-3-[(methylamino)sulfonyl]benzyl}-5-oxo-4,5-dihydro-12,4-triazin-6-yl)ethyl]-5-phenylpentansäureamid umgesetzt.
Ausbeute: 200 mg (10 % d. Th.)
¹H-NMR (Methanol-d₄, Diastereomerengemisch): δ/ppm 1,42 (d, 3H), 1,48-1,67 (m, 2H), 1,69-1,84 (m, 2H), 2,15 (s, 3H), 2,49 (s, 3H), 2,54-2,65 (m, 5H, s bei 2,58), 3,48 (m, 1H), 3,97 (s, 2H), 5,12 (q, 1H), 7,06-7,89 (m, 8H).

### Herstellungsbeispiele

### Beispiel 1

7-(1-Acetyl-4-phenylbutyl)-5-methyl-2-(4-methylbenzyl)imidazo[5,1-f][1,2,4]triazin-4(3H)-on

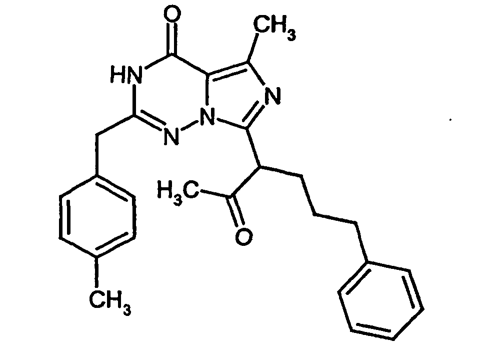

520 mg (1,16 mmol) 2-Acetyl-N-{1-[3-(4-methylbenzyl)-5-oxo-4,5-dihydro-1,2,4-triazin-6-yl]ethyl}-5-phenylpentansäureamid in 10 ml Dichlorethan werden mit 180 mg (1,16 mmol) Phosphoroxychlorid versetzt und 1 h bei 100°C am Rückfluß gerührt. Die abgekühlte Mischung wird mit gesättigter Natriumhydrogencarbonatlösung neutralisiert und vom Lösemittel abgezogen. Mit Dichlormethan/Methanol 70/1 wird das Produkt chromatographiert.
Ausbeute: 300 mg (60,1 % d. Th.)
Schmelzpunkt (Feststoff aus Diethylether): 119°C
¹H-NMR (400 MHz, Methanol-d₄): δ/ppm 1,45-1,54 (m, 2H), 2,00 (s, 3H), 2,00-2,13 (m, 2H), 2,28 (s, 3H), 2,53 (s, 3H), 2,53-2,60 (m, 2H), 3,78 (s, 2H), 4,31 (m, 1H), 7,05-7,22 (m, 9H).

### Beispiel 2

7-(1-Acetyl-4-phenylbutyl)-2-(4-methoxybenzyl)-5-methylimidazo[5,1-f][1,2,4]tria zin-4(3H)-on

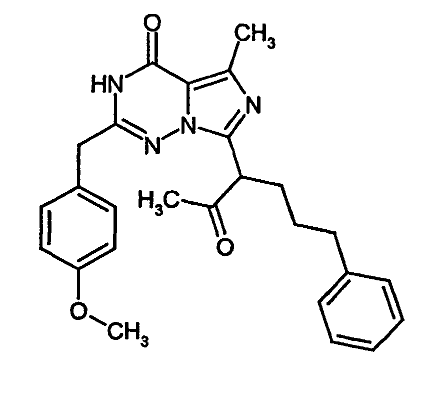

Analog **Beispiel 1** werden 290 mg (0,63 mmol) 2-Acetyl-N-{1-[3-(4-methoxybenzyl)-5-oxo-4,5-dihydro-1,2,4-triazin-6-yl]ethyl}-5-phenylpentansäureamid und 100 mg (0,63 mmol) Phosphoroxychlorid zu 7-(1-Acetyl-4-phenylbutyl)-2-(4-methoxybenzyl)-5-methylimidazo[5,1-f][1,2,4]triazin-4(3H)-on umgesetzt.
Ausbeute: 155 mg (55,6 % d. Th.)
Schmelzpunkt (Feststoff aus Diethylether): 138°C
¹H-NMR (400 MHz, Methanol-d₄): δ/ppm 1,45-1,57 (m, 2H), 2,00 (s, 3H), 2,00-2,16 (m, 2H), 2,49-2,62 (m, 5H, s bei 2,53), 3,74 (s, 3H), 3,76 (s, 2H), 4,32 (m, 1 H), 6,83 (d, 2H), 7,08 (d, 2H), 7,08-7,23 (m, 5H).

### Beispiel 3

7-(1-Acetyl-4-phenylbutyl)-2-(chinolin-6-ylmethyl)-5-methylimidazo[5,1-f][1,2,4]triazin-4(3H)-on

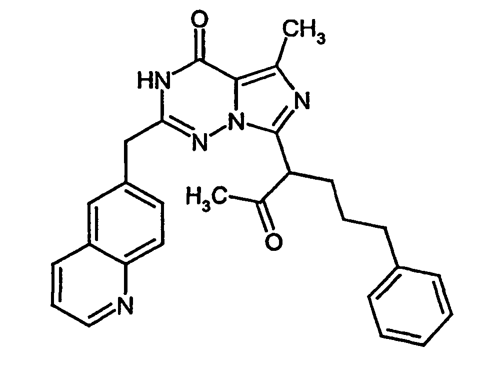

Analog **Beispiel 1** werden 2-Acetyl-N-{1-[3-(chinolin-6-ylmethyl)-5-oxo-4,5-dihydro-1,2,4-triazin-6-yl]ethyl}-5-phenylpentansäureamid und Phosphoroxychlorid zu 7-(1-Acetyl-4-phenylbutyl)-2-(chinolin-6-ylmethyl)-5-methylimidazo[5,1-f][1,2,4]-triazin-4(3H)-on umgesetzt.
R_{f}= 0,60 (CH₂Cl₂:MeOH =10:1)
¹H-NMR (DMSO-d₆): δ/ppm 0,9-2,6 (m, 12H), 4,0-4,3 (m, 3H), 6,9-8,8 (m, 11H).
MS: m/z = 466 (M+H)

### Beispiel 4

7-(1-Acetyl-4-phenylbutyl)-2-(3,4-dimethoxybenzyl)-5-methylimidazo[5,1-f][1,2,4]triazin-4(3H)-on

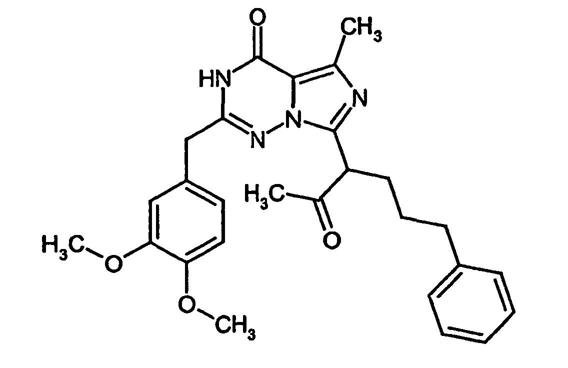

Analog **Beispiel 1** werden 1,30 g (2,64 mmol) 2-Acetyl-N {1-[3-(3,4-dimethoxybenzyl)-5-oxo-4,5-dihydro-1,2,4-triazin-6-yl]ethyl}-5-phenylpentansäureamid und 1,65 mg (10,7 mmol) Phosphoroxychlorid zu 7-(1-Acetyl-4-phenylbutyl)-2-(3,4-dimethoxybenzyl)-5-methylimidazo[5,1-fJ[1,2,4]triazin-4(3H)-on umgesetzt.
Ausbeute: 755 mg (60,2 % d. Th.)
Schmelzpunkt (Feststoff aus Essigsäureethylester/Diethylether): 154°C
¹H-NMR (400 MHz, Methanol-d₄): δ/ppm 1,44-1,58 (m, 2H), 2,00 (s, 3H), 2,00-2,15 (m, 2H), 2,49-2,63 (m, 5H, s bei 2,53), 3,76 (s, 2H), 3,78 (s, 6H), 4,30 (m, 1 H), 6,85-7,21 (m, 8H).

### Beispiel 5

7-[(3E)-1-Acetyl-4-phenyl-3-butenyl]-2-(3,4-dimethoxybenzyl)-5-methylimidazo[5,1-f][1,2,4]triazin-4(3H)-on

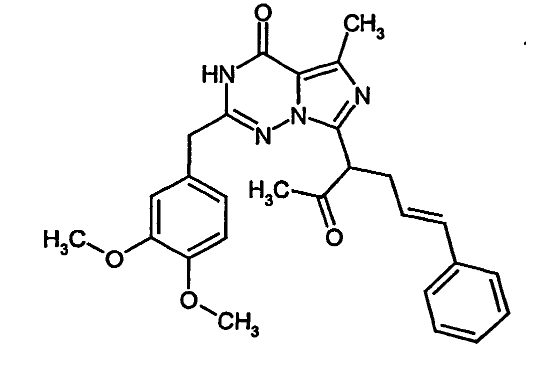

Analog **Beispiel 1** werden 540 mg (1,10 mmol) (4E)-2-Acetyl-N-{1-[3-(3,4-dimethoxybenzyl)-5-oxo-4,5-dihydro-1,2,4-triazin-6-yl]ethyl}-5-phenyl-4-pentenamid und 540 mg (3,50 mmol) Phosphoroxychlorid zu 7-[(3E)-1-Acetyl-4-phenyl-3-butenyl]-2-(3,4-dimethoxybenzyl)-5-methylimidazo[5,1-f][1,2,4]triazin-4(3H)-on umgesetzt.
Ausbeute: 351 mg (67,7 % d. Th.)
Rf-Wert (CH₂Cl₂/MeOH 10/1): 0,57
¹H-NMR (400 MHz, Methanol-d₄): δ/ppm 2,06 (s, 3H), 2,53 (s, 3H), 2,85-3,02 (m, 2H), 3,77 (s, 2H), 3,79 (s, 6H), 4,48 (m, 1H), 6,06-6,14 (m, 1H), 6,28 (d, 1H), 6,84-7,23 (m, 8H).

### Beispiel 6

7-(1-Acetyl-1-methyl-4-phenylbutyl)-2-(3,4-dimethoxybenzyl)-5-methylimidazo[5,1-f][1,2,4]triazin-4(3H)-on

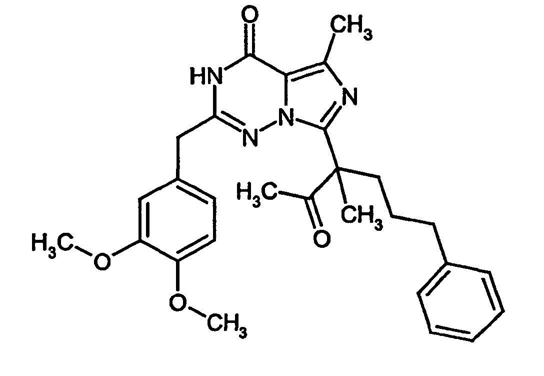

**Analog Beispiel 1** werden 3,08 g (6,07 mmol) 2-Acetyl-N-{1-[3-(3,4-dimethoxybenzyl)-5-oxo-4,5-dihydro-1,2,4-triazin-6-yl]ethyl}-2-methyl-5-phenylpentansäureamid und 4,44 mg (29,0 mmol) Phosphoroxychlorid zu 7-(1-Acetyl-1-methyl-4-phenylbutyl)-2-(3,4-dimethoxybenzyl)-5-methylimidazo[5,1-f][1,2,4]triazin-4(3H)-on umgesetzt.
Ausbeute: 2,50 mg (84,2 % d. Th.)
Rf-Wert (CH₂Cl₂/MeOH 10/1): 0,53
¹H-NMR (400 MHz, Methanol-d₄): δ/ppm 1,10 und 1,57 (je m, 2H), 1,51 (s, 3H), 1,81 (s, 3H), 1,94 und 3,20 (je m, 2H), 2,38-2,58 (m, 5H, s bei 2,54), 3,61 (s, 2H), 3,80 (s, 3H), 3,84 (s, 3H), 6,77-7,18 (m, 8H).

### Beispiel 7

7-(1-Acetyl-4-cyclohexylbutyl)-2-(3,4-dimethoxybenzyl)-5-methylimidazo[5,1-f][1,2,4]triazin-4(3H)-on

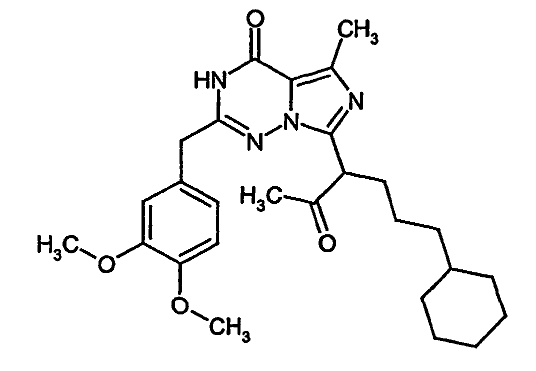

Analog **Beispiel 1** werden 240 mg (0,47 mmol) 2-Acetyl-5-cyclohexyl-N-{1-[3-(3,4-dimethoxybenzyl)-5-oxo-4,5-dihydro-1,2,4-triazin-6-yl]ethyl}pentansäureamid und 350 mg (2,30 mmol) Phosphoroxychlorid zu 7-(1-Acetyl-4-cyclohexylbutyl)-2-(3,4-dimethoxybenzyl)-5-methylimidazo[5,1-f][1,2,4]triazin-4(3H)-on umgesetzt.
Ausbeute: 147 mg (64,9 % d. Th.)
Rf-Wert (CH₂Cl₂/MeOH 10/1): 0,46
¹H-NMR (400 MHz, Methanol-d₄): δ/ppm 0,72-0,87 (m, 2H), 1,08-1,27 (m, 8H), 1,55-1,70 (m, 6H), 1,97-2,09 (m, 4H, s bei 2,03), 2,54 (s, 3H), 3,78 (s, 2H), 3,81 (s, 3H), 3,83 (s, 3H), 4,27 (m, 1H), 6,87-6,97 (m, 3H).

### Beispiel 8

7-(1-Acetyl-4-phenylbutyl)-2-(3,4-dimethoxybenzyl)-5-ethylimidazo[5,1-f][1,2,4]triazin-4(3H)-on

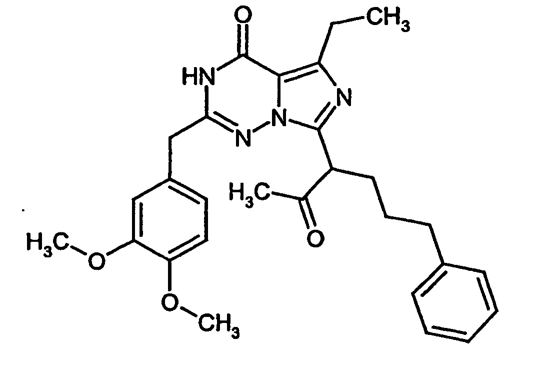

Analog **Beispiel 1** werden 530 mg (1,04 nunol) 2-Acetyl-N-{1-[3-(3,4-dimethoxybenzyl)-5-oxo-4,5-dihydro-1,2,4-triazin-6-yl]propyl}-5-phenylpentansäureamid und 610 mg (4,00 mmol) Phosphoroxychlorid zu 7-(1-Acetyl-4-phenylbutyl)-2-(3,4-dimethoxybenzyl)-5-ethylimidazo[5,1-f][1,2,4]triazin-4(3H)-on umgesetzt.
Ausbeute: 371 mg (73,1 % d. Th.)
Rf-Wert (CH₂Cl₂/MeOH 10/1): 0,70
¹H-NMR (400 MHz, Methanol-d₄): δ/ppm 1,26 (t, 3H), 1,42-1,58 (m, 2H), 2,00 (s, 3H), 2,00-2,15 (m, 2H), 2,50-2,66 (m, 2H), 2,94 (q, 2H), 3,76 (s, 2H), 3,78 (s, 6 H), 4,31 (m, 1H), 6,85-7,23 (m, 8H).

### Beispiel 9

7-(1-Acetyl-4-phenylbutyl)-2-(3-ethoxy-4-methoxybenzyl)-5-methylimidazo[5,1-f][1,2,4]triazin-4(3H)-on

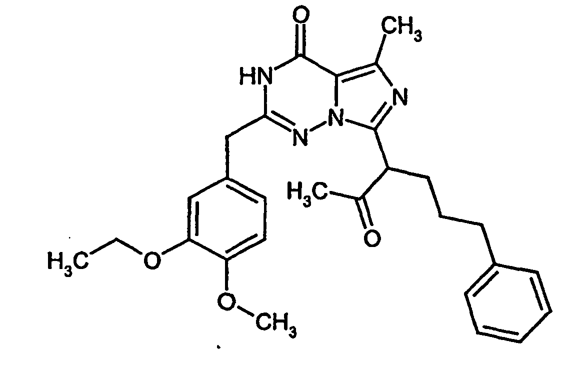

Analog **Beispiel 1** werden 123 mg (0,24 nunol) 2-Acetyl-N-{1-[3-(3-ethoxy-4-methoxybenzyl)-5-oxo-4,5-dihydro-1,2,4-triazin-6-yl]propyl}-5-phenylpentansäureamid und 41,0 mg (0,27 mmol) Phosphoroxychlorid zu 7-(1-Acetyl-4-phenylbutyl)-2-(3-ethoxy-4-methoxybenzyl)-5-methylimidazo[5,1-1][1,2,4]triazin-4(3H)-on umgesetzt.
Ausbeute: 52 mg (40,8% d. Th.)
Rf-Wert (CH₂Cl₂/MeOH 100/5): 0,48
¹H-NMR (400 MHz, Methanol-d₄): δ/ppm 1,35 (t, 3H), 1,45-1,58 (m, 2H), 1,98-2,17 (m, 5H, s bei 2,00), 2,49-2,63 (m, 5H, s bei 2,53), 3,75 (s, 2H), 3,78 (s, 3H), 4,01 (q, 2H), 4,31 (m, 1H), 6,85-7,21 (m, 8H).

### Beispiel 10

7-(1-Acetyl-4-phenylbutyl)-5-methyl-2-(1-naphthylmethyl)imidazo[5,1-f][1,2,4]triazin-4(3H)-on

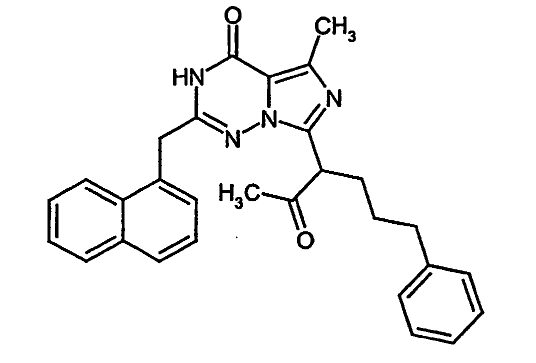

Analog **Beispiel 1** werden 80 mg (0,17 mmol) 2-Acetyl-N-(1-[3-(1-naphthylmethyl)-5-oxo-4,5-dihydro-1,2,4-triazin-6-yl]ethyl}-5-phenylpentansäureamid und 28,0 mg (0,18 mmol) Phosphoroxychlorid zu 7-(1-Acetyl-4-phenylbutyl)-5-methyl-2-(1-naph-thylmethyl)imidazo[5,1-f][1,2,4]triazin-4(3H)-on umgesetzt.
Ausbeute: 10 mg (13,0 % d. Th.)
Rf-Wert (CH₂Cl₂/MeOH 100/5): 0,48
¹H-NMR (400 MHz, Methanol-d₄): δ/ppm 1,24-1,30 (m, 2H), 1,71 (s, 3H), 1,75-1,96 (m, 2H), 2,30-2,45 (m, 2H), 2,51 (s, 3H), 4,02 (m, 1H), 4,34 (s, 2H), 6,93-8,13 (m, 12H).

### Beispiel 11

5-{[7-(1-Acetyl-4-phenylbutyl)-5-methyl-4-oxo-3,4-dihydroimidazo[5,1-f][1,2,4]triazin-2-yl]methyl}-N,2-dimethylbenzylsulfonamid

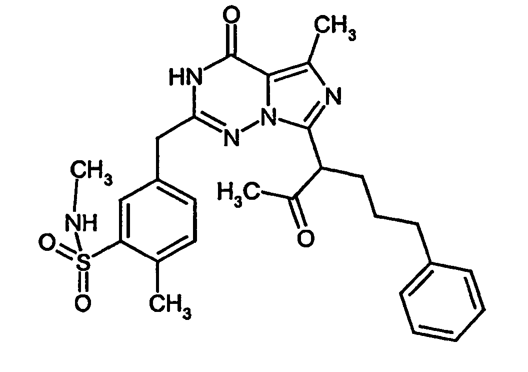

Analog **Beispiel 1** werden 250 mg (0,46 mmol) 2-Acetyl-N-[1-(3-{4-methyl-3-[(methylamino)sulfonyl]benzyll-5-oxo-4,5-dihydro-12,4-triazin-6-yl)ethyl]-5-phenylpentansäureamid und 0,32 mg (2,06 mmol) Phosphoroxychlorid zu 5-{[7-(1-acetyl-4-phenylbutyl)-5-methyl-4-oxo-3,4-dihydroimidazo[5,1-f][1,2,4]triazin-2-yl]-methyl}-N,2-dimethylbenzolsulfonamid umgesetzt.
Ausbeute: 110 mg (45,5 % d. Th.)
¹H-NMR (200 MHz, Methanol-d₄): δ/ppm 1,41-1,69 (m, 2H), 1,92-2,22 (m, 5H, s bei 1,98), 2,44-2,63 (m, 11H, s bei 2,48, 2,54 und 2,58), 3,89 (s, 2H), 4,25 (m, 1H), 7,03-7,95 (m, 8H).

### Beispiel 12

7-[1-(1-Hydroxyethyl)-4-phenylbutyl]-5-methyl-2-(4-methylbenzyl)imidazo[5,1-f][1,2,4]triazin-4(3H)-on

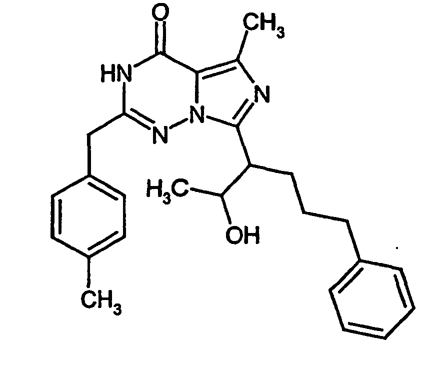

160 mg (0,37 mmol) 7-(1-Acetyl-4-phenylbutyl)-5-methyl-2-(4-methylbenzyl)imidazo[5,1-f][1,2,4]triazin-4(3H)-on werden in 5 ml Ethanol gelöst und portionsweise mit 14 mg (0,37 mmol) Natriumborhydrid versetzt. Der Ansatz wird 1 h bei Raumtemperatur gerührt, dann wird mit einigen Tropfen 2 N Salzsäure neutralisiert. Es wird das Lösemittel im Vakuum abgezogen, dann mit Laufmittel Dichlormethan/Methanol 40/1 chromatographiert.
Ausbeute: 75 mg (46,7 % d. Th.)
Schmelzpunkt (Feststoff aus Ether/Cyclohexan): 143°C
¹H-NMR (400 MHz, Methanol-d₄, Diastereomerengemisch): δ/ppm 0,93 und 1,19 (je d, 3H), 1,26-1,44 (m, 2H), 1,69-2,14 (m, 2H), 2,27 (s, 3H), 2,42-2,62 (m, 5H, s bei 2,52 und 2,53), 3,33-3,45 (m, 1H), 3,75 und 3,76 (je s, 2H), 3,97-4,11 (m, 1H), 6,98-7,22 (m, 9H).

### Beispiel 13

7-[1-(1-Hydroxyethyl)-4-phenylbutyl]-2-(chinolin-6-ylmethyl)-5-methylimidazo[5,1-f][1,2,4]triazin-4(3H)-on

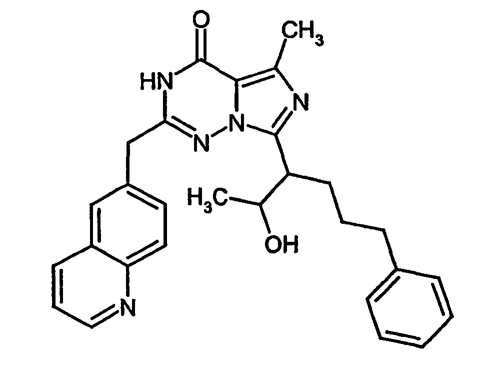

7-(1-Acetyl-4-phenylbutyl)-2-(chinolin-6-ylmethyl)-5-methylimidazo[5,1-f][1,2,4]triazin-4(3H)-on werden analog **Beispiel 12** mit Natriumborhydrid zu 7-[1-(1-Hydroxyethyl)-4-phenylbutyl]-2-(chinolin-6-ylmethyl)-5-methylimidazo[5,1-f][1,2,4]triazin-4(3H)-on umgesetzt.
R_{f}-Wert (CH₂Cl₂/MeOH 10/1): 0,53
¹H-NMR (DMSO-d₆, Diastereomerengemisch): δ/ppm 0,9-2,6 (m, 12H) 3,3-3,7 (m, 1H), 3,9-4,1 (m, 3H), 6,9-8,8 (m, 11).
MS: m/z = 468 (M+H).

### Beispiel 14

7-[1-(1-Hydroxyethyl)-4-phenylbutyl]-2-(4-methoxybenzyl)-5-methylimidazo[5,1-f][1,2,4]triazin-4(3H)-on

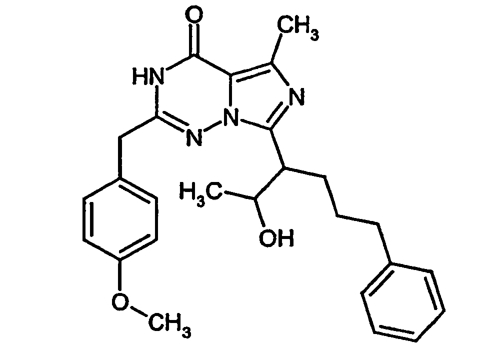

80 mg (0,19 mmol) 7-(1-Acetyl-4-phenylbutyl)-2-(4-methoxybenzyl)-5-methylimidazo[5,1-f][1,2,4]triazin-4(3H)-on werden analog **Beispiel 12** mit 9 mg (0,19 mmol) Natriumborhydrid zu 7-[1-(1-Hydroxyethyl)-4-phenylbutyl]-2-(4-methoxybenzyl)-5-methylimidazo[5,1-f][1,2,4]triazin-4(3H)-on umgesetzt.
Ausbeute: 70 mg (82,0 % d. Th.)
Rf-Wert (CH₂C1₂/MeOH 10/1): 0,61
¹H-NMR (400 MHz, Methanol-d₄, Diastereomerengemisch): δ/ppm 0,94 und 1,19 (je d, 3H), 1,28-1,44 (m, 2H), 1,70-2,14 (m, 2H), 2,42-2,63 (m, 5H, s bei 2,53), 3,34-3,45 (m, 1H), 3,71-3,77 (m, 5H), 3,98-4,12 (m, 1H), 6,79 (d, 2H), 7,02 (d, 2 H), 7,08-7,24 (m, 5H).

### Beispiel 15

2-(3,4-Dimethoxybenzyl)-7-[1-(1-hydroxyethyl)-4-phenylbutyl]-5-methylimidazo[5,1-f][1,2,4]triazin-4(3H)-on

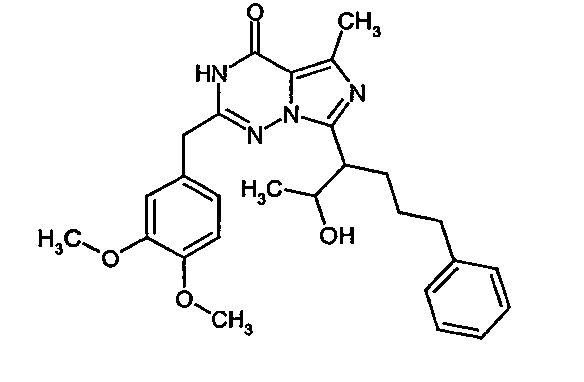

110 mg (0,22 mmol) 7-(1-Acetyl-4-phenylbutyl)-2-(3,4-dimethoxybenzyl)-5-methylimidazo[5,1-f][1,2,4]triazin-4(3H)-on werden analog **Beispiel 12** mit 20 mg (0,53 mmol) Natriumborhydrid zu 2-(3,4-Dimethoxybenzyl)-7-[1-(1-hydroxyethyl)-4-phenylbutyl]-5-methylimidazo[5,1-f][1,2,4]triazin-4(3H)-on umgesetzt.
Ausbeute: 89 mg (84,4 % d. Th.)
Rf-Wert (CH₂Cl₂/MeOH 10/1): 0,59
HPLC (RP 18, Temp.: 40°C, Fluß 1,25 ml/min., 50% Acetonitril + Wasser):
Verhältnis der Diastereomeren 33 : 60 (Beispiel 16 + 17 : Beispiel 18 + 19)
¹H-NMR (400 MHz, Methanol-d4, Diastereomerengemisch): δ/ppm 0,94 und 1,19 (je d, 3H), 1,28-1,45 (m, 2H), 1,70-2,15 (m, 2H), 2,41-2,61 (m, 5H, s bei 2,53), 3,34-3,45 (m, 1H), 3,73-3,75 (m, 8H), 3,98-4,11 (m, 1H), 6,79-7,21 (m, 8H).

Das **Beispiel 15** wird chromatographisch unter reversed phase Bedingungen (Stability C30, 5µm) mit Acetonitril/Wasser (1/1, v/v) als Elutionsmittel in die beiden diastereomeren Verbindungen getrennt. Die entsprechenden enantiomerenreinen Verbindungen (im Folgenden die Beispiele 16, 17, 18 und 19) lassen sich durch chromatographische Trennung der racemischen Diastereomeren an einer chiralen stationären Kieselgelphase gewinnen.

Besonders geeignet für die Trennung der Racemate sind chirale stationäre Polyamid-Kieselgelphasen (CSP) auf Basis der Monomeren N-Methaeryloyl-L-leucin-d-menthylamid oder N-Methacryloyl-L-leucin-1-menthylamid (vgl. EP-A-0 379 917) unter Verwendung von beispielsweise Ethylacetat als Elutionsmittel.

Die chromatographische Racemattrennung des zuerst eluierenden Diastereomeren aus **Beispiel 15** liefert die beiden Enantiomeren **Beispiel 16** und **Beispiel 17**. Analog dazu werden aus dem später eluierenden Diastereomeren die beiden Enantiomeren **Beispiel 18 und Beispiel 19 gewonnen .**

### Beispiel 16

2-(3,4-Dimethoxybenzyl)-7-{(1R)-1-[(1R)-1-hydroxyethyl]-4-phenylbutyl}-5-methylimidazo[5,1-f][1,2,4]triazin-4(3H)-on 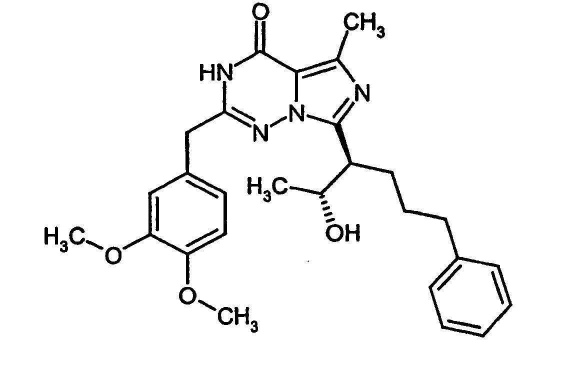 ¹H-NMR (400 MHz, DMSO-d₆): δ/ppm 0,82 (d, 3H), 1,20-1,35 (m, 2H), 1,69-2,06 (m, 2H), 2,38-2,53 (m, 5H, s bei 2,43, von DMSO-Signal überlagert), 3,17-3,25 (m, 1H), 3,65-3,72 (m, 8H), 3,78-3,88 (m, 1H), 4,79 (d, OH), 6,78-7,25 (m, 8H), 11,60 (s, NH).
[α]²⁰_{D} = -16,9° (c = 0.41500 g/100 ml; Lösungsmittel Methanol)
Schmelzpunkt: 167°C

### Beispiel 17

2-(3,4-Dimethoxybenzyl)-7-{(1S)-1-[(1S)-1-hydroxyethyl]-4-phenylbutyl}-5-methylimidazo[5,1-f][1,2,4]triazin-4(3H)-on 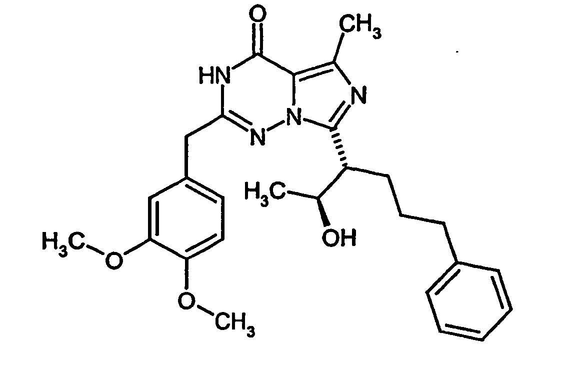 [α]²⁰_{D}=+18,4° (c = 0.42500 g/100 ml; Lösungsmittel Methanol)
Schmelzpunkt: 166°C

Die Trennung der Enantiomeren **Beispiel 18** und **Beispiel 19** erfolgte analog **Beispiel 16** ausgehend vom später eluierenden Diastereomeren.

### Beispiel 18

2-(3,4-Dimethoxybenzyl)-7-{(1R)-1-[(1S)-1-hydroxyethyl]-4-phenylbutyl}-5-methylimidazo[5,1-f][1,2,4]triazin-4(3H)-on 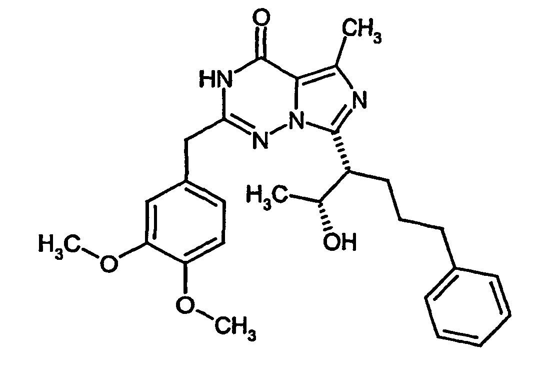 Charakterisierung: [α]²⁰_{D}= +26,3° (c = 0.44400 g/100 ml; Lösungsmittel Methanol)
Schmelzpunkt: 167°C
¹H-NMR (400 MHz, DMSO-d₆): δ/ppm 1,05 (d, 3H), 1,22-1,38 (m, 2H), 1,65-1,85 (m, 2H), 2,39-2,55 (m, 5H, s bei 2,43, von DMSO-Signal überlagert), 3,26-3,35 (m, 1H), 3,66-3,72 (m, 8H), 3,88-3,97 (m, 1H), 4,54 (d, OH), 6,78-7,25 (m, 8H), 11,58 (s, NH).

### Beispiel 19

2-(3,4-Dimethoxybenzyl)-7-{(1S)-1-[(1R)-1-hydroxyethyl]-4-phenylbutyl}-5-methylimidazo[5,1-f][1,2,4]triazin-4(3H)-on 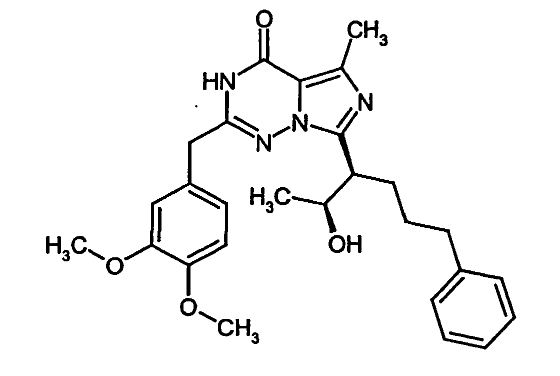 Charakterisierung: [α]²⁰_{D} = -27,9° (c = 0.45800 g/100 ml; Lösungsmittel Methanol)
Schmelzpunkt: 167°C

Außerdem können die Verbindungen der **Beispiele 16** und **17** auch bevorzugt erhalten werden durch diastereoselektive Reduktion von 7-(1-Acetyl-4-phenylbutyl)-2-(3,4-dimethoxybenzyl)-5-methylimidazo[5,1-f][1,2,4]triazin-4(3H)-on.

Dazu werden 190 mg (0,41 mmol) 7-(1-Acetyl-4-phenylbutyl)-2-(3,4-dimethoxybenzyl)-5-methylimidazo[5,1-f][1,2,4]triazin-4(3H)-on in 20 ml Dichlormethan/Methanol 100/1 gelöst und, mit 6,10 mg (0,45 mmol) Zinkchlorid versetzt, 30 min bei Raumtemperatur gerührt. Nach dem Abkühlen auf 0°C werden 30 mg Natriumborhydrid portionsweise zugefügt und im Anschluß unter Eisbadkühlung 2,5 h gerührt. Dann wird der Ansatz mit wenigen Tropfen 2 N Salzsäure neutralisiert, im Vakuum eingeengt und chromatographiert mit Laufinittel Dichlormethan/Methanol 80/1 und 40/1.
Ausbeute: 158 mg (81,5% d. Th.) Diastereomerengemisch
HPLC (RP 18, Temp.: 40°C, Fluß 1,25 ml/min., 50% Acetonitril + Wasser):
Verhältnis der Diastereomeren 95 : 5 (Beispiel 16 + 17 : Beispiel 18 + 19)

Das Diastereomerengemisch wird anschließend wie in **Beispiel 15** angeführt unter reversed phase-chromatographischen Bedingungen gereinigt und durch Chromatographie an der chiralen stationären Phase in die reinen Enantiomeren getrennt.

### Beispiel 20

2-(3,4-Dimethoxybenzyl)-7-[(3E)-1-(1-hydroxyethyl)-4-phenyl-3-butenyl]-5-methylimidazo[5,1-f][1,2,4]triazin-4(3H)-on

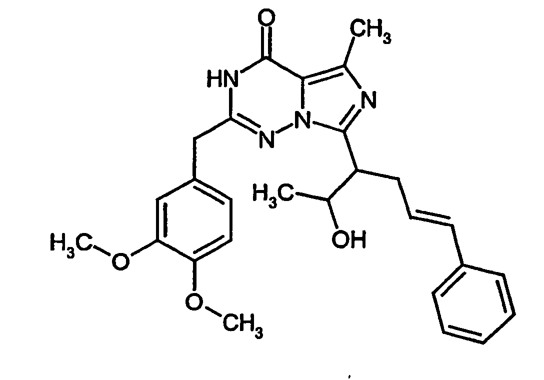

70 mg (0,14 mmol) 7-[(3E)-1-Acetyl-4-phenyl-3-butenyl]-2-(3,4-dimethoxybenzyl)-5-methylimidazo[5,1-f][1,2,4]triazin-4(3H)-on werden analog **Beispiel 12** mit 5 mg (0,14 mmol) Natriumborhydrid zu 2-(3,4-Dimethoxybenzyl)-7-[(3E)-1-(1-hydroxyethyl)-4-phenyl-3-butenyl]-5-methylimidazo[5,1-f][1,2,4]triazin-4(3H)-on umgesetzt.
Ausbeute: 55 mg (84,3% d. Th.)
Rf-Wert (CH₂Cl₂/MeOH 10/1): 0,40
¹H-NMR (400 MHz, Methanol-d₄, Diastereomerengemisch): δ/ppm 1,02 und 1,27 (je d, 3H), 2,53 und 2,54 (je s, 2H), 2,65-2,78 (m, 2H), 3,49-3,60 (m, 1H), 3,74-3,80 (m, 8H), 4,10-4,24 (m, 1H), 5,95-6,04 (m, 1H), 6,16-6,25 (m, 1H), 6,78-7,19 (m, 8H).

### Beispiel 21

2-(3,4-Dimethoxybenzyl)-7-[1-(1-hydroxyethyl)-1-methyl-4-phenylbutyl]-5-methylimidazo[5,1-f][1,2,4]triazin-4(3H)-on

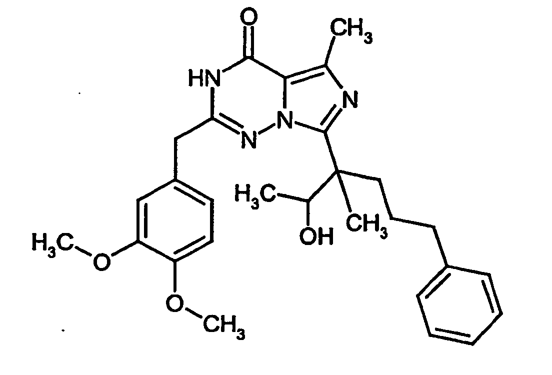

100 mg (0,20 mmol) 7-(1-Acetyl-1-methyl-4-phenylbutyl)-2-(3,4-dimethoxybenzyl)-5-methylimidazo[5,1-f][1,2,4]triazin-4(3H)-on werden analog **Beispiel 12** mit 23 mg (0,60 mmol) Natriumborhydrid zu 2-(3,4-Dimethoxybenzyl)-7-[1-(1-hydroxyethyl)-1-methyl-4-phenylbutyl]-5-methylimidazo[5,1-f][1,2,4]triazin-4(3H)-on umgesetzt.
Ausbeute: 78 mg (77,7 % d. Th.)
Rf-Wert (CH₂Cl₂/MeOH 10/1): 0,56
¹H-NMR (400 MHz, Methanol-d₄, Diastereomerengemisch): δ/ppm 0,81 und 1,06 (je d, 3H), 0,84-1,01 und 1,39-1,55 (je m, 2H), 1,36 und 1,37 (je s, 3H), 1,73-2,16 (je m, 2H), 2,31-2,49 (m, 2H), 2,51 (s, 3H), 3,58-3,73 (m, 2H), 3,76-3,82 (m, 6H), 4,39-4,59 (m, 1H), 6,80-7,20 (m, 8H).

Das **Beispiel 21** wird chromatographisch unter reversed phase Bedingungen analog **Beispiel 15** in die beiden diastereomeren Verbindungen getrennt. Die entsprechenden enantiomerenreinen Verbindungen (im Folgenden die Beispiele 22, 23, 24, 25) lassen sich durch chromatographische Trennung der racemischen Diastereomeren an einer chiralen stationären Kieselgelphase gewinnen (siehe oben).

### Beispiel 22

Charakterisierung: [α]²⁰_{D} = -69,1° (c = 0.49750 g/100 ml; Lösungsmittel Methanol)

### Beispiel 23

Charakterisierung: [α]²⁰_{D} = -32,1° (c = 0.34400 g/100 ml; Lösungsmittel Methanol)

### Beispiel 24

¹H-NMR (400 MHz, Methanol-d₄): δ/ppm 0,81 (d, 3H), 0,89-1,01 (m, 1H), 1,37 (s, 3H), 1,45-1,57 (m, 1H), 1,74-1,83 (m, 1H), 2,32-2,49 (m, 2H), 2,52 (s, 3H), 3,58-3,70 (m, 2H), 3,77-3,82 (m, 6H), 4,53-4,59 (m, 1H), 6,83-7,18 (m, 8H). Charakterisierung: [α]²⁰_{D} = +73,3° (c = 0.52850 g/100 ml; Lösungsmittel Methanol)

### Beispiel 25

¹H-NMR (400 MHz, Methanol-d₄): δ/ppm 1,06 (d, 3H), 0,85-0,97 (m, 1H), 1,37 (s, 3H), 1,40-1,50 (m, 2H), 2,08-2,18 (m, 1H), 2,31-2,49 (m, 2 H), 2,52 (s, 3H), 3,62-3,75 (m, 2H), 3,78-3,82 (m, 6H), 4,38-4,44 (m, 1H), 6,81-7,20 (m, 8H).
Charakterisierung: [α]²⁰_{D} = +31,0° (c = 0.45600 g/100 ml; Lösungsmittel Methanol)

### Beispiel 26

7-[4-Cyclohexyl-1-(1-hydroxyethyl)butyl]-2-(3,4-dimethoxybenzyl)-5-methylimidazo[5,1-f][1,2,4]triazin-4(3H)-on

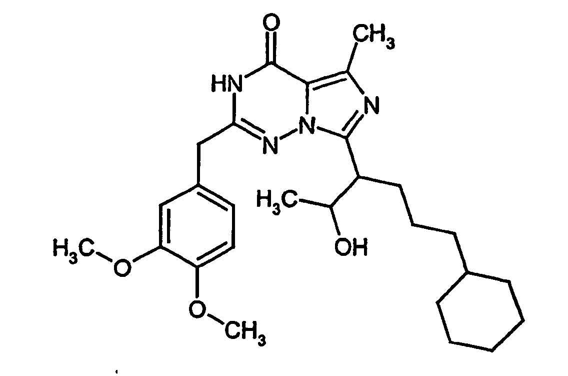

60 mg (0,13 mmol) 7-(1-Acetyl-4-cyclohexylbutyl)-2-(3,4-dimethoxybenzyl)-5-methylimidazo[5,1-f][1,2,4]triazin-4(3H)-on werden analog **Beispiel 12** mit 15 mg (0,40 mmol) Natriumborhydrid zu 7-[4-Cyclohexyl-1-(1-hydroxyethyl)butyl]-2-(3,4-dimethoxybenzyl)-5-methylimidazo[5,1-f][1,2,4]triazin-4(3H)-on umgesetzt.
Ausbeute: 52 mg (82,2 % d. Th.)
Rf-Wert (CH₂Cl₂/MeOH 10/1): 0,39
¹H-NMR (400 MHz, Methanol-d₄, Diastereomerengemisch): δ/ppm 0,66-0,92 (m, 2 H), 0,93-1,24 (m, 11H), 1,48-2,07 (m, 7H), 2,54 (s, 3H), 3,33-3,41 (m, 1H), 3,77-3,84 (m, 8H), 3,98-4,12 (m, 1H), 6,89-6,99 (m, 3H).

### Beispiel 27

2-(3,4-Dimethoxybenzyl)-S-ethyl-7-[1-(1-hydroxyethyl)-4-phenylbutyl]imidazo[5,1-f][1,2,4]triazin-4(3H)-on

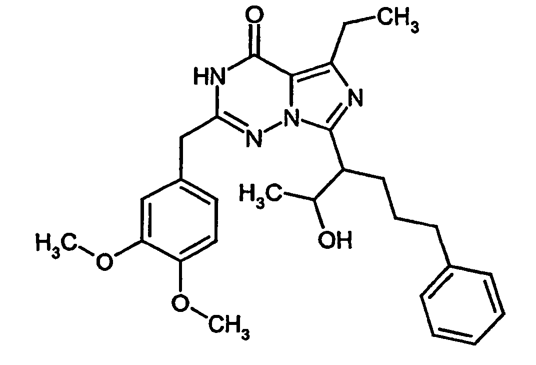

50 mg (0,10 mmol) 7-(1-Acetyl-4-phenylbutyl)-2-(3,4-dimethoxybenzyl)-5-ethylimidazo[5,1-f][1,2,4]triazin-4(3H)-on werden analog **Beispiel 12** mit 19 mg (0,50 mmol) Natriumborhydrid zu 2-(3,4-Dimethoxybenzyl)-5-ethyl-7-[1-(1-hydroxyethyl)-4-phenylbutyl]imidazo[5,1-f][1,2,4]triazin-4(3H)-on umgesetzt.
Ausbeute: 30 mg (59,8 % d. Th.)
Rf-Wert (CH₂Cl₂/MeOH 10/1): 0,60
¹H-NMR (400 MHz, Methanol-d₄, Diastereomerengemisch): δ/ppm 0,94 und 1,17 (je d, 3H), 1,21-1,30 (m, 3H), 1,31-1,43 (m, 2H), 1,68-2,13 (m, 2H), 2,39-2,61 (m, 2H), 2,89-2,99 (m, 2H), 3,35-3,46 (m, 1H), 3,72-3,79 (m, 8H), 4,01-4,14 (m, 1 H), 6,79-7,21 (m, 8H).

### Beispiel 28

2-(3-Ethoxy-4-methoxybenzyl)-7-[1-(1-hydroxyethyl)-4-phenylbutyl]-5-methylimidazo[5,1-f][1,2,4]triazin-4(3H)-on

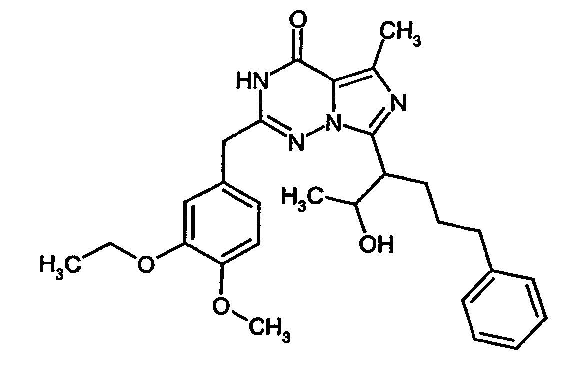

58 mg (0,12 mmol) 7-(1-Acetyl-4-phenylbutyl)-2-(3-ethoxy-4-methoxybenzyl)-5-methylimidazo[5,1-f][1,2,4]thazin-4(3H)-on werden analog **Beispiel 12** mit 5 mg (0,12 mmol) Natriumborhydrid zu 2-(3-Ethoxy-4-rnethoxybenzyl)-7-[1-(1-hydroxyethyl)-4-phenylbutyl]-5-methylimidazo[5,1-f][1,2,4]triazin-4(3H)-on umgesetzt.
Ausbeute: 56 mg (96,2 % d. Th.)
Rf-Wert (CH₂Cl₂/MeOH 100/5): 0,23
¹H-NMR (400 MHz, DMSO-d₆, Diastereomerengemisch): δ/ppm 0,82 und 1,05 (je d, 3H), 1,22-1,35 (m, 5H), 1,66-2,05 (m, 2H), 2,40-2,55 (m, 5H, s bei 2,42 und 2,43), 3,17-3,24 (m, 1H), 3,66 (s, 2H), 3,68 (s, 3H), 3,79-3,85 (m, 1H), 3,90-3,98 (m, 2H), 6,78-7,24 (m, 8H).

### Beispiel 29

5-({7-[1-(1-Hydroxyethyl)-4-phenylbutyl]-5-methyl-4-oxo-3,4-dihydroimidazo[5,1-f][1,2,4]triazin-2-yl]methyl)-N,2-dimethylbenzylsulfonamid

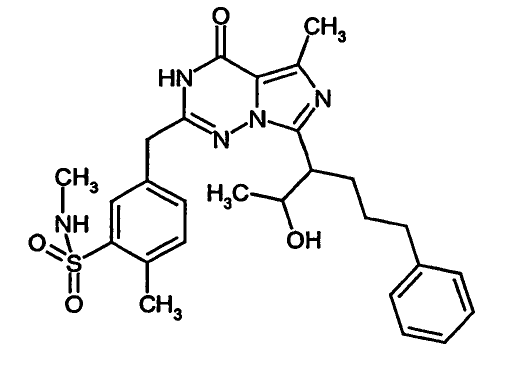

100 mg (0,19 mmol) 5-{[7-(1-Acetyl-4-phenylbutyl)-5-methyl-4-oxo-3,4-dihydroimidazo[5,1-f][1,2,4]triazin-2-yl]methyl}-N,2-dimethylbenzylsulfonamid werden analog **Beispiel 12** mit 7,2 mg (0,19 mmol) Natriumborhydrid zu 5-({7-[1-(1-Hydroxyethyl)-4-phenylbutyl]-5-methyl-4-oxo-3,4-dihydroimidazo[5,1-f][1,2,4]triazin-2-yl}methyl)-N,2-dimethylbenzylsulfonamid umgesetzt.
Ausbeute: 59 mg (57,4 % d. Th.)
¹H-NMR (400 MHz, Methanol-d₄, Diastereomerengemisch): δ/ppm 0,92 und 1,15 (je d, 3H), 1,27-1,43 (m, 2H), 1,64-2,10 (m, 2H), 2,38-2,59 (m, 11H), 3,25-3,46 (m, 1H), 3,88 (s, 2H), 3,96-4,07 (m, 1H), 6,98-7,93 (m, 8H).

## Patentansprüche

1. Verbindungen der allgemeinen Formel (I), 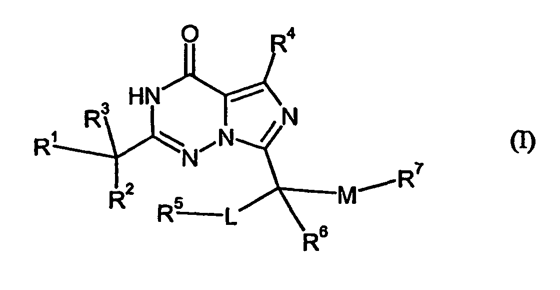 worin
R¹ Phenyl, Naphthyl, Chinolinyl oder Isochinolinyl, die bis zu dreifach gleich oder verschieden mit Resten ausgewählt aus der Gruppe bestehend aus (C₁-C₄)-Alkyl, (C₁-C₄)-Alkoxy, Halogen, Cyano -NHCOR⁸, -NHSO₂R⁹, -SO₂NR¹⁰R¹¹, -SO₂R¹², und -NR¹³R¹⁴ substituiert sein können, bedeutet,
worin
R⁸, R¹⁰, R¹¹, R¹³ und R¹⁴ unabhängig voneinander Wasserstoff oder (C₁-C₄)-Alkyl sind, und
R⁹ und R¹² unabhängig voneinander (C₁-C₄)-Alkyl sind,
oder
R¹⁰ und R¹¹ zusammen mit dem benachbarten Stickstoffatom einen Azetidin-1-yl-, Pyrrol-1-yl-, Piperid-1-yl-, Azepin-1-yl, 4-Methyl-piperazin-1-yl- oder Morpholin-1-yl-Rest bilden,
oder
R¹³ und R¹⁴ zusammen mit dem benachbarten Stickstoffatom einen Azetidin-1-yl-, Pyrrol-1-yl-, Piperid-1-yl-, Azepin-1-yl, 4-Methyl-piperazin-1-yl- oder Morpholin-1-yl-Rest bilden,
R² und R³ unabhängig voneinander Wasserstoff oder Fluor bedeuten,
R⁴ (C₁-C₄)-Alkyl bedeutet,
R⁵ (C₁-C₃)-Alkyl bedeutet,
R⁶ Wasserstoff oder Methyl bedeutet,
R⁷ Phenyl, Thiophenyl, Furanyl, die bis zu dreifach gleich oder verschieden mit Resten ausgewählt aus der Gruppe bestehend aus (C₁-C₄)-Alkyl, (C₁-C₄)-Alkoxy, Halogen und Cyano substituiert sein können, oder (C₅-C₈)-Cycloalkyl bedeutet,
L Carbonyl oder Hydroxymethandiyl bedeutet, und
M (C₂-C₅)-Alkandiyl, (C₂-C₅)-Alkendiyl oder (C₂-C₅)-ADdndiyl bedeutet,
und deren physiologisch verträgliche Salze.

2. Verbindungen nach Anspruch 1, wobei R¹ Phenyl, dessen meta- und/oder para-Positionen bis zu dreifach gleich oder verschieden mit Resten ausgewählt aus der Gruppe bestehend aus (C₁-C₄)-Alkyl, (C₁-C₄)-Alkoxy und -SO₂NR¹⁰R¹¹ substituiert sind, bedeutet und worin R¹⁰ und R¹¹ die in Anspruch 1 angegebene Bedeutung haben.

3. Verbindungen nach Anspruch 1 oder 2, wobei R⁷ Phenyl bedeutet.

4. Verbindungen nach Anspruch 1,
wobei
R¹ Phenyl, dessen meta- und/oder para-Positionen bis zu dreifach gleich oder verschieden mit Resten ausgewählt aus der Gruppe bestehend aus (C₁-C₄)-Alkyl, (C₁-C₄)-Alkoxy und -SO₂NR¹⁰R¹¹ substituiert sind, Naphthyl oder Chinolinyl bedeutet,
worin R¹⁰ und R¹¹ unabhängig voneinander Wasserstoff oder (C₁-C₄)-Alkyl sind,
R¹ und R² Wasserstoff bedeuten,
R⁴ Methyl oder Ethyl bedeutet,
R⁵ Methyl bedeutet,
R⁶ Wasserstoff oder Methyl bedeutet,
L Carbonyl oder Hydroxymethandiyl bedeutet, und
M geradkettiges (C₂-C₅)-Alkan-1,ω-diyl, geradkettiges (C₂-C₅)-Alken-1,ω-diyl oder geradkettiges (C₂-C₅)-Alkin-1,ω-diyl bedeutet.

5. Verfahren zur Herstellung von Verbindungen der allgemeinen Formel (I) nach Anspruch 1,
wobei
[A] eine Verbindung der allgemeinen Formel (IIa), 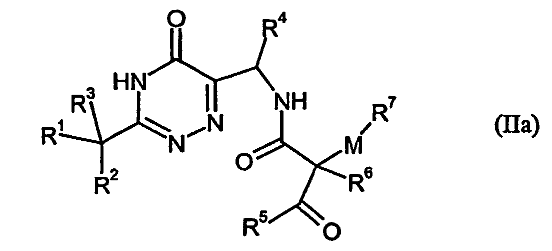 worin R¹, R², R³, R⁴, R⁵, R⁶, R⁷ und M die in Anspruch 1 angegebene Bedeutung haben,
unter geeigneten Kondensationsbedingungen zu einer Verbindung der allgemeinen Formel (Ia), 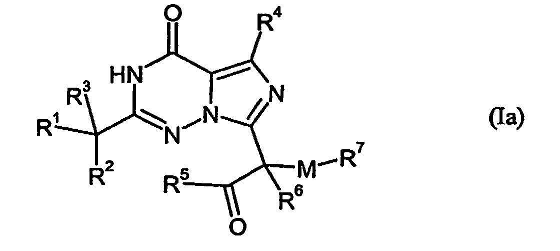 worin R¹, R², R³, R⁴, R⁵, R⁶, R⁷ und M die in Anspruch 1 angegebene Bedeutung haben,
umgesetzt wird,
und dann gegebenenfalls
[B] zu einer Verbindung der allgemeinen Formel (Ib) 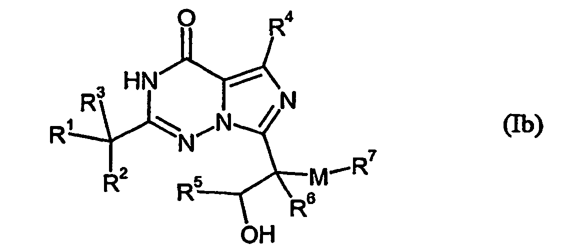
worin R¹, R², R³, R⁴, R⁵, R⁶, R⁷ und M die in Anspruch 1 angegebene Bedeutung haben,
unter geeigneten Bedingungen reduziert wird.

6. Verbindungen der allgemeinen Formel (II), 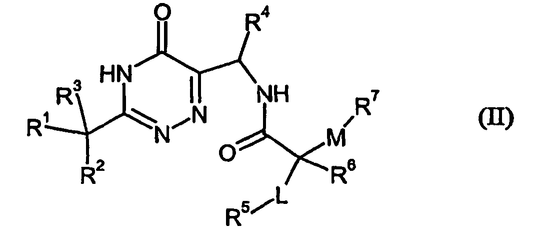 worin R¹, R², R³, R⁴, R⁵, R⁶, R⁷, L und M die in Anspruch 1 angegebene Bedeutung haben,
und deren Salze.

7. Verbindungen nach einem der Ansprüche 1 bis 4 zur Behandlung und/oder Prophylaxe von Krankheiten.

8. Arzneimittel enthaltend eine Verbindung nach einem der Ansprüche 1 bis 4.

9. Verbindungen nach einem der Ansprüche 1 bis 4 zur Verbesserung der Wahrnehmung, Konzentrationsleistung, Lernleistung und/oder Gedächtnisleistung.

10. Verbindungen nach einem der Ansprüche 1 bis 4 zur Behandlung und/oder Prophylaxe von Störungen der Wahrnehmung, Konzentrationsleistung, Lernleistung und/oder Gedächtnisleistung.

11. Verwendung von Verbindungen nach einem der Ansprüche 1 bis 4 zur Herstellung eines Arzneimittels zur Verbesserung der Wahrnehmung, Konzentrationsleistung, Lernleistung und/oder Gedächtnisleistung.

12. Verwendung von Verbindungen nach einem der Ansprüche 1 bis 4 zur Herstellung eines Arzneimittels zur Behandlung und/oder Prophylaxe von Störungen der Wahrnehmung, Konzentrationsleistung, Lernleistung und/oder Gedächtnisleistung.

13. Verwendung nach Anspruch 12, wobei die Störung eine Folge von Demenz ist.

14. Verwendung von Verbindungen nach einem der Ansprüche 1 bis 4 zur Herstellung eines Arzneimittels zur Behandlung und/oder Prophylaxe von Demenz.

## Claims

1. Compounds of the general formula (I), 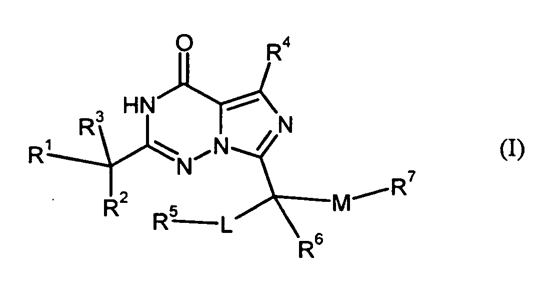 in which
R¹ denotes phenyl, naphthyl, quinolinyl or isoquinolinyl, each of which can be substituted up to three times identically or differently by radicals selected from the group consisting of (C₁-C₄)-alkyl, (C₁-C₄)-alkoxy, halogen, cyano, -NHCOR⁸, -NHSO₂R⁹, -SO₂NR¹⁰R¹¹, -SO₂R¹², and -NR¹³R¹⁴,
in which
R⁸, R¹⁰, R¹¹, R¹³ and R¹⁴ independently of one another are hydrogen or (C₁-C₄)-alkyl, and
R⁹ and R¹² independently of one another are (C₁-C₄)-alkyl,
or
R¹⁰ und R¹¹ together with the adjacent nitrogen atom form an azetidin-1-yl, pyrrol-1-yl, piperid-1-yl, azepin-1-yl, 4-methyl-piperazin-1-yl or morpholin-1-yl radical,
or
R¹³ and R¹⁴ together with the adjacent nitrogen atom form an azetidin-1-yl, pyrrol-1-yl, piperid-1-yl, azepin-1-yl, 4-methyl-piperazin-1-yl or morpholin-1-yl radical,
R² and R³ independently of one another denote hydrogen or fluorine,
R⁴ denotes (C₁-C₄)-alkyl,
R⁵ denotes (C₁-C₃)-alkyl,
R⁶ denotes hydrogen or methyl,
R⁷ denotes phenyl, thiophenyl, furanyl, each of which can be substituted up to three times identically or differently by radicals selected from the group consisting of (C₁-C₄)-alkyl, (C₁-C₄)-alkoxy, halogen and cyano, or (C₅-C₈)-cycloalkyl,
L denotes carbonyl or hydroxymethanediyl, and
M denotes (C₂-C₅)-alkanediyl, (C₂-C₅)-alkenediyl or (C₂-C₅)-alkinediyl,
and their physiologically tolerable salts.

2. Compounds according to Claim 1, where R¹ denotes phenyl, whose meta and/or para positions are substituted up to three times identically or differently by radicals selected from the group consisting of (C₁-C₄)-alkyl, (C₁-C₄)-alkoxy and -SO₂NR¹⁰R¹¹, and R¹⁰ and R¹¹ have the meaning indicated in Claim 1.

3. Compounds according to Claim 1 or 2, where R⁷ denotes phenyl.

4. Compounds according to Claim 1,
where
R¹ denotes phenyl, whose meta and/or para positions are substituted up to three times identically or differently by radicals selected from the group consisting of (C₁-C₄)-alkyl, (C₁-C₄)-alkoxy and -SO₂NR¹⁰R¹¹, naphthyl or quinolinyl,
in which R¹⁰ and R¹¹ independently of one another are hydrogen or (C₁-C₄)-alkyl,
R¹ and R² denote hydrogen,
R⁴ denotes methyl or ethyl,
R⁵ denotes methyl,
R⁶ denotes hydrogen or methyl,
L denotes carbonyl or hydroxymethanediyl, and
M denotes straight-chain (C₂-C₅)-alkane-1,ω-diyl, straight-chain (C₂-C₅)-alkene-1,ω-diyl or straight-chain (C₂-C₅)-alkine-1,ω-diyl.

5. Process for the preparation of compounds of the general formula (I) according to Claim 1,
where
[A] a compound of the general formula (IIa), 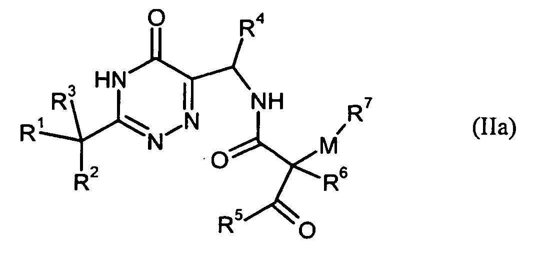 in which R¹, R², R³, R⁴, R⁵, R⁶, R⁷ and M have the meaning indicated in Claim 1,
is reacted under suitable condensation conditions to give a compound of the general formula (Ia), 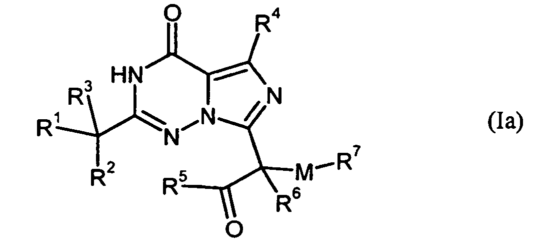 in which R¹, R², R³, R⁴, R⁵, R⁶, R⁷ and M have the meaning indicated in Claim 1,
and then, if appropriate,
[B] is reduced under suitable conditions to give a compound of the general formula (Ib) 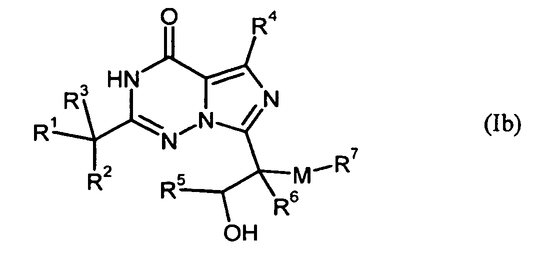 in which R¹, R², R³, R⁴, R⁵, R⁶, R⁷ and M have the meaning indicated in Claim 1.

6. Compounds of the general formula (II), 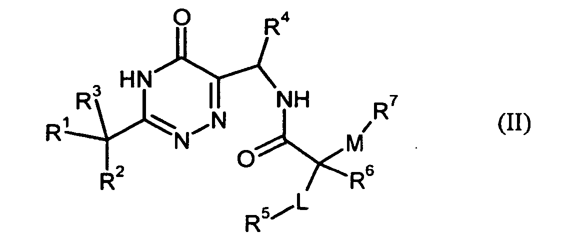 in which R¹, R², R³, R⁴, R⁵, R⁶, R⁷, L and M have the meaning indicated in Claim 1,
and their salts.

7. Compounds according to one of Claims 1 to 4 for the treatment and/or prophylaxis of illnesses.

8. Medicaments comprising a compound according to one of Claims 1 to 4.

9. Compounds according to one of Claims 1 to 4 for improving perception, concentration power, learning power and/or memory power.

10. Compounds according to one of Claims 1 to 4 for the treatment and/or prophylaxis of disorders of perception, concentration power, learning power and/or memory power.

11. Use of compounds according to one of Claims 1 to 4 for the production of a medicament for improving perception, concentration power, learning power and/or memory power.

12. Use of compounds according to one of Claims 1 to 4 for the production of a medicament for the treatment and/or prophylaxis of disorders of perception, concentration power, learning power and/or memory power.

13. Use according to Claim 12, the disorder being a result of dementia.

14. Use of compounds according to one of Claims 1 to 4 for the production of a medicament for the treatment and/or prophylaxis of dementia.

## Revendications

1. Composés de formule générale (I), 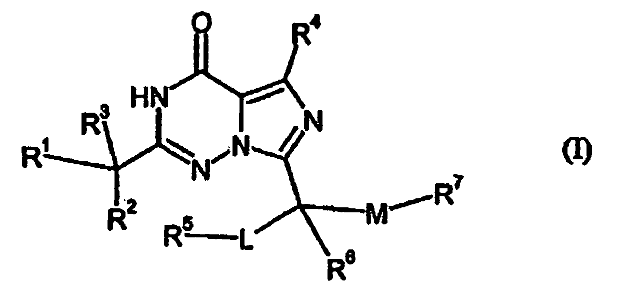 dans laquelle
R¹ est un reste phényle, un reste naphtyle, un reste quinolinyle ou un reste isoquinolinyle, qui peuvent être substitués jusqu'à trois fois identiques ou différentes avec des restes choisis dans le groupe constitué des restes alkyle en C₁ à C₄, alkoxy en C₁ à C₄, halogéno, cyano, -NHCOR⁸, -NHSO₂R⁹, -SO₂NR¹⁰R¹¹, -SO₂R¹² et -NR¹³R¹⁴,
où
R⁸, R¹⁰, R¹¹, R¹³ et R¹⁴ représentent, indépendamment les uns des autres, l'hydrogène ou un reste alkyle en C₁ à C₄, et
R⁹ et R¹² représentent, indépendamment l'un de l'autre, un reste alkyle en C₁ à C₄,
ou bien
R¹⁰ et R¹¹ forment, conjointement avec l'atome contigu d'azote, un reste azétidine-1-yle, pyrrole-1-yle, pipéride-1-yle, azépine-1-yle, 4-méthylpipérazine-1-yle ou morpholine-1-yle,
ou bien
R¹³ et R¹⁴ forment, conjointement avec l'atome contigu d'azote, un reste azétidine-1-yle, pyrrole-1-yle, pipéride-1-yle, azépine-1-yle, 4-méthylpipérazine-1-yle ou morpholine-1-yle,
R² et R³ représentent indépendamment l'un de l'autre l'hydrogène ou le fluor,
R⁴ est un reste alkyle en C₁ à C₄,
R⁵ est un reste alkyle en C₁ à C₃,
R⁶ représente l'hydrogène ou un reste méthyle,
R⁷ est un reste phényle, un reste thiophényle, un reste furannyle, qui peuvent être substitués jusqu'à trois fois identiques ou différentes par des restes choisis dans le groupe constitué des restes alkyle en C₁ à C₄, alkoxy en C₁ à C₄, halogéno et cyano, ou représente un reste cycloalkyle en C₅ à C₈,
L est un groupe carbonyle ou un reste hydroxyméthanediyle, et
M est un reste alcanediyle en C₂ à C₅, alcènediyle en C₂ à C₅ ou alcynediyle en C₂ à C₅,
et leurs sels acceptables du point de vue physiologique.

2. Composés suivant la revendication 1, dans lesquels R¹ est un reste phényle dont les positions méta- et/ou la position para- sont substituées jusqu'à trois fois identiques ou différentes avec des restes choisis dans le groupe constitué des restes alkyle en C₁ à C₄, alkoxy en C₁ à C₄ et -SO₂NR¹⁰R¹¹ et où R¹⁰ et R¹¹ ont la définition indiquée dans la revendication 1.

3. Composés suivant la revendication 1 ou 2, dans lesquels R⁷ désignent le reste phényle.

4. Composés suivant la revendication 1,
dans lesquels
R¹ est un reste phényle dont les positions méta- et/ou la position para- peuvent être substituées jusqu'à trois fois identiques ou différentes avec des restes choisis dans le groupe constitué des restes alkyle en C₁ à C₄, alkoxy en C₁ à C₄ et -SO₂NR¹⁰R¹¹, un reste naphtyle ou un reste quinolinyle,
R¹⁰ et R¹¹ représentant indépendamment l'un de l'autre l'hydrogène ou un reste alkyle en C₁ à C₄,
R¹ et R² représentent l'hydrogène,
R⁴ est un reste méthyle ou éthyle,
R⁵ est un reste méthyle,
R⁶ représente l'hydrogène ou un reste méthyle,
L est un groupe carbonyle ou un reste hydroxyméthanediyle, et
M est un reste alcane-1,ω-diyle en C₂ à C₅ à chaîne droite, un reste alcène-1,ω-diyle en C₂ à C₅ à chaîne droite ou un reste alcyne-1,ω-diyle en C₂ à C₅ à chaîne droite.

5. Procédé de production de composés de formule générale (I) suivant la revendication 1,
dans lequel
[A] on fait réagir un composé de formule générale (IIa), 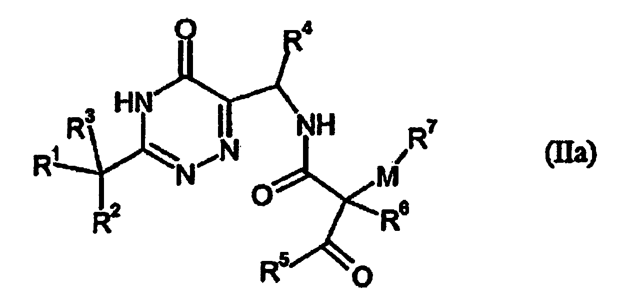 dans laquelle R¹, R², R³, R⁴, R⁵, R⁶, R⁷ et M ont la définition indiquée dans la revendication 1,
dans des conditions convenables de condensation pour obtenir un composé de formule générale (Ia), 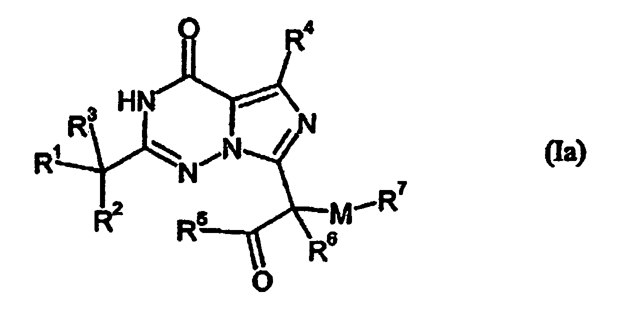 dans laquelle R¹, R², R³, R⁴, R⁵, R⁶, R⁷ et M ont la définition indiquée dans la revendication 1,
puis, le cas échéant,
[B] on réduit ce composé en un composé de formule générale (Ib) 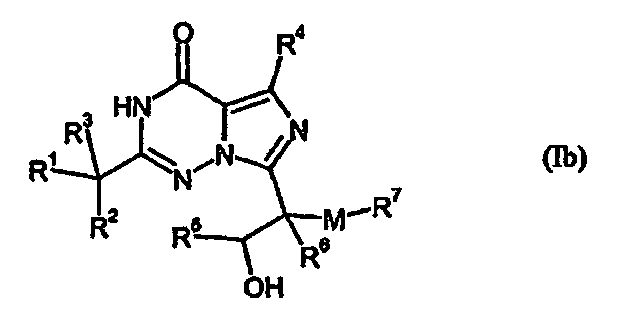
dans laquelle R¹, R², R³, R⁴, R⁵, R⁶, R⁷ et M ont la définition indiquée dans la revendication 1,
dans des conditions convenables.

6. Composés de formule générale (II), 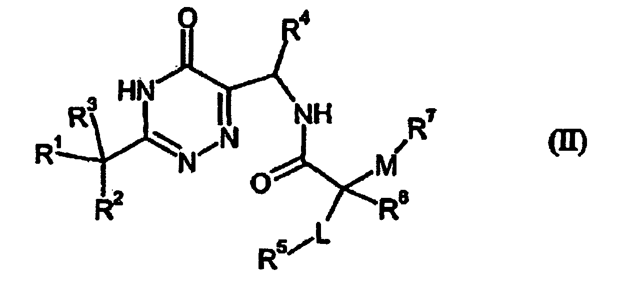 dans laquelle R¹, R², R³, R⁴, R⁵, R⁶, R⁷, L et M ont la définition indiquée dans la revendication 1,
et leurs sels.

7. Composés selon l'une des revendications 1 à 4, destinés au traitement et/ou à la prophylaxie de maladies.

8. Médicament contenant un composé selon l'une des revendications 1 à 4.

9. Composés selon l'une des revendications 1 à 4, destinés à améliorer la perception, la capacité de concentration, la capacité d'apprentissage et/ou la capacité de mémoire.

10. Composés selon l'une des revendications 1 à 4, destinés au traitement et/ou à la prophylaxie de troubles de la perception, de la capacité de concentration, de la capacité d'apprentissage et/ou de la capacité de mémoire.

11. Utilisation de composés selon l'une des revendications 1 à 4 pour la préparation d'un médicament destiné à améliorer la perception, la capacité de concentration, la capacité d'apprentissage et/ou la capacité de mémoire.

12. Utilisation de composés selon l'une des revendications 1 à 4 pour la préparation d'un médicament destiné au traitement et/ou à la prophylaxie de troubles de la perception, de la capacité de concentration, de la capacité d'apprentissage et/ou de la capacité de mémoire.

13. Utilisation suivant la revendication 12, dans laquelle le trouble est une conséquence de la démence.

14. Utilisation de composés selon l'une des revendications 1 à 4, pour la préparation d'un médicament destiné au traitement et à la prophylaxie de la démence.
